# EUROPÄISCHE PATENTSCHRIFT

(11) **EP 1 773 814 B1**
(45) Veröffentlichungstag und Bekanntmachung des Hinweises auf die Patenterteilung: **07.11.2007**
(21) Anmeldenummer: 05786049.6
(22) Anmeldetag: 13.07.2005
(51) Int. Cl.: C07D 401/12, A61K 31/454, C07D 209/34, A61P 25/00, A61K 31/404

(54) **SUBSTITUIERTE OXINDOL-DERIVATE UND DIESE ENTHALTENDE ARZNEIMITTEL**
SUBSTITUTED OXINDOL DERIVATIVES AND MEDICAMENTS CONTAINING THE SAME
DERIVES D'OXINDOLE SUBSTITUES, ET MEDICAMENTS LES RENFERMANT

(30) Priorität: 13.07.2004 DE 102004033834; 13.07.2004 US 587407 P
(43) Veröffentlichungstag der Anmeldung: 18.04.2007
(73) Patentinhaber: Abbott GmbH & Co. KG, 65205 Wiesbaden (DE)
(72) Erfinder: Dr. LUBISCH, Wilfried, 69118 Heidelberg (DE); OOST, Thorsten, 69115 Heidelberg (DE); WERNET, Wolfgang, 67434 Neustadt (DE); UNGER, Liliane, 67065 Ludwigshafen (DE); HORNBERGER, Wilfried, 67434 Neustadt (DE); GENESTE, Herve, 67141 Neuhofen (DE)
(74) Vertreter: Grünecker, Kinkeldey, Stockmair & Schwanhäusser Anwaltssozietät
(86) Internationale Anmeldenummer: PCT/EP2005/007631
(87) Internationale Veröffentlichungsnummer: WO 2006/005609

(56) Entgegenhaltungen:
- WO-A-03/008407
- US-A- 5 594 023

## Beschreibung

Die vorliegende Erfindung betrifft neue Oxindol-Derivate und diese enthaltende Arzneimittel zur Behandlung von Krankheiten.

Vasopressin ist ein endogenes Hormon, das verschiedenste Wirkungen an Organen und Gewebe ausübt. In verschiedenen Krankheitszuständen vermutet man, daß das Vasopressin-System eine Rolle spielt, wie zum Beispiel Herzinsuffizienz und Bluthochdruck. Derzeit sind drei Rezeptoren (V1a, V1b bzw. V3 und V2) bekannt, über die Vasopressin seine zahlreichen Wirkungen vermittelt. Daher werden Antagonisten dieser Rezeptoren als mögliche neue therapeutische Ansätze zur Behandlung von Krankheiten untersucht (M. Thibonnier, Exp. Opin. Invest. Drugs 1998, 7(5), 729-740).

In der vorliegenden Anmeldung werden neue substituierte Oxindole beschrieben, die in 1-Stellung eine Aryl-sulfonyl-Gruppe tragen. 1-Phenylsulfonyl-1,3-dihydro-2H-indol-2-one wurden bereits als Liganden der Vasopressin-Rezeptoren beschrieben. In WO 93/15051, WO95/18105, WO 98/25901, WO 01/55130, WO 01/55134, WO 01/64668 und WO01/98295 wurden Derivate beschrieben, die vom Oxindol-Gerüst abgeleitet sind und in 1-Stellung Arylsulfonlygruppen tragen. Diese Verbindungen unterscheiden sich wesentlich in der Substitution in 3-Stellung.

Insbesondere werden in der WO 93/15051 und WO 98/25901 1-Phenylsulfonyl-1,3-dihydro-2H-indol-2-one als Liganden der Vasopressinrezeptoren beschrieben, bei denen das Oxindol-Gerüst in der 3-Stellung durch zwei Alkylradikale substituiert ist, die ebenfalls ein Cycloalkylradikal (Spiroverknüpfung) sein können. Als Alternative kann der Spiroring Heteroatome, wie Sauerstoff und Stickstoff (wahlweise mit Substituenten), enthalten.

Die WO 95/18105 beschreibt 1-Phenylsulfonyl-1,3-dihydro-2H-indol-2-one als Liganden der Vasopressinrezeptoren, die ein Stickstoffatom in der 3-Stellung besitzen. Zusätzlich sind in der 3-Stellung Radikale gebunden, die Alkyl, Cycloalkyl, Phenyl oder Benzylradikale sein können (jeweils wahlweise mit Substituenten).

Andere Veröffentlichungen, zum Beispiel WO 01/55130, beschreiben Verbindungen, die Stickstoff enthaltende Ringe besitzen (z.B. Prolin, Homoprolin, Morpholin, Tetrahydroisochinolin, Dihydroindol; jeweils wahlweise mit Substituenten), die über ihr Stickstoffatom zur 3-Stellung des Oxindol-Gerüsts gebunden sind, die jedoch durch Phenylsulfonyl- oder Phenyl-Gruppen (wahlweise mit Substituenten) sowohl in der 1-Stellung als auch der 3-Stellung am Oxindolring substituiert sind.

In WO 03/008407 sind 1-Phenylsulfonyl-oxindole beschrieben, in denen in 3 Stellung Pyridylpiperazine über eine Oxycarbonyl-Gruppe und analoge funktionelle Gruppen am Oxindol gebunden sind.

Aufgabe der vorliegenden Erfindung ist es, weitere Verbindungen zur Behandlung oder Prophylaxe von verschiedenen Vasopressin-abhängigen oder Oxytocin-abhängigen Krankheiten zur Verfügung zu stellen, die eine hohe und selektive Aktivität aufweisen.

Die Aufgabe wird gelöst durch eine Verbindung oder Verbindungen der allgemeinen Formel (I), 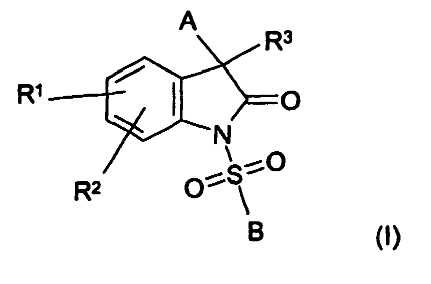 worin
- A: C₆₋₁₀-Aryl ist, das mit maximal vier Resten R⁴ substituiert sein kann, die unabhängig voneinander ausgewählt sind aus der Gruppe, bestehend aus Wasserstoff, Chlor, Brom, Jod, Fluor, (CH₂)₀₋₂-CN, CF₃, OCF₃, CONH₂, CONH(C₁-C₄-Alkyl), CON(C₁-C₄-Alkyl)(C₁-C₄-Alkyl), NHCHO, NHCONH₂, N(C₀-C₄-Alkylen)CONH₂, N(C₀-C₄-Alkylen)CONH(C₁-C₄-Alkyl), NHCOCH₃, NO₂, (CH₂)₀₋₂-OH, O-C₁-C₆-Alkyl, (CH₂)₀₋₂-O-C₁-C₄-Alkyl, O-C₀-C₄-Alkylen-Phenyl, Phenyl, C₁-C₆-Alkyl, C₂-C₆-Alkenyl und C₂-C₆-Alkinyl,
- B: ein aromatischer oder teilaromatischer C₆₋₁₀-Mono- oder -Bicyclus ist, der mit den Resten R⁶, R⁷, R⁸ und/oder R⁹ substituiert sein kann, wobei R⁶, R⁷, R⁸ und R⁹ unabhängig voneinander ausgewählt sind aus der Gruppe, bestehend aus Wasserstoff, Chlor, Brom, Jod, Fluor, (CH₂)₀₋₂-CN, CF₃, OCF₃, CONH₂, CONH(C₁-C₄-Alkyl), CON(C₁-C₄-Alkyl)(C₁-C₄-Alkyl), NHCHO, N(C₀₋₄-Alkylen)CONH(C₁-C₄-Alkyl), NHCOCH₃, NO₂, OH, O-C₁-C₄-Alkyl, (CH₂)₀₋₂-O-(CH₂)₀₋₃-CH₃, O-C₀-C₄-Alkylen-Phenyl, Phenyl, C₁-C₆-Alkyl, C₂-C₆-Alkenyl und C₂-C₆-Alkinyl,
- R¹: Wasserstoff, C₁-C₆-Alkyl, OH, O-(C₁-C₄-Alkyl), N(C₁-C₄-Alkyl)(C₁-C₄-Alkyl), CN, CONH₂, OCF₃, CF₃, Br, F, Cl, J, NO₂, NHCHO, NHCO(C₁-C₄-Alkyl) oder NHCONH₂ ist,
- R²: Wasserstoff, C₁-C₄-Alkyl, O-(C₁-C₄-Alkyl), Cl oder F ist,
- R³: ein Rest (W)-(X)-(Y)-Z ist, wobei
W C₁-C₄-Alkylen, (C₀-C₄-Alkylen)-O-(C₀-C₄-Alkylen) oder (C₀-C₄-Alkylen)-NR¹⁵-(C₀-C₄-Alkylen) ist, worin R¹⁵ Wasserstoff oder C₁-C₄-Alkyl ist,
X CO, SO₂, (C=NH) oder (C=N-CN) ist und
Y ein Rest ausgewählt aus der Gruppe, bestehend aus 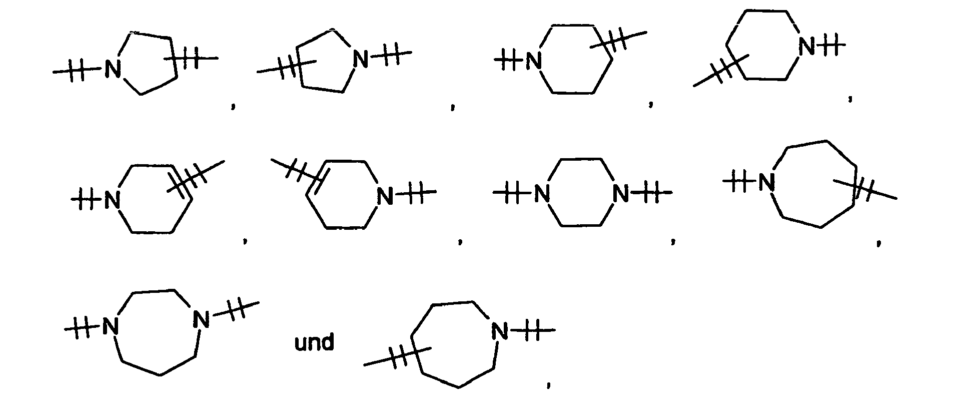 ist, wobei Y zusätzlich durch R¹⁰ und/oder R¹¹ substituiert sein kann, und
R¹⁰ Wasserstoff, C₁-C₆-Alkyl, C₂-C₆-Alkenyl, C₂-C₆-Alkinyl, OH, O-C₁-C₄-Alkyl, O-C₀-C₄-Alkylen-Phenyl, NH₂, NH(C₁-C₄-Alkyl) oder N(C₁-C₄-Alkyl)(C₁-C₄-Alkyl) ist,
R¹¹ Wasserstoff, C₁-C₆-Alkyl, C₂-C₆-Alkenyl, C₂-C₆-Alkinyl, OH, O-C₁-C₄-Alkyl, O-C₀-C₄-Alkylen-Phenyl, NH₂, NH(C₁-C₄-Alkyl) oder N(C₁-C₄-Alkyl)(C₁-C₄-Alkyl) ist, und
Z ein Rest ausgewählt aus der Gruppe, bestehend aus 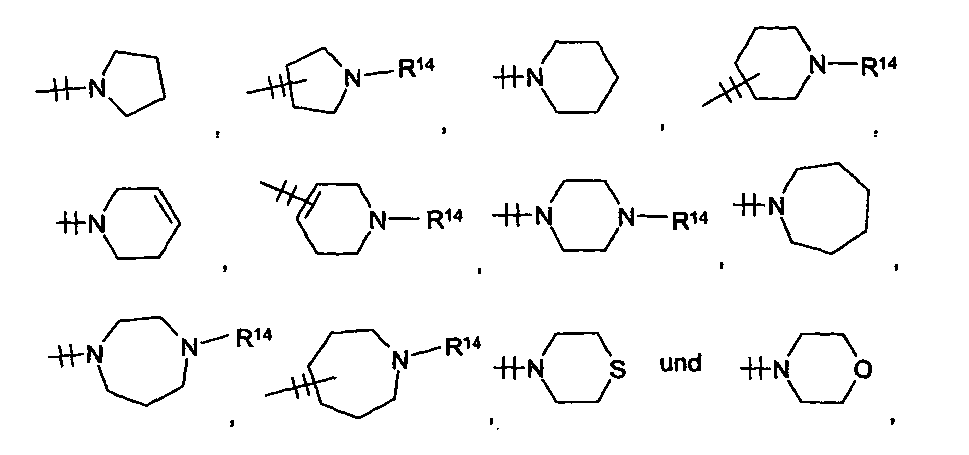 ist und Z zusätzlich durch R¹² und/oder R¹³ substituiert sein kann, wobei
R¹² Wasserstoff, C₁-C₆-Alkyl, C₂-C₆-Alkenyl, C₂-C₆-Alkinyl, OH, O-C₁-C₄-Alkyl, O-C₀-C₄-Alkylen-Phenyl, NH₂, NH(C₁-C₄-Alkyl) oder N(C₁-C₄-Alkyl)(C₁-C₄-Alkyl) ist,
R¹³ Wasserstoff, C₁-C₆-Alkyl, C₂-C₆-Alkenyl, C₂-C₆-Alkinyl, OH, O(C₁-C₄-Alkyl), O-C₀-C₄-Alkylen-Phenyl, NH₂, NH(C₁-C₄-Alkyl) oder N(C₁-C₄-Alkyl)(C₁-C₄-Alkyl) ist,
R¹⁴ Wasserstoff, C₁-C₆-Alkyl, C₂-C₆-Alkenyl, C₂-C₆-Alkinyl oder C₀-C₄-Alkylen-Phenyl ist, und
ihre tautomeren, enantiomeren und/oder diastereomeren Formen, sowie die physiologisch verträglichen Salze der vorstehend genannten Verbindung oder Verbindungen.

In einer bevorzugten Ausführungsform ist in den Verbindungen der allgemeinen Formel (I) A ein Phenyl-Ring, der mit maximal vier Resten R⁴ substituiert sein kann, und B ein Phenyl-Ring, der mit den Resten R⁶, R⁷, R⁸ und/oder R⁹ substituiert sein kann.

Weiterhin sind eine Verbindung oder Verbindungen der allgemeinen Formel (I) bevorzugt, worin
- A: ein Phenyl-Ring ist, der mit maximal zwei Resten R⁴ substituiert sein kann, die unabhängig voneinander ausgewählt sind aus der Gruppe, bestehend aus Wasserstoff, Chlor, O-C₁-C₄-Alkyl, (CH₂)₀₋₂-O-(CH₂)₀₋₂-CH₃ und C₁-C₆-Alkyl,
- B: ein Phenyl-Ring ist, der mit den Resten R⁶, R⁷, R⁸ und/oder R⁹ substituiert sein kann, wobei R⁶, R⁷, R⁸ und R⁹ unabhängig voneinander ausgewählt sind aus der Gruppe, bestehend aus Wasserstoff, Fluor, Chlor, O-C₁-C₄-Alkyl, (CH₂)₀₋₂-O-(CH₂)₀₋₂-CH₃ und C₁-C₆-Alkyl,

- R¹: Wasserstoff, CN, F, Cl, C₁₋₄-Alkyl, OH oder O-(C₁₋₄-Alkyl) ist,
- R²: Wasserstoff ist,
- R³: ein Rest (W)-(X)-(Y)-Z ist, wobei
W O, CH₂NH, NHCH₂, OCH₂, CH₂O oder NH ist,
X CO ist,
Y ein Rest ausgewählt aus der Gruppe, bestehend aus 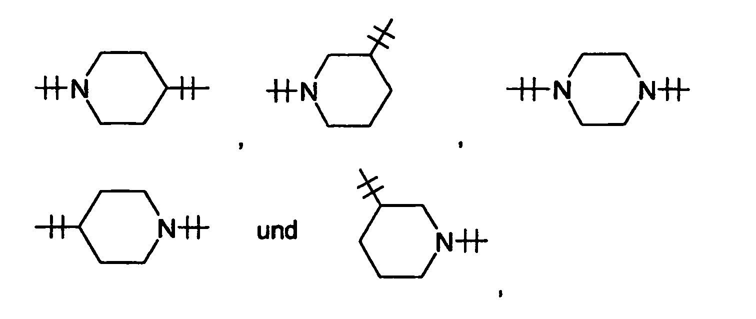 Ist,
Z ein Rest ausgewählt aus der Gruppe, bestehend aus 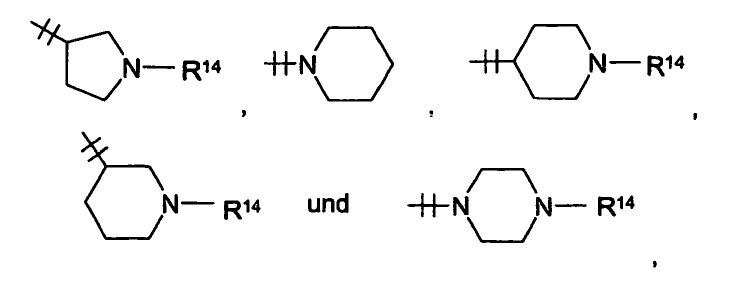 ist, wobei Z zusätzlich durch R¹² und/oder R¹³ substituiert sein kann, wobei
R¹² Wasserstoff oder C₁-C₄-Alkyl ist,
R¹³ Wasserstoff oder C₁-C₄-Alkyl ist und
R¹⁴ Wasserstoff, C₁-C₄-Alkyl, C₂-C₄-Alkenyl oder C₂-C₄-Alkinyl ist.

Besonders bevorzugt sind eine Verbindung oder Verbindungen der allgemeinen Formel (I), worin
- A: ein Phenyl-Ring ist, der mit maximal zwei Resten R⁴ substituiert sein kann, die unabhängig voneinander ausgewählt sind aus der Gruppe, bestehend aus Wasserstoff, Chlor, O-C₁-C₄-Alkyl, (CH₂)₀₋₂-O-(CH₂)₀₋₂-CH₃ und C₁-C₆-Alkyl,
- B: ein Phenyl-Ring ist, der mit den Resten R⁶ und/oder R⁷ substituiert sein kann, wobei R⁶ und R⁷ unabhängig voneinander ausgewählt sind aus der Gruppe, bestehend aus Wasserstoff, Fluor, Chlor, O-C₁-C₄-Alkyl und C₁-C₆-Alkyl,

- R¹: Wasserstoff, F, Cl, CH₃, CN, CH₂CH₃, OCH₃ oder OCH₂CH₃ ist,
- R²: Wasserstoff ist,
- R³: ein Rest (W)-(X)-(Y)-Z ist, wobei
W O, CH₂ oder NH ist,
X CO ist,
Y ein Rest ausgewählt aus der Gruppe 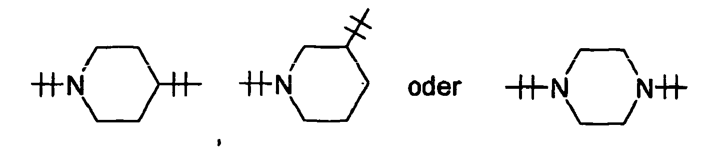 ist
und
Z ein Rest ausgewählt aus der Gruppe 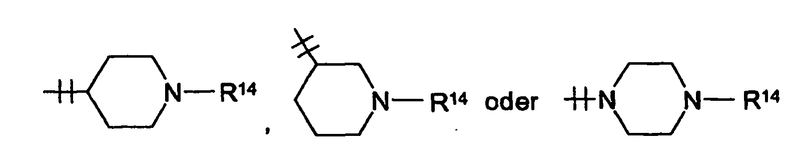 ist,
wobei Z durch R¹² und/oder R¹³ substituiert sein kann, wobei
R¹² Wasserstoff oder C₁-C₄-Alkyl ist,
R¹³ Wasserstoff oder C₁-C₄-Alkyl ist und
R¹⁴ Wasserstoff, C₁-C₄-Alkyl, C₂-C₄-Alkenyl oder C₂-C₄-Alkinyl ist.

Besonders bevorzugt sind weiterhin Verbindungen der allgemeinen Formel (I), worin
- A: ein Phenyl-Ring ist, der mit maximal zwei Resten R⁴ substituiert sein kann, die unabhängig voneinander ausgewählt sind aus der Gruppe, bestehend aus Wasserstoff, Chlor, O-C₁-C₄-Alkyl, (CH₂)₀₋₂-O-(CH₂)₀₋₂-CH₃ und C₁-C₆-Alkyl,
- B: ein Phenyl-Ring ist, der mit den Resten R⁶ und/oder R⁷ substituiert sein kann, wobei R⁶ und R⁷ unabhängig voneinander ausgewählt sind aus der Gruppe, bestehend aus Wasserstoff, Fluor, Chlor, O-C₁-C₄-Alkyl und C₁-C₆Alkyl,

- R¹: Cl, CH₃, CN, CH₂CH₃ oder OCH₃ ist,
- R²: Wasserstoff ist,
- R³: ein Rest (W)-(X)-(Y)-Z ist, wobei
W CH₂, O oder NH ist,
X CO ist,
Y ein Rest 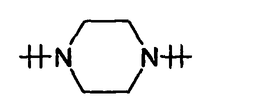 ist,
Z ein Rest 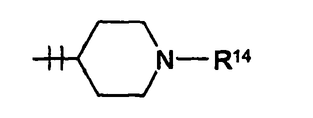 ist, wobei
R¹⁴ Wasserstoff, C₁-C₄-Alkyl, C₂-C₄-Alkenyl oder C₂-C₄-Alkinyl ist.

Ferner sind eine Verbindung oder Verbindungen der allgemeinen Formel (I) besonders bevorzugt, worin
- A: ein Phenyl-Ring ist, der mit maximal zwei Resten R⁴ substituiert sein kann, die unabhängig voneinander ausgewählt sind aus der Gruppe, bestehend aus Wasserstoff, Chlor, O-C₁-C₄-Alkyl und C₁-C₄-Alkyl,
- B: ein Phenyl-Ring ist, der mit den Resten R⁶ und/oder R⁷ substituiert ist, wobei R⁶ und R⁷ unabhängig voneinander ausgewählt sind aus der Gruppe, bestehend aus Wasserstoff, Fluor, Chlor, O-C₁-C₄-Alkyl und C₁-C₆-Alkyl,

- R¹: Wasserstoff, Cl, CH₃, CN, CH₂CH₃, OCH₃ oder OCH₂CH₃ ist,
- R²: Wasserstoff ist,
- R³: ein Rest (W)-(X)-(Y)-Z ist, wobei
W CH₂, O oder NH ist,
X CO ist,
Y ein Rest 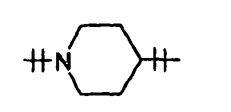 ist, und
Z ein Rest 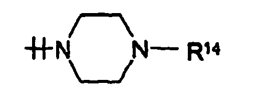 ist,
wobei
R¹⁴ Wasserstoff, C₁-C₄-Alkyl, C₂-C₄-Alkenyl oder C₂-C₄-Alkinyl ist.

Der Ausdruck "maximal vier Reste R⁴" in Verbindung mit der Variable A bedeutet die Gegenwart von keinem, einem, zwei, drei oder vier Substituenten an A, wobei die Reste R⁴ gleich oder verschieden sein können.

Der Ausdruck "maximal zwei Reste R⁴" in Verbindung mit der Variable A bedeutet die Gegenwart von keinem, einem, oder zwei Substituenten an A, wobei die Reste R⁴ gleich oder verschieden sein können.

Der Ausdruck "R¹⁰ und/oder R¹¹" in Verbindung mit der Variable Y bedeutet ein oder zwei gleiche oder verschiedene Reste ausgewählt aus der Gruppe bestehend aus R¹⁰ und R¹¹.

Der Ausdruck "R¹² und/oder R¹³" in Verbindung mit der Variable Z bedeutet ein oder zwei gleiche oder verschiedene Reste ausgewählt aus der Gruppe bestehend aus R¹² und R¹³.

Die Variablen, die die erfindungsgemäßen Verbindungen der Formel (I) kennzeichnen, besitzen unabhängig voneinander die folgenden bevorzugten Bedeutungen.

A ist vorzugsweise ein Phenyl-Ring, der mit maximal vier Resten R⁴ substituiert sein kann, noch bevorzugter ein Phenyl-Ring, der mit maximal zwei Resten R⁴ substituiert sein kann. In einer Ausführungsform ist A unsubstituiertes Phenyl.

In einer anderen Ausführungsform ist A mit einem Substituenten substituiert. Wenn A substituiert ist, werden die Substituenten R⁴ unabhängig voneinander ausgewählt aus der Gruppe, bestehend aus Wasserstoff, Chlor, Brom, Jod, Fluor, (CH₂)₀₋₂-CN, CF₃, OCF₃, CONH₂, CONH(C₁-C₄-Alkyl), CON(C₁-C₄-Alkyl)(C₁-C₄-Alkyl), NHCHO, NHCONH₂, N(C₀-C₄-Alkylen)CONH₂, N(C₀-C₄-Alkylen)CONH(C₁-C₄-Alkyl), NHCOCH₃, NO₂, (CH₂)₀₋₂OH, O-C₁-C₆-Alkyl, (CH₂)₀₋₂-O-C₁-C₄-Alkyl, O-C₀-C₄-Alkylen-Phenyl, Phenyl, C₁-C₆-Alkyl, C₂-C₆-Alkenyl und C₂-C₆-Alkinyl, vorzugsweise Wasserstoff, Chlor, O-C₁-C₄-Alkyl, (CH₂)₀₋₂-O-(CH₂)₀₋₂-CH₃ und C₁-C₆-Alkyl, noch bevorzugter Wasserstoff, Chlor, O-C₁-C₄-Alkyl und C₁-C₄-Alkyl. Wenn es sich bei A um einen Phenylring handelt, befindet sich ein Substituent vorzugsweise in der 2-Stellung, wobei weitere Substituenten in den Stellungen 3, 4 oder 5 sein können, noch bevorzugter befindet sich ein Substituent in 2-Stellung und ein weiterer in 3-, 4- oder 5-Stellung, am meisten bevorzugt befindet sich ein Substituent in 2-Stellung.

B ist vorzugsweise ein Phenyl-Ring, der mit den Resten R⁶, R⁷, R⁸ und/oder R⁹ substituiert sein kann. Bevorzugt ist B mit keinem, einem, zwei, drei oder vier gleichen oder verschiedenen Resten ausgewählt aus R⁶, R⁷, R⁸ und R⁹ substituiert. Noch bevorzugter ist B ein Phenyl-Ring, der mit den Resten R⁶ und/oder R⁷ substituiert sein kann. In einer Ausführungsform ist B unsubstituiertes Phenyl. In einer anderen Ausführungsform ist B mit einem Substituenten R⁶ substituiert. Wenn B substituiert ist, werden die Substituenten R⁶, R⁷, R⁸ und/oder R⁹ unabhängig voneinander ausgewählt aus der Gruppe, bestehend aus Wasserstoff, Chlor, Brom, Jod, Fluor, (CH₂)₀₋₂-CN, CF₃, OCF₃, CONH₂, CONH(C₁-C₄-Alkyl), CON(C₁-C₄-Alkyl)(C₁-C₄-Alkyl), NHCHO, N(C₀₋₄-Alkylen)CONH(C₁-C₄-Alkyl), NHCOCH₃, NO₂, OH, O-C₁-C₄-Alkyl, (CH₂)₀₋₂-O-(CH₂)₀₋₃-CH₃, O-C₀-C₄-Alkylen-Phenyl, Phenyl, C₁-C₆-Alkyl, C₂-C₆-Alkenyl und C₂-C₆-Alkinyl, vorzugsweise Wasserstoff, Fluor, Chlor, O-C₁-C₄-Alkyl, (CH₂)₀₋₂-O-(CH₂)₀₋₂-CH₃ und C₁-C₆-Alkyl, noch bevorzugter Wasserstoff, Fluor, Chlor, O-C₁-C₄-Alkyl und C₁-C₆-Alkyl. Wenn es sich bei B um einen Phenyl-Ring handelt, befinden sich die Substituenten vorzugsweise in 2-, 3-, 4-, 5- und/oder 6-Stellung, bevorzugt sind maximal 4 Substituenten, von denen zwei Substituenten in 2- und 4-Stellung oder ein Substituent entweder in 2- oder 4-Stellung ist, noch bevorzugter sind zwei Substituenten in 2- und 4-Stellung oder ein Substituent ist entweder in 2- oder 4-Stellung.
R¹ ist vorzugsweise Wasserstoff, CN, F, Cl, C₁₋₄-Alkyl oder O-(C₁₋₄-Alkyl), noch bevorzugter Wasserstoff, F, Cl, CH₃, CN, CH₂CH₃, OCH₃ oder OCH₂CH₃, am meisten bevorzugt Cl, CH₃, CN, CH₂CH₃ oder OCH₃. R¹ befindet sich vorzugsweise in der 4-, 5- oder 6-Stellung, noch bevorzugter in der 4- oder 5-Stellung, am meisten bevorzugt in der 5-Stellung.

R² ist vorzugsweise Wasserstoff.

R³ ist ein Rest (W)-(X)-(Y)-Z, wobei sich bevorzugte Definitionen von R³ aus den Definitionen von W, X, Y und Z ergeben, bei denen wenigstens eine der Definitionen von W, X, Y und Z eine beliebige bevorzugte Ausführungsform, wie nachstehend erläutert, darstellt. Vorzugsweise stellen alle Definitionen von W, X, Y und Z eine beliebige bevorzugte Ausführungsform dar. Am meisten bevorzugt ist R³ ein Rest (W)-(X)-(Y)-Z, wobei alle Definitionen von W, X, Y und Z die jeweils am meisten bevorzugte Ausführungsform darstellen.

W ist vorzugsweise O, (C₁-C₄-Alkylen)NH, NH(C₁-C₄-Alkylen), O(C₁-C₄-Alkylen), (C₁-C₄-Alkylen)O oder NH, noch bevorzugter O, CH₂NH, NHCH₂, OCH₂, CH₂O oder NH, am meisten bevorzugt CH₂, O oder NH.

X ist vorzugsweise CO oder SO₂, am meisten bevorzugt CO. Y ist vorzugsweise 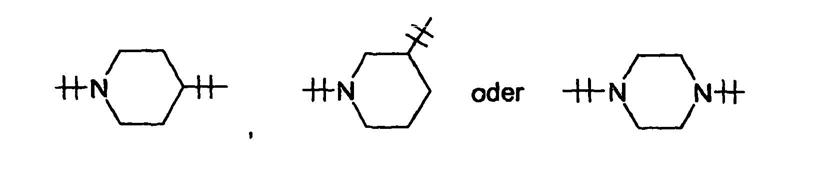 und am meisten bevorzugt

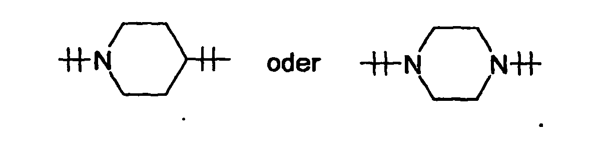

R¹⁰ ist vorzugsweise Wasserstoff oder C₁-C₄-Alkyl, wobei die Alkyl-Gruppe in 2-, 3-, 5- oder 6-Stellung stehen kann, bevorzugt Wasserstoff oder eine C₁-C₄-Alkyl-Gruppe, die in 2-Stellung steht und besonders bevorzugt Wasserstoff.

R¹¹ ist vorzugsweise Wasserstoff oder C₁-C₄-Alkyl, wobei die Alkyl-Gruppe in 2-, 3-, 5- oder 6-Stellung stehen kann, bevorzugt Wasserstoff oder eine C₁-C₄-Alkyl-Gruppe, die in 2-Stellung steht und besonders bevorzugt Wasserstoff.

Z ist vorzugsweise

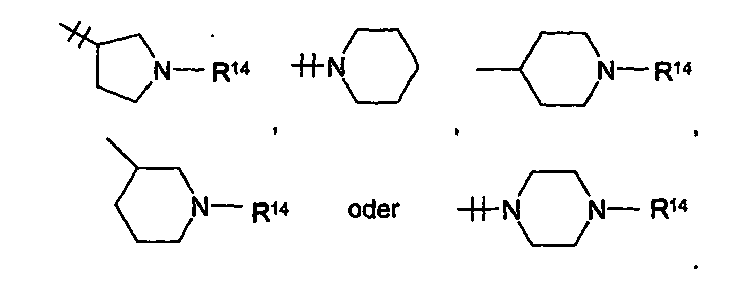

Noch bevorzugter ist Z

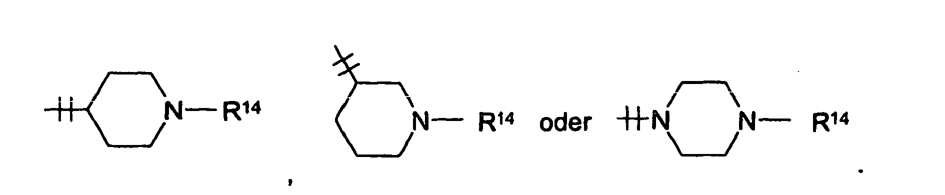

In einer Ausführungsform ist Z

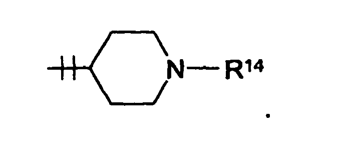

In einer anderen Ausführungsform ist Z

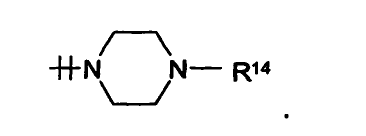

R¹² ist vorzugsweise Wasserstoff oder C₁-C₄-Alkyl, wobei die Alkyl-Gruppe in 2-, 3-, 4- oder 6-Stellung stehen kann, bevorzugt Wasserstoff oder eine C₁-C₄-Alkyl-Gruppe, die in 2-Stellung steht und besonders bevorzugt Wasserstoff.

R¹³ ist vorzugsweise Wasserstoff oder C₁-C₄-Alkyl, wobei die Alkyl-Gruppe in 2-, 3-, 4- oder 6-oder Stellung stehen kann, bevorzugt Wasserstoff oder eine C₁-C₄-Alkyl-Gruppe, die in 2-Stellung steht und besonders bevorzugt Wasserstoff.

R¹⁴ ist vorzugsweise Wasserstoff, C₁-C₄-Alkyl, C₂-C₄-Alkenyl oder C₂-C₄-Alkinyl, noch bevorzugter Wasserstoff, CH₃, CH₂CH₃, CH₂CH₂CH₃ oder CH(CH₃)₂, am meisten bevorzugt CH₃.

R¹⁵ ist vorzugsweise Wasserstoff oder C₁-C₄-Alkyl, bevorzugt Wasserstoff, CH₃, CH₂CH₃ oder CH₂CH₂CH₃, am meisten bevorzugt Wasserstoff oder CH₃.

Daraus ergeben sich folgende besonders bevorzugte Gruppen für R³:

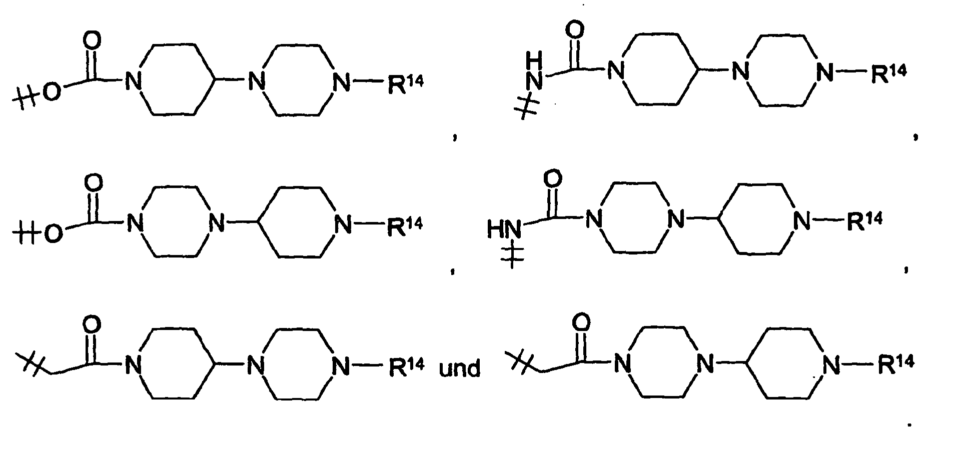

Jede dieser bevorzugten Definitionen einer Variablen kann mit beliebigen Definitionen der restlichen Variablen kombiniert werden.

Ebenfalls besonders bevorzugt sind folgende Verbindungen:
4-(4-Methyl-piperidin-1-yl)-piperazin-1-karbonsäure-[1-benzolsulfonyl-5-chlor-2-oxo-3-(2-methoxy-phenyl)-2,3-dihydro-1H-indol-3-yl]-ester
4-(1-Methyl-piperidin-4-yl)-piperazin-1-karbonsäure-[1-(2,4-dimethoxybenzolsulfonyl)-5-methoxy-2-oxo-3-(2-propoxyphenyl)-2,3-dihydro-1H-indol-3-yl]-ester
4-(1-Methyl-piperidin-4-yl)-piperazin-1-karbonsäure-[1-(2,4-dimethoxybenzolsulfonyl)-5-chlor-2-oxo-3-(2-methoxyphenyl)-2,3-dihydro-1H-indol-3-yl]-amid-dihydrochlorid
4-(1-Methyl-piperidin-4-yl)-piperazin-1-karbonsäure-[1-benzolsulfonyl-5-chlor-2-oxo-3-phenyl-2,3-dihydro-1H-indol-3-yl-ester]-dihydrochlorid
4-(4-Methyl-piperazin-1-yl)-piperidin-1-karbonsäure-[1-benzolsulfonyl-5-chlor-2-oxo-3-phenyl-2,3-dihydro-1H-indol-3-yl]-ester
4-(1-Methyl-piperidin-4-yl)-piperazin-1-karbonsäure-[1-(2,4-dimethoxybenzolsulfonyl)-5-methoxy-2-oxo-3-(2-methoxyphenyl)-2,3-dihydro-1H-indol-3-yl]-ester-dihydrochlorid
4-(4-Methyl-piperazin-1-yl)-piperidin-1-karbonsäure-[1-benzolsulfonyl-5-methyl-2-oxo-3-phenyl-2,3-dihydro-1H-indol-3-yl]-ester-dihydrochlorid
4-(1-Methyl-piperidin-4-yl)-piperazin-1-karbonsäure-[1-(2,4-dimethoxybenzolsulfonyl)-5-methoxy-2-oxo-3-(2-methylphenyl)-2,3-dihydro-1H-indol-3-yl]-ester-dihydrochlorid
4-(Piperidin-4-yl)-piperazin-1-karbonsäure-[1-(2,4-dimethoxybenzolsulfonyl)-5-methoxy-2-oxo-3-(2-methoxyphenyl)-2,3-dihydro-1H-indol-3-yl]-ester
4-( 1-Methyl-piperidin-4-yl)-piperazin-1-karbonsäure-[1-(2,4-dimethoxybenzolsulfonyl)5-fluoro-2-oxo-3-(2-methoxyphenyl)-2,3-dihydro-1H-indol-3-yl]-ester
4-(1-Methyl-piperidin-4-yl)-piperazin-1-karbonsäure-[1-(2-cyano-benzolsulfonyl)-5-methoxy-2-oxo-3-(2-methylphenyl)-2,3-dihydro-1H-indol-3-yl]-ester-dihydrochlorid
4-(1-Methyl-piperidin-4-yl)-piperazin-1-karbonsäure-[1-(4-brom-2-methylbenzolsulfonyl)-5-methoxy-2-oxo-3-(2-methylphenyl)-2,3-dihydro-1H-indol-3-yl]-ester-dihydrochlorid
4-(1-Benzyl-piperidin-4-yl)-piperazin-1-karbonsäure-[1-(2,4-dimethoxybenzolsulfonyl)-5-methoxy-2-oxo-3-(2-methoxyphenyl)-2,3-dihydro-1H-indol-3-yl]-ester
4-(1-Methyl-piperidin-4-yl)-piperazin-1-karbonsäure-[1-(2,4-dimethoxybenzolsulfonyl)-5-methyl-2-oxo-3-(2-methylphenyl)-2,3-dihydro-1H-indol-3-yl]-ester-dihydrochlorid
4-(1-Methyl-piperidin-4-yl)-piperazin-1-karbonsäure-[1-(2,4-dimethoxybenzolsulfonyl)-5-cyano-2-oxo-3-(2-methylphenyl)-2,3-dihydro-1H-indol-3-yl]-ester-dihydrochlorid
4-(1-Methyl-piperidin-4-yl)-piperazin-1-karbonsäure-[1-(2,4-dimethoxybenzolsulfonyl)-5-methoxy-2-oxo-3-(2-methoxyphenyl)-2,3-dihydro-1H-indol-3-yl]-amid-dihydrochlorid
4-(1-Methyl-piperidin-4-yl)-piperazin-1-karbonsäure-[1-(2,4-dimethoxybenzolsulfonyl)-5-methyl-2-oxo-3-(2-methoxyphenyl)-2,3-dihydro-1 H-indol-3-yl] ester-dihydrochlorid
4-(1-Methyl-piperidin-4-yl)-piperazin-1-karbonsäure-[1-(2,4-dimethoxybenzolsulfonyl)-5-methoxy-2-oxo-3-(2-isopropylphenyl)-2,3-dihydro-1H-indol-3-yl]-ester-dihydrochlorid
4-(1-Methyl-piperidin-4-yl)-piperazin-1-karbonsäure-[1-(4-methoxybenzolsulfonyl)-5-methoxy-2-oxo-3-(2-isopropylphenyl)-2,3-dihydro-1 H-indol-3-yl]-ester dihydrochlorid
4-(1-Methyl-piperidin-4-ylrpiperazin-1-karbonsäure-[1-(3,4-dimethoxybenzol-sulfonyl)-5-methoxy-2-oxo-3-(2-isopropylphenyl)-2,3-dihydro-1H-indol-3-yl]-ester-dihydrochlorid
4-(1-Methyl-piperidin-4-yl)-piperazin-1-karbonsäure-[1-(4-isopropyl-benzolsulfonyl)-5-methoxy-2-oxo-3-(2-isopropylphenyl)-2,3-dihydro-1H-indol-3-yl]-ester-dihydrochlorid
4-(-1-Methyl-piperidin-4-yl)-piperazin-1-karbonsäure-[1-(2-cyano-benzol sulfonyl)-5-methoxy-2-oxo-3-(2-isopropylphenyl)-2,3-dihydro-1H-indol-3-yl]-ester-dihydrochlorid
4-(1-Methyl-piperidin-4-yl)-piperazin-1-karbonsäure-[1-(2,4-dimethoxybenzolsulfonyl)-2-oxo-3-(2-methoxyphenyl)-2,3-dihydro-1H-indol-3-yl]-ester-dihydrochlorid
4-(1-Methyl-piperidin-4-yl)-piperazin-1-karbonsäure-[1-(2,4-dimethoxybenzolsulfonyl)-5-methoxy-2-oxo-3-(2-isopropoxyphenyl)-2,3-dihydro-1H-indol)-3-yl]-ester-dihydrochlorid
4-(1-Methyl-piperidin-4-yl)-piperazin-1-karbonsäure-[1-(2-cyano-benzolsulfonyl)-5-methoxy-2-oxo-3-(2-isopropoxyphenyl)-2,3-dihydro-1H-indol-3-yl]-ester-dihydrochlorid
4-(1 -Methyl-piperidin-4-yl)-piperazin-1-karbonsäure-[1-(2-methoxy-4-methylbenzol)-sulfonyl-5-methoxy-2-oxo-3-(2-isopropoxyphenyl)-2,3-dihydro-1 H-indol-3-yl]-ester-dihydrochlorid
4-(1-Methyl-piperidin-4-yl)-piperazin-1-karbonsäure-[1-(2-methyl-benzolsulfonyl)-5-methoxy-2-oxo-3-(2-isopropylphenyl)-2,3-dihydro-1H-indol-3-yl]-ester-dihydrochlorid
4-(11-Methyl-piperidin-4-yl)-piperazin-1-karbonsäure-[1-(2-methyl-benzolsulfonyl)-5-methoxy2-oxo-3-(2-isopropoxyphenyl)-2,3-dihydro-1H-indol-3-yl]-ester-dihydrochlorid
4-(1-Methyl-piperidin-4-yl)-piperazin-1-karbonsäure-[1-(2-methyl-benzolsulfonyl)-5-methoxy-2-oxo-3-(2-ethoxyphenyl)-2,3-dihydro-1H-indol-3-yl]-ester-dihydrochlorid
4-(1-Methyl-piperidin-4-yl)-piperazin-1-karbonsäure-[1-(2,4-dimethoxybenzolsutfonyl)-5-methoxy-2-oxo-3-(2-ethoxyphenyl)-2,3-dihydro-1H-indol-3-yl]-ester-dihydrochlorid
4-(1-Methyl-piperidin-4-yl)-piperazin-1-karbonsäure-[1-(2-methyl-benzolsulfonyl)-5-methoxy-2-oxo-3-(2-methoxyphenyl)-2,3-dihydro-1H-indol-3-yl]-ester-dihydrochlorid
4-(1-Methyl-piperidin-4-yl}-piperazin-1-karbonsäure-[1-(3-cyano-benzolsulfonyl)-5-methoxy-2-oxo-3-(2-ethoxyphenyl)-2,3-dihydro-1H-indol-3-yl]-ester-dihydrochlorid
4-(1-Methyl-piperidin-4-yl)-piperazin-1-karbonsäure-[1-(4-ethyl-benzolsulfonyl)-5-methoxy-2-oxo-3-(2-ethoxyphenyl)-2,3-dihydro-1H-indol-3-yl]-ester-dihydrochlorid
4-(1-Methyl-piperidin-4-yl)-piperazin-1-karbonsäure-[1-(2-methyl-benzolsulfonyl)-5-methoxy-2-oxo-3-(2-propoxyphenyl)-2,3-dihydro-1H-indol-3-yl]-ester-dihydrochlorid
4-(1-Methyl-piperidin-4-yl)-piperazin-1-karbonsäure-[1-(2-methoxy-4-methylbenzolsulfonyl)-5-methoxy-2-oxo-3-(2-propoxyphenyl)-2,3-dihydro-1H-indol-3-yl]-ester-dihydrochlorid
4-(1-Methy)-pipendin-4-yl)-piperazin-1-karbonsäure-[1-benzolsulfonyl-5-methyl-2-oxo-3-phenyl-2,3-dihydro-1H-indol-3-yl]-ester-dihydrochlorid
4-(1-Methyl-piperidin-4-yl)-piperazin-1-karbonsäure-[1-(2,4-dimethoxybenzolsulfonyl)-5-methoxy-2-oxo-3-(2-ethylphenyl)-2,3-dihydro-1H-indol-3-yl]-ester dihydrochlorid
4-(1-Methyl-piperidin-4-yl)-piperazin-1-karbonsäure-[1-(2,4-dimethoxybenzolsulfonyl)-5-methoxy-2-oxo-3-(2,5-dimethoxyphenyl)-2,3-dihydro-1H-indol-3-yl]-ester-dihydrochlorid
4-(1-Methyl-piperidin-4-yl)-piperazin-1-karbonsäure-[1-(2-methyl-benzolsulfonyl)-5-methoxy-2-oxo-3-(2,5-dimethoxyphenyl)-2,3-dihydro-1H-indol-3-yl]-ester-dihydrochlorid
4-(1-Methyl-piperidin-4-yl)-piperazin-1-karbonsäure-[1-(benzolsulfonyl]-5-methoxy-2-oxo-3-(2,5-dimethoxyphenyl)-2,3-dihydro-1H-indol-3-yl]-ester-dihydrochlorid
-(4-Methyl-piperidin-1-yl)-piperazin-1-karbonsäure-[1-benzolsulfonyl-5-chlor-2-oxo-3-(2-methoxy-phenyl)-2,3-dihydro-1H-indol-3-yl] ester
4-(1-Methyl-piperidin-4-yl)-piperazin-1-karbonsäure-[1-(2,4-dimethoxybenzolsulfonyl)-5-methoxy-2-oxo-3-(2-propoxyphenynyl)-2,3-dihydro-1H-indol-3-yl] ester
4-(1 -Methyl-piperidin-4-yl)-piperazin-1-carbonsäure-[1-(2,4-dimethoxybenzolsulfonyl)-5-chlor-2-oxo-3-(2-methoxyphenyl)-2,3-dihydro-1 H-indol-3-yl] amid dihydrochlorid
4-(1-Methyl-piperidin-4-yl)-piperazin-1-karbonsäure-[1-benzolsulfonyl-5-chlor-2-oxo-3-phenyl-2,3-dihydro-1 H-indol-3-yl-ester-dihydrochlorid
4-(4-Methyl-piperazin-1-yl)-piperidin-1-karbonsäure-[1-benzolsulfonyl-5-chlor-2-oxo-3-phenyl-2,3-dihydro-1H-indol-3-yl] ester
4-(1-Methyl-piperidin-4-yl)-piperazin-1-karbonsäure-[1-(2,4-dimethoxybenzolsulfonyl)-5-methoxy-2-oxo-3-(2-methoxyphenyl)-2,3-dihydro-1 H-indol-3-yl] ester dihydrochlorid
4-(4-Methyl-piperazin-1-yl)-piperidin-1-karbonsäure-[1-benzolsulfonyl-5-methyl-2-oxo-3-phenyl-2,3-dihydro-1H-indol-3-yl] ester dihydrochlorid
4-(1-Methyl-piperidin-4-yl)-piperazin-1-karbonsäure-[1-(2,4-dimethoxybenzolsulfonyl)-5-methoxy-2-oxo-3-(2-methylphenyl)2,3-dihydro-1H-indol-3-yl] ester dihydrochlorid
4-(Piperidin-4-yl)-piperazin-1-karbonsäure-[1-(2,4-dimethoxybenzolsulfonyl)-5-methoxy-2-oxo-3-(2-methoxyphenyl)-2,3-dihydro-1H-indol-3-yl] ester
4-(1-Methyl-piperidin-4-yl)-piperazin-1-karbonsäure-[1-(2,4-dimethoxy benzolsulfonyl)-5-fluoro-2-oxo-3-(2-methoxyphenyl)-2,3-dihydro-1H-indol-3-yl] ester
4-(1-Methyl-piperidin-4-yl)-piperazin-1-karbonsäure-[1-(2-cyano-benzolsulfonyl)-5-methoxy-2-oxo-3-(2-methylphenyl)-2,3-dihydro-1H-indol-3-yl]-ester dihydrochlorid
4-(1-Methyl-piperidin-4-yl)-piperazin-1-karbonsäure-[1-(4-brom-2-methyl-benzolsulfonyl)-5-methoxy-2-oxo-3-(2-methylphenyl)-2,3-dihydro-1 H-indol-3-yl]-ester-dihydrochlorid
4-(1-Benzyl-piperidin-4-yl)-piperazin-1-karbonsäure-[1-(2,4-dimethoxybenzolsulfonyl)-5-methoxy-2-oxo-3-(2-methoxyphenyl)-2,3-dihydro-1H-indol-3-yl]-ester
4-(1-Methyl-piperidin-4-yl)-piperazin-1-karbonsäure-[1-(2,4-dimethoxybenzolsulfonyl)-5-methyl-2-oxo-3-(2-methylphenyl)-2,3-dihydro-1H-indol-3-yl]-ester-dihydrochlorid
4-(1-Methyl-piperidin-4-yl)-piperazin-1-karbonsäure-[1-(2,4-dimethoxybenzolsulfonyl)-5-cyano-2-oxo-3-(2-methylphenyl)-2,3-dihydro-1H-indol-3-yl]-ester-dihydrochlorid
4-(1-Methyl-piperidin-4-yl)-piperazin-1-karbonsäure-[1-(2,4-dimethoxybenzolsulfonyl)-5-methoxy-2-oxo-3-(2-methoxyphenyl)-2,3-dihydro-1H-indol-3-yl]-amid dihydrochlorid
4-(1-Methyl-piperidin-4-yl)-piperazin-1-karbonsäure-[1-(2,4-dimethoxybenzolsulfonyl)-5-methyl-2-oxo-3-(2-methoxyphenyl)-2,3-dihydro-1H-indol-3-yl]-ester-dihydrochlorid
4-(1-Methyl-piperidin-4-yl)piperazin-1-karbonsäure-[1-(2,4-dimethoxybenzolsulfonyl)-5-methoxy-2-oxo-3-(2-isopropylphenyl)-2,3-dihydro-1 H-indol-3-yl]-ester-dihydrochlorid
4-(1-Methyl-piperidin-4-yl)-piperazin-1-karbonsäure-[1-(4-methoxybenzolsulfonyl)-5-methoxy-2-oxo-3-(2-isopropylphenyl)-2,3-dihydro-1H-indol-3-yl]-ester-dihydrochlorid
4-(1-Methyl-piperidin-4-yl)-piperazin-1-karbonsäure-[1-(3,4-dimethoxybenzol-sulfonyl)-5-methoxy-2-oxo-3-(2-isopropylphenyl)-2,3-dihydro-1 H-indol-3-yl]-ester-dihydrochlorid
4-(1-Methyl-piperidin-4-yl)-piperazin-1-karbonsäure-[1-(4-isopropyl-benzolsulfonyl)-5-methoxy-2-oxo-3-(2-isopropylphenyl)-2,3-dihydro-1H-indol-3-yl]-ester-dihydrochlorid
4-(1-Methyl-piperidin-4-yl)-piperazin-1-karbonsäure-[1-(2-cyano-benzolsulfonyl)-5-methoxy-2-oxo-3-(2-isopropylphenyl)-2,3-dihydro-1H-indol-3-yl]-ester-dihydrochlorid
4-(1-Methyl-piperidin-4-yl)-piperazin-1-karbonsäure-[1-(2,4-dimethoxy benzolsulfonyl)-2-oxo-3-(2-methoxyphenyl)-2,3-dihydro-1H-indol-3-yl]-ester-dihydrochlorid
4-(-Methyl-piperidin-4-yl)-piperazin-1-karbonsäure-[1-(2,4-dimethoxybenzolsulfonyl)-5-methoxy-2-oxo-3-(2-isopropoxyphenyl)-2,3-dihydro-1H-indol-3-yl]-ester-dihydrochlorid
4-(1-Methyl-piperidin-4-yl)-piperazin-1-karbonsäure-[1-(2-cyano-benzolsulfonyl)-5-methoxy-2-oxo-3-(2-Isopropoxyphenyl)-2,3-dihydro-1H-indol-3-yl]-ester dihydrochlorid
4-(1 -Methyl-piperidin-4-yl)-piperazin-1-karbonsäure-[1-(2-methoxy-4-methylbenzol)-suffonyl-5-methoxy-2-oxo-3-(2-isopropoxyphenyl)-2,3-dihydro-1H-indol-3-yl]-ester-dihydrochlorid
4-(1-Methyl-piperidin-4-yl)-piperazin-1-karbonsäure-[1-(2-methyl-benzolsulfonyl)-5-methoxy-2-oxo-3-(2-isopropylphenyl)-2,3-dihydro-1H-indol-3-yl)-ester-dihydrochlorid
4-(1-Methyl-piperidin-4-yl)-piperazin-1-karbonsäure-{1-(2-methyl-benzolsulfonyl)-5-methoxy-2-oxo-3-(2-isopropoxyphenyl)2,3-dihydro-1H-indol-3-yl] ester dihydrochlorid
4-(1-Methyl-piperidin-4-yl)-piperazin-1-karbonsäure-[1-(2-methyl-benzolsulfonyl)-5-methoxy-2-oxo-3-(2-ethoxyphenyl)-2,3-dihydro-1H-indol-3-yl]-ester-dihydrochlorid
4-(1-Methyl-piperidin-4-yl)-piperazin-1-karbonsäure-[1-(2,4-dimethoxybenzolsulfonyl)-5-methoxy-2-oxo-3-(2-ethoxyphenyl)-2,3-dihydro-1H-indol-3-yl]-ester-dihydrochlorid
4-(1-Methyl-piperidin-4-yl)-piperazin-1-karbonsäure-[1-(2-methyl-benzolsulfonyl)-5-methoxy-2-oxo-3-(2-methoxyphenyl)-2,3-dihydro-1H-indol-3-yl]-ester-dihydrochlorid
4-(1 -Methyl-piperidin-4-yl)-piperazin-1-karbonsäure-[1-(3-cyano-benzolsulfonyl)-5-methoxy-2-oxo-3-(2-ethoxyphenyl)-2,3-dihydro-1H-indol-3-yl]-ester-dihydrochlorid
4-(1-Methyl-piperidin-4-yl)-piperazin-1-karbonsäure-[1-(4-ethylbenzolsulfonyl)-5-methoxy-2-oxo-3-(2-ethoxyphenyl)-2,3-dihydro-1H-indol-3-yl]-ester-dihydrochlorid
4-(1 -Methyl-piperidin-4-yl)-piperazin-1-karbonsäure-[1-(2-methyl-benzolsulfonyl)-5-methoxy-2-oxo-3-(2-propoxyphenyl)-2,3-dihydro-1 H-indol-3-yl]-ester-dihydrochlorid
4-(1-Methyl-piperidin-4-yl)-piperazin-1-karbonsäure-[1-(2-methoxy-4-methylbenzolsulfonyl)-5-methoxy 2-oxo-3-(2-propoxyphenyl)-2,3-dihydro-1H-indol-3-yl] ester dihydrochlorid
4-(1 1-Methyl-piperidin-4-yl)-piperazin-1-karbonsäure-[1-benzolsulfonyl-5-methyl-2-oxo-3-phenyl-2,3-dihydro-1H-indol-3-yl]-ester-dihydrochlorid
4-(1-Methyl-piperidin-4-yl)-piperazin-1-karbonsäure-[1-(2,4-dimethoxy benzolsulfonyl)-5-methoxy-2-oxo-3-(2-ethylphenyl)-2,3-dihydro-1H-indol-3-yl]-ester-dihydrochlorid
4-(1-Methyl-piperidin-4-yl)-piperazin-1-karbonsäure-[1-(2,4-dimethoxybenzolsulfonyl)-5-methoxy-2-oxo-3-(2,5-dimethoxyphenyl)-2,3-dihydro-1H-indol-3-yl]-ester-dihydrochlorid
4-(1-Methyl-piperidin-4-yl)-piperazin-1-karbonsäure-[1-(2-methyl-benzolsulfonyl)-5-methoxy 2-oxo-3-(2,5-dimethoxyphenyl)-2,3-dihydro-1H-indol-3-yl]-ester-dihydrochlorid
4-(1-Methyl-piperidin-4-yl)-piperazin-1-karbonsäure-[1-(benzolsulfonyl]-5-methoxy-2-oxo-3-(2,5-dimethoxyphenyl)-2,3-dihydro-1 H-indol-3-yl]-ester dihydrochlorid
4-(1-Methyl-piperidin-4-yl)-piperazin-1-karbonsäure-[1-(4-cyano-1-benzolsulfonyl]-5-chlor-2-oxo-3-(2-ethoxyphenyl)-2,3-dihydro-1H-indol-3-yl]-ester
4-(1-Methyl-piperidin-4-yl)-piperazin-1-karbonsäure-[1-(2-methoxy-1-benzolsuffonyl]-5-chlor-2-oxo-3-(2-ethoxyphenyl)-2,3-dihydro-1H-indol-3-yl]-ester
4-(1-Methyl-piperidin-4-yl)-piperazin-1-karbonsäure-[1-(2-methoxy-4-methyl-1-benzolsulfonyl]-5-chlor-2-oxo-3-(2-ethoxyphenyl)-2,3-dihydro-1H-indol-3-yl]-ester
4-(1-Methyl-piperidin-4-yl)-piperazin-1-karbonsäure-[1-benzolsulfonyl-5-chlor-2-oxo-3-(2-ethoxyphenyl)-2,3-dihydro-1H-indol-3-yl]-ester
4-(1-Methyl-piperidin-4-yl)-piperazin-1-karbonsäure-[1-(2,4-dimethoxy-1-benzolsulfonyl]-5-chlor-2-oxo-3-(2-ethoxyphenyl)-2,3-dihydro-1H-indol-3-yl]-ester
4-(1-Methyl-piperidin-4-yl)-piperazin-1-karbonsäure-[1-(2,4-dimethoxy-1-benzolsulfonyl]-5-chlor-2-oxo-3-(2-methoxyphenyl)-2,3-dihydro-1H-indol-3-yl]-ester-dihydrochlorid
4-(1-Methyl-piperidin-4-yl)-piperazin-1-karbonsäure-[1-(2-methoxy-4-methyl-1-benzolsulfonyl]-5-chlor-2-oxo-3-(2-ethoxyphenyl)-2,3-dihydro-1H-indol-3-yl]-amid
4-(1-Methyl-piperidin-4-yl)-piperazin-1-karbonsäure-[1-(2,4,6-trimethyl-1-benzolsulfonyl]-5-chlor-2-oxo-3-(2-ethoxyphenyl)-2,3-dihydro-1H-indol-3-yl]-ester
4-(1-Methyl-piperidin-4-yl)-piperazin-1-carbonsäure-[1-(2,4-dichlor-1-benzolsulfonyl]-5-methoxy-2-oxo-3-(2-ethoxyphenyl)-2,3-dihydro-1 H-indol-3-yl]-ester
4-(1-Methyl-piperidin-4-yl)-piperazin-1-karbonsäure-[1-(2-trifluormethoxy-1-benzolsulfonyl]-5-methoxy-2-oxo-3-(2-ethoxyphenyl)-2,3-dihydro-1H-indol-3-yl]-ester
4-(1-Methyl-piperidin-4-yl)-piperazin-1-karbonsäure-[1-(2-methoxy-1-benzolsulfonyl]-5-methoxy-2-oxo-3-(2-ethoxyphenyl)-2,3-dihydro-1H-indol-3-yl]-ester
4-(1-Methyl-piperidin-4-yl)-piperazin-1-karbonsäure-[1-benzolsulfonyl-5-methoxy-2-oxo-3-(2-ethoxyphenyl)-2,3-dihydro-1H-indol-3-yl] ester
1-(2,4-Dimethoxy-1-benzolsulfonyl)-5-methoxy-3-(2-methoxyphenyl)-3{2[4-methyl-piperidin-1-yl)-piperazin-1-yl]-2-oxo-ethoxy}-1,3-dihydroindolon dihydrochlorid
1-(2,4-Dimethoxy-1-benzolsulfonyl-5-methoxy-3-(2-methoxyphenyl)-3{2[4-methyl-piperazin-1-yl)-piperidin-1-yl]-2-oxo-ethoxy)-1,3-dihydroindolon
4-(4-Methyl-piperazin-1-yl)-piperidin-1-karbonsäure-[1-(2,4-dimethoxy-1-benzolsulfonyl]-5-methoxy-2-oxo-3-(2-methoxyphenyl)-2,3-dihydro-1H-indol-3-yl]-ester-dihydrochlorid
4-(1-Methyl-piperidin-4-yl)-piperazin-1-karbonsäure-[1-(2-methyl-1-benzolsulfonyl]-5-methoxy-2-oxo-3-[2-(2-methoxyethyl)phenyl]-2,3-dihydro-1H-indol-3-yl]-ester-dihydrochlorid
4-(1-Methyl-piperidin-4-yl)-piperazin-1-karbonsäure-[1-(2-methoxy-4-methyl-1-benzolsulfonyl]-5-methoxy-2-oxo-3-[2-(2-methoxyethyl)phenyl]-2,3-dihydro-1H-indol-3-yl]-ester-dihydrochlorid
4-(1-Methyl-piperidin-4-yl)-piperazin-1-karbonsäure-[1-(2,4-dimethoxy-1-benzolsulfonyl]-5-methoxy-2-oxo-3-[2-(2-methoxyethyl)phenyl]-2,3-dihydro-1H-indol-3-yl]-ester-dihydrochlorid
4-(1-Methyl-piperidin-4-yl)-piperazin-1-karbonsäure-[5-chloro-1-(2,4-dimethoxy-benzolsulfonyl)-3-(2-methoxymethyl-phenyl)-2-oxo-2,3-dihydro-1H-indol-3-yl]-ester
4-(1-Methyl-piperidin-4-yl)-piperazin-1-karbonsäure-[1-(2,4-dimethoxybenzolsulfonyl)-5-methoxy-2-oxo-3-(2-ethoxyphenyl)-2,3-dihydro-1H-indol-3-yl]-amid
4-(1-Methyl-piperidin-4-yl)-piperazin-1-karbonsäure-[1-benzolsulfonyl-5-methoxy-2-oxo-3-(2-ethoxyphenyl)-2,3-dihydro-1H-indol-3-yl]-amid-dihydrochlorid
4-(-1-Methyl-piperidin-4-yl)-piperazin-1-karbonsäure-[1-benzolsulfonyl-5-cyano-2-oxo-3-(2-ethoxyphenyl)-2,3-dihydro-1H-indol-3-yl]-ester
4-(1-Methyl-piperidin-4-yl)-piperazin-1-karbonsäure-[1-(2,4-dimethoxy-1-benzolsulfonyl)-5-cyano-2-oxo-3-(2-ethoxyphenyl)-2,3-dihydro-1H-indo)-3-yl]-ester
4-(1-Methyl-piperidin-4-yl)-piperazin-1-karbonsäure-[1-(2,4-dichlor-1-benzolsulfonyl)-5-cyano-2-oxo-3-(2-ethoxyphenyl)-2,3-dihydro-1H-indol-3-yl]-ester
4-(1-Methyl-piperidin-4-yl)-piperazin-1-karbonsäure-[1-(2-methoxy-1-benzolsulfonyl)-5-cyano-2-oxo-3-(2-ethoxyphenyl)-2,3-dihydro-1H-indol-3-yl]-ester
4-(1-Methyl-piperidin-4-yl)-piperazin-1-karbonsäure-[1-(2,4-dimethoxybenzolsulfonyl)-5-chlor-2-oxo-3-(2-ethoxyphenyl)-2,3-dihydro-1H-indol-3-yl]-amid-dihydrochlorid
4-(1-Methyl-piperidin-4-yl)-piperazin-1-karbonsäure-[1-benzolsulfonyl-5-chlor-2-oxo-3-(2-ethoxyphenyl)-2,3-dihydro-1H-indol-3-yl]-amid dihydrochlorid
4-(1-Methyl-piperidin-4-yl)-piperazin-1-karbonsäure-[1-(2,4,6-trimethyl-1-benzolsulfonyl)-5-chlor-2-oxo-3-(2-ethoxyphenyl)-2,3-dihydro-1H-indol-3-yl]-amid-dihydrochlorid
4-(1-Methyl-piperidin-4-yl)-piperazin-1-karbonsäure-[1-(4-isopropyl-1-benzolsufonyl)-5-chlor-2-oxo-3-(2-ethoxyphenyl)-2,3-dihydro-1H-indol-3-yl]-amid
4-(1-Methyl-piperidin-4-yl)-piperazin-1-karbonsäure-[1-(4-cyano-1-benzolsulfonyl)-chlor-2-oxo-3-(2-ethoxyphenyl)-2,3-dihydro-1H-indol-3-yl]-amid
4-(1-Methyl-piperidin-4-yl)-piperazin-1-karbonsäure-[5-chlor-3-(2-ethoxyphenyl)-1-(2-methoxy-benzolsulfonyl)-2-oxo-2,3-dihydro-1H-indol-3-yl]-amid
4-(1-Methyl-piperidin-4-yl)-piperazin-1-karbonsäure-[1-(2,4-dimethoxybenzolsulfonyl)-3-(2-ethoxy-phenyl)-5-fluoro-2-oxo-2,3-dihydro-1H-indol-3-yl]-ester
4-(1-Methyl-piperidin-4-yl)-piperazin-1-karbonsäure-[1-benzolsulfonyl-3-(2-ethoxy-phenyl)-5-fluoro-2-oxo-2,3-dihydro-1H-indol-3-yl]-ester
4-(1-Methyl-piperidin-4-yl)-piperazin-1-karbonsäure-[3-(2-ethoxy-phenyl)-5-methoxy-1-(4-methoxy-benzolsulfonyl)-2-oxo-2,3-dihydro-1H-indol-3-yl]-ester
4-(1-Methyl-piperidin-4-yl)-piperazin-1-karbonsäure-[3-(2-isopropoxyphenyl)-5-methoxy-1-(4-methoxy-benzolsulfonyl)-2-oxo-2,3-dihydro-1H-indol-3-yl]-ester
4-(1-Methyl-piperidin-4-yl)-piperazin-1-karbonsäure-[5-methoxy-1-(4-methoxy-benzolsulfonyl)-2-oxo-3-(2-propoxy-phenyl)-2,3-d-hydro-1H-indol-3-yl]-ester
4-(1-Methyl-piperidin-4-yl)-piperazin-1-karbonsäure-[5-chlor-3-(2-ethoxyphenyl)-1-(4-methoxy-benzolsulfonyl)-2-oxo-2,3-dihydro-1H-indol-3-yl]-amid
4-(1-Methyl-piperidin-4-yl)-piperazin-1-karbonsäure-{5-methoxy-1-(2-methoxy-benzolsulfonyl)-3-[2-(2-methoxy-ethyl)-phenyl]-2-oxo-2,3-dihydro-1 H-indol-3-yl}-amid-dihydrochlorid
4-(-1-Methyl-piperidin-4-yl)-piperazin-1-karbonsäure-[5-methoxy-1-(2-methoxy-benzolsulfonyl)-2-oxo-3-(2-propoxy-phenyl)-2,3-dihydro-1H-indol-3-yl]-ester
4-(1-Methyl-piperidin-4-yl)-piperazin-1-karbonsäure-[3-(2-isopropoxyphenyl)-5-methoxy-1-(2-methoxy-benzolsulfonyl)-2-oxo-2,3-dihydro-1 H-indol-3-yl]-ester
4-(1-Methyl-piperidin-4-yl)-piperazin-1-karbonsäure-[5-methoxy-1-(4-methoxy-benzolsulfonyl)-3-(2-methoxymethyl-phenyl)-2-oxo-2,3-dihydro-1H-indol-3-yl]-ester
4-(1-Methyl-piperidin-4-yl)-piperazin-1-karbonsäure-[1-benzolsulfonyl-5-chlor-3-(2-methoxymethyl-phenyl)-2-oxo-2,3-dihydro-1H-indol-3-yl]-ester
4-(4-Methyl-piperazin-1-yl)-piperidin-1-karbonsäure-[3-(2-ethoxy-phenyl)-5-methoxy-1-(2-methoxy-benzolsulfonyl)-2-oxo-2,3-dihydro-1H-indol-3-yl]-ester
4-(4-Methyl-piperazin-1-yl)-piperidin-1-karbonsäure-[1-(2,4-dimethoxybenzolsulfonyl)-3-(2-ethoxy-phenyl)-5-methoxy-2-oxo-2,3-dihydro-1H-indol-3-yl]-ester
4-(4-Methyl-piperazin-1-yl)-piperidin-1-karbonsäure-[3-(2-ethoxy-phenyl)-5-methoxy-1-(4-methoxy-benzolsulfonyl)2-oxo-2,3-dihydro-1H-indol-3-yl]-ester
4-(4-Methyl-piperazin-1-yl)piperidin-1-karbonsäure-[3-(2-isopropoxyphenyl)-5-methoxy-1-(2-methoxy-benzolsulfonyl)-2-oxo-2,3-dihydro-1H-indol-3-yl]-ester
4-(4-Methyl-piperazin-1-yl)-piperidin-1-karbonsäure-[1-(2,4-dimethoxybenzolsulfonyl)-3-(2-isopropoxy-phenyl)-5-methoxy-2-oxo-2,3-dihydro-1H-indol-3-yl]-ester
4-(4-Methyl-piperazin-1-yl)-piperidin-1-karbonsäure-[5-chlor-3-(2-ethoxyphenyl)-1-(4-methoxy-benzolsulfonyl)-2-oxo-2,3-dihydro-1H-indol-3-yl]-ester
4-(4-Methyl-piperazin-1-yl)-piperidin-1-karbonsäure-[3-(2-isopropoxyphenyl)-5-methoxy-1-(4-methoxy-benzolsulfonyl)-2-oxo-2,3-dihydro-1H-indol-3-yl]-ester
4-(4-Methyl-piperazin-1-yl)-piperidin-1-karbonsäure-[5-chlor-3-(2-ethoxyphenyl)-1-(2-methoxy-benzolsulfonyl)-2-oxo-2,3-dihydro-1H-indol-3-yl]-ester
4-(4-Methyl-piperazin-1-yl)-piperidin-1-karbonsäure-[5-chlor-1-(2,4-dimethoxy-benzolsulfonyl)-3-(2-ethoxy-phenyl)-2-oxo-2,3-dihydro-1H-indol-3-yl]-ester
4-(1-Methyl-piperidin-4-yl)-piperazin-1-karbonsäure-[1-(2-methoxybenzolsulfonyl)-3-(2-methoxy-phenyl)-2-oxo-6-trifluoromethyl-2,3-dihydro-1H-indol-3-yl]-ester
4-(1-Methyl-piperidin-4-yl)piperazin-1-karbonsäure-[3-(2-ethoxy-phenyl)-5-fluoro-1-(2-methoxy-benzolsulfonyl)-2-oxo-2,3-dihydro-1H-indol-3-yl]-ester
4-(1-Methyl-piperidin-4-yl)-piperazin-1-karbonsäure-[3-(2-ethoxy-phenyl)-5-fluoro-1-(4-methoxy-benzolsulfonyl)-2-oxo-2,3-dihydro-1H-indol-3-yl]-ester dihydrochlorid
4-(1-Methyl-piperidin-4-yl)-piperazin-1-karbonsäure-[1-(4-cyano-benzolsulfonyl)-3-(2-ethoxy-phenyl)-5-fluor-2-oxo-2 ,3-dihydro-1H-indol-3-yl]-ester
4-(1-Methyl-piperidin-4-yl)-piperazin-1-karbonsäure-[5-chlor-3-(2-ethoxyphenyl)-1-(4-methoxy-benzolsulfonyl)-2-oxo-2,3-dihydro-1H-indol-3-yl]-ester dihydrochlorid
4-(1-Methyl-piperidin-4-yl)-piperazin-1-karbonsäure-[5-chlor-1-(2-methoxy-benzolsulfonyl)-3-(2-methoxymethyl-phenyl)-2-oxo-2,3-dihydro-1H-indol-3-yl]-ester
4-(1-Methyl-piperidin-4-yl)-piperazin-1-karbonsäure-[3-(2-ethoxy-phenyl)-5-methoxy-1-(2-methoxy-benzolsulfonyl)-2-oxo-2,3-dihydro-1H-indol-3-yl]-amide
4-(1-Methyl-piperidin-4-yl)-piperazin-1-karbonsäure-[3-(2-ethoxy-phenyl)-5-methoxy-1-(4-methoxy-benzolsulfonyl)-2-oxo-2,3-dihydro-1H-indol-3-yl]-amid
4-(1-Methyl-piperidin-4-yl)-piperazin-1-karbonsäure-[1-(2,4-dimethoxy benzolsulfonyl)-3-(2-methoxy-phenyl)-2-oxo-6-trifluoromethyl-2,3-dihydro-1H-indol-3-yl]-ester
4-(4-Methyl-piperazin-1-yl)-piperidin-1-karbonsäure-[5-chlor-3-(2-ethoxyphenyl)-1-(4-methoxy-benzolsulfonyl)-2-oxo-2,3-dihydro-1H-indol-3-yl]-amid dihydrochlorid
4-(4-Methyl-piperazin-1-yl)-piperidin-1-karbonsäure-[5-chlor-1-(2,4-dimethoxy-benzolsulfonyl)-3-(2-ethoxy-phenyl)-2-oxo-2,3-dihydro-1H-indol-3-yl]-amid dihydrochlorid
4-(4-Methyl-piperazin-1-yl)-piperidin-1-karbonsäure-[5-chlor-3-(2-ethoxyphenyl)-1-(2-methoxy-benzolsulfonyl)-2-oxo-2,3-dihydro-1H-indol-3-yl]-amid
4-(4-Methyl-piperidin-1-yl)-piperazin-1-karbonsäure-[5-cyano-3-(2-ethoxyphenyl)-2-oxo-1-(toluol-2-sulfonyl)-2,3-dihydro-1H-indol-3-yl]-ester
4-(1-Methyl-piperidin-4-yl)-piperazin-1-karbonsäure-[5-cyano-3-(2-ethoxyphenyl)-2-oxo-1-(toluol-4-sulfonyl)-2 ,3-dihydro-1H-indol-3-yl]-ester
4-(1-Methyl-piperidin-4-yl)-piperazin-1-karbonsäure-[1-(2-chlorbenzolsulfonyl)-5-cyano-3-(2-ethoxy-phenyl)-2-oxo-2,3-dihydro-1H-indol-3-yl]-ester
4-(1-Methyl-piperidin-4yl)-piperazin-1-karbonsäure-[5-cyano-1-(2,5-dimethoxy-benzolsulfonyl)-3-(2-ethoxy-phenyl)-2-oxo-2,3-dihydro-1H-indol-3-yl]-ester
4-(1-Methyl-piperidin-4-yl)-piperazin-1-karbonsäure-[5-cyano-1-(2-cyano-benzolsulfonyl)-3-(2-ethoxy-phenyl)-2-oxo-2,3-dihydro-1H-indol-3-yl]-ester
4-(1-Methyl-piperidin-4-yl)-piperazin-1-karbonsäure-[5-cyano-1-(2,4-difluoro-benzolsulfonyl)-3-(2-ethoxy-phenyl}-2-oxo-2,3-dihydro-1H-indol-3-yl]-ester
4-(1-Methyl-piperidin-4-yl)-piperazin-1-karbonsäure-[5-cyano-1-(4-fluoro-benzolsulfonyl)-3-(2-ethoxy-phenyl)-2-oxo-2,3-dihydro-1H-indol-3-yl]-ester
4-(1-Methyl-piperidin-4-yl)-piperazin-1-karbonsäure-(5-cyano-3-(2-ethoxyphenyl)-1-(4-isopropyl-benzolsulfonyl)-2-oxo-2,3-dihydro-1H-indol-3-yl]-ester
4-(1-Methyl-piperidin-4-yl)-piperazin-1-karbonsäure-[5-cyano-3-(2-ethoxyphenyl)-1 -(2-fluoro-benzolsulfonyl)-2-oxo-2,3-dihydro-1H-indol-3-yl]-ester
4-(1-Methyl-piperidin-4-yl)-piperazin-1-karbonsäure-1-[5-chlor-2-methoxy-benzolsulfonyl)-5-cyano-3-(2-ethoxy-phenyl)-2-oxo-2,3-dihydro-1H-indol-3-yl]-ester
4-(1-Methyl-piperidin-4-yl)-piperazin-1-karbonsäure-[5-cyano-3-(2-ethoxyphenyl)-1-(2-methoxy-5-methyl-benzolsulfonyl)-2-oxo-2,3-dihydro-1H-indol-3-yl]-ester
4-(1-Methyl-piperidin-4-yl)-piperazin-1-karbonsäure-[5-cyano-3-(2-ethoxyphenyl)-1-(2-methoxy-4-methyl-benzolsulfonyl)-2-oxo-2,3-dihydro-1H-indol-3-yl]-ester
4-(1-Methyl-piperidin-4-yl)-piperazin-1-karbonsäure-[1-benzolsulfonyl-5-cyano-3-(2-ethoxy-phenyl)-2-oxo-2,3-dihydro-1H-indol-3-yl]amid dihydrochlorid
4-(1-Methyl-piperidin-4-yl)-piperazin-1-karbonsäure-[5-cyano-3-(2-ethoxyphenyl)-1-(4-methoxy-benzolsulfonyl)-oxo-2,3-dihydro-1H-indol-3-yl]-amid
4-(1-Methyl-piperidin-4-yl)-piperazin-1-karbonsäure-[5-cyano-3-(2-ethoxyphenyl)-1-(2-methoxy-benzolsulfonyl)-2-oxo-2,3-dihydro-1H-indol-3-yl]-amid dihydrochlorid
4-(1-Methyl-piperidin-4-yl)-piperazin-1-karbonsäure-[5-cyano-3-(2-ethoxyphenyl)-1-(2,4-dimethoxy-benzolsulfonyl)-2-oxo-2,3-dihydro-1H-indol-3-yl]-amid
4-(1-Methyl-piperidin-4-yl)-piperazin-1-karbonsäure-[1-(4-acetylamino-benzolsulfonyl)-5-cyano-3-(2-ethoxy-phenyl)-2-oxo-2,3-dihydro-1H-indol-3-yl]-ester
4-(1-Methyl-piperidin-4-yl)-piperazin-1-karbonsäure-[1-(2,4-dimethoxy-benzolsulfonyl)-3-(2-ethoxy-phenyl)-5-fluoro-2-oxo-2,3-dihydro-1H-indol-3-yl]-amid
4-(1-Methyl-piperidin-4-yl)-piperazin-1-karbonsäure-[1-(2-methoxy-benzolsulfonyl)-3-(2-ethoxy-phenyl)-5-fluoro-2-oxo-2,3-dihydro-1H-indol-3-yl]-amid
4-(1-Methyl-piperidin-4-yl)-piperazin-1-karbonsäure-[1-(4-methoxy-benzolsulfonyl)-3-(2-ethoxy-phenyl)-5-fluor-2-oxo-2,3-dihydro-1H-indol-3-yl]-amid dihydrochlorid
4-(1-Methyl-piperidin-4-yl)-piperazin-1-karbonsäure-[3-(2-ethoxy-phenyl)-5-isopropyl-1-(2-methoxy-benzolsulfonyl)-2-oxo-2,3-dihydro-1H-indol-3-yl]-ester
4-(1-Methyl-piperazin-4-yl)-piperidin-1-karbonsäure-[5-cyano-3-(2-ethoxyphenyl)-1-(2-methoxy-benzolsulfonyl)-2-oxo-2,3-dihydro-1H-indol-3-yl]-amid
4-(1-Methyl-piperidin-4-yl)-piperazin-1-karbonsäure-[3-(2-ethoxy-phenyl)-5-fluoro-1-(2-methoxy-benzolsulfonyl)-2-oxo-2,3-dihydro-1H-indol-3-yl]-amid
4-(1-Methyl-piperidin-4-yl)-piperazin-1-karbonsäure-[3-(2-ethoxy-phenyl)-5-isopropyl-1-(4-methoxy-benzolsulfonyl)-2-oxo-2,3-dihydro-1H-indol-3-yl]-ester
4-(1-Methyl-piperazin-4-yl)-piperidin-1-karbonsäure-[5-cyano-3-(2-ethoxyphenyl)-1-(2,4-dimethoxy-benzolsulfonyl)-2-oxo-2,3-dihydro-1H-indol-3-yl]-amid
(-)-4-(1-Methyl-piperidin-4-yl)-piperazin-1-karbonsäure-[5-cyano-3-(2-ethoxy-phenyl)-1-(2-methoxy-benzolsulfonyl)-2-oxo-2,3-dihydro-1H-indol-3-yl]-ester
(+)-4-(1-Methyl-piperidin-4-yl)-piperazin-1-karbonsäure-[5-cyano-3-(2-ethoxy phenyl)-1-(2-methoxy-benzolsulfonyl)-2-oxo-2,3-dihydro-1H-indol-3-yl]-ester
(-)-4-(1-Methyl-piperidin-4-yl)-piperazin-1-karbonsäure-[5-cyano-3-(2-ethoxy-phenyl)-1-(2,4-dimethoxy-benzolsulfonyl)-2-oxo-2,3-dihydro-1H-indol-3-yl]-ester
(+)-4-(1-Methyl-piperidin-4-yl)-piperazin-1-karbonsäure-[5-cyano-3-(2-ethoxy-phenyl)-1-(2,4-dimethoxy-benzolsulfonyl)-2-oxo-2,3-dihydro-1H-indol-3-yl]-ester
4-(4-Methyl-piperazin-1-yl)-piperidin-1-karbonsäure-[1-benzolsulfonyl-5-cyano-3-(2-ethoxy-phenyl)-2-oxo-2,3-dihydro-1H-indol-3-yl]-amid
4-(1-Methyl-piperidin-4-yl)-piperazin-1-karbonsäure-[1-benzolsulfonyl-3-(2-ethoxy-phenyl)-5-isopropyl-2-oxo-2,3-dihydro-1 H-indol-3-yl]-ester
4-(1-Methyl-piperidin-4-yl)-piperazin-1-karbonsäure-[4-chlor-3-(2-methoxyphenyl)-1-(2,4-dimethoxy-benzolsulfonyl)-2-oxo-2,3-dihydro-1H-indol-3-yl]-ester
4-(1-Methyl-piperidin-4-yl)-piperazin-1-karbonsäure-[4-chlor-3-(2-methoxyphenyl)-1-(4-methoxy-benzolsulfonyl)-2-oxo-2,3-dihydro-1H-indol-3-y]l-ester
4-(1-Methyl-piperazin-4-yl)-piperidin-1-karbonsäure-[3-(2-ethoxy-phenyl)-5-methoxy-1-(2-methoxy-benzolsulfonyl)-2-oxo-2,3-dihydro-1H-indol-3-yl]-amid-dihydrochlorid
4-(1-Methyl-piperazin-4-yl)-piperidin-1-karbonsäure-[3-(2-ethoxy-phenyl)-5-methoxy-1-(2,4-dimethoxy-benzolsulfonyl)-2-oxo-2,3-dihydro-1H-indol-3-yl]-amid-dihydrochlorid
4-(1-Methyl-piperazin-4-yl)-piperidin-1-karbonsäure-[3-(2-ethoxy-phenyl)-5-methoxy-1-(4-methoxy-benzolsulfonyl)-2-oxo-2,3-dihydro-1 H-indol-3-yl]-amid-dihydrochlorid
4-(4-Methyl-piperazin-1-yl)-piperidin-1-karbonsäure-[1-benzolsulfonyl-3-(2-ethoxy-phenyl)-5-methoxy-2-oxo-2,3-dihydro-1H-indol-3-yl]-amid-dihydrochlorid
4-(1-Methyl-piperidin-4-yl)-piperazin-1-karbonsäure-[6-chlor-3-(2-methoxyphenyl)-1-(4-methoxy-benzolsulfonyl)-2-oxo-2,3-dihydro-1H-indol-3-yl]-ester
4-(1-Methyl-piperidin-4-yl)-piperazin-1-karbonsäure-[1-(2,4-dimethoxy-benzolsutfonyl)-3-(2-ethoxy-phenyl)-5-isopropyl-2-oxo-2,3-dihydro-1H-indol-3-yl]-ester
4-Piperidin-4-yl-piperazin-1-karbonsäure-[5-cyano-1-(2,4-dimethoxy-benzolsulfonyl)-3-(2-ethoxy-phenyl)-2-oxo-2,3-dihydro-1H-indol-3-yl]-ester-dihydrochlorid
4-(4-Methyl-piperazin-1-yl)-piperidin-1-karbonsäure-[5-cyano-3-(2-ethoxyphenyl)-1-(4-methoxy-benzolsulfonyl)-2-oxo-2,3-dihydro-1H-indol-3-yl]-amid 4-(4-Ethyl-piperidin-1-yl)-piperazin-1-karbonsäure-[5-cyano-3-(2-ethoxyphenyl)-1-(2,4-dimethoxy-benzolsulfonyl)-2-oxo-2,3-dihydro-1H-indol-3-yl]-ester
4-(4-Propyl-piperidin-1-yl)-piperazin-1-karbonsäure-[5-cyano-3-(2-ethoxyphenyl)-1-(2,4-dimethoxy-benzolsulfonyl)-2-oxo-2,3-dihydro-1H-indol-3-yl]-ester
4-(4-Isopropyl-piperidin-1-yl)-piperazin-1-karbonsäure-[5-cyano-3-(2-ethoxyphenyl)-1-(2,4-dimethoxy-benzolsulfonyl)-2-oxo-2,3-dihydro-1H-indol-3-yl]-ester
4-(4-Methyl-piperidin-1-yl)-piperazin-1-karbonsäure-14-methyl-3-(2-ethoxyphenyl)-1-(2,4-dimethoxy-benzolsulfonyl)-2-oxo-2,3-dihydro-1H-indol-3-yl]-ester
4-(4-Methyl-piperidin-1-yl)-piperazin-1-karbonsäure-[5-cyano-3-(2-ethoxyphenyl)-1-(3,4-dibrom-benzolsulfonyl)-2-oxo-2,3-dihydro-1H-indol-3-yl]-ester
4-(4-Methyl-piperidin-1-yl)-piperazin-1-karbonsäure-[4-methoxy-3-(2-methoxy-phenyl)-1-(2-methoxy-benzolsulfonyl)-2-oxo-2,3-dihydro-1H-indol-3-yl]-ester
4-(4-Methyl-piperidin-1-yl)-piperazin-1-karbonsäure-[4-methoxy-3-(2-methoxy-phenyl)-1-(2,4-dimethoxy-benzolsulfonyl)-2-oxo-2,3-dihydro-1 H-indol-3-yl]-ester
4-(4-Methyl-piperidin-1-yl)-piperazin-1-karbonsäure-[5-methoxy-3-(2-ethoxyphenyl)-1-(2,4-dimethoxy-benzolsulfonyl)-2-oxo-2,3-dihydro-1H-indol-3-yl]-ester
4-Piperazin-1-yl-piperidin-1-karbonsäure-[5-cyano-1-(2,4-dimethoxy-benzolsulfonyl)-3-(2-ethoxy-phenyl)-2-oxo-2,3-dihydro-1H-indol-3-yl]-amid-dihydrochlorid
4-(4-Ethyl-piperazin-1-yl)-piperidin-1-karbonsäure-[5-cyano-1-(2,4-dimethoxy-benzolsulfonyl)-3-(2-ethoxy-phenyl)-2-oxo-2,3-dihydro-1H-indol-3-yl]-amid
4-(4-Propyl-piperazin-1-yl)-piperidin-1-karbonsäure-[5-cyano-1-(2,4-dimethoxy-benzolsulfonyl)-3-(2-ethoxy-phenyl)-2-oxo-2,3-dihydro-1H-indol-3-yl]-amid
4-(4-Propyl-piperazin-1-yl-piperidin-1-karbonsäure-[5-cyano-1-benzolsulfonyl-3-(2-ethoxy-phenyl)-2-oxo-2.3-dihydro-1H-indol-3-yl]-amid
4-(4-Propyl-piperazin-1-yl)-piperidin-1-karbonsäure-[5-cyano-1-(2-methoxy benzolsulfonyl)-3-(2-ethoxy-phenyl)-2-oxo-2,3-dihydro-1H-indol-3-yl]-amid
4-(4-Isopropyl-piperazin-1-yl)-piperidin-1-karbonsäure-[5-cyano-1-(2,4-dimethoxy-benzolsulfonyl)-3-(2-ethoxy-phenyl)-2-oxo-2,3-dihydro-1H-indol-3-yl]-amid-dihydrochlorid
4-(4-Propyl-piperazin-1-yl)-piperidin-1-karbonsäure-[5-cyano-1-(2,4-difluor-benzolsulfonyl)-3-(2-ethoxy-phenyl)-2-oxo-2,3-dlhydro-1H-indol-3-yl]-ester
4-(4-Propyl-piperazin-1-yl)-piperidin-1-karbonsäure-[5-cyano-1-(4-methoxy-benzolsulfonyl)-3-(2-ethoxy-phenyl)-2-oxo-2,3-dihydro-1H-indol-3-yl]-ester
4-(4-Propyl-piperazin-1-yl)-piperidin-1-karbonsäure-[5-cyano-1-(2-fluor-benzolsulfonyl)-3-(2-ethoxy-phenyl)-2-oxo-2,3-dihydro-1H-indol-3-yl]-ester
4-(4-Propyl-piperazin-1-yl)-piperidin-1-karbonsäure-[5-cyano-1-benzolsulfonyl-3-(2-ethoxy-phenyl)-2-oxo-2,3-dihydro-1H-indol-3-y]-ester
4-(4-Propyl-piperazin-1-yl)-piperidin-1-karbonsäure-[5-cyano-1-(4-methoxy-benzolsulfonyl)-3-(2-ethoxy-phenyl)-2-oxo-2,3-dihydro-1H-indol-3-yl]-amid
4-(4-Propyl-piperazin-1-yl)-piperidin-1-karbonsäure-[5-cyano-1-(2-fluoro-benzolsulfonyl)-3-(2-ethoxy-phenyl)-2-oxo-2,3-dihydro-1H-indol-3-yl]-amid
4-(4-Propyl-piperazin-1-yl)-piperidin-1-karbonsäure-[5-cyano-1-(2,4-difluoro-benzolsulfonyl)-3-(2-ethoxy-phenyl)-2-oxo-2,3-dihydro-1H-indol-3-yl]-amid
4-(4-Propyl-piperazin-1-yl)-piperidin-1-karbonsäure-[5-cyano-1-(2-methoxy-benzolsulfonyl)-3-(2-ethoxy-phenyl)-2-oxo-2,3-dihydro-1H-indol-3-yl]-ester
4-(4-Methyl-piperazin-1-yl)-piperidin-1-karbonsäure-[5-cyano-1-benzolsulfonyl-3-(2-ethoxy-phenyl)-2-oxo-2,3-dihydro-1H-indol-3-yl]-ester dihydrochlorid
4-(4-Methyl-piperazin-1-yl)-piperidin-1-karbonsäure-[5-cyano-1-(2,4-difluor-benzolsulfonyl)-3-(2-ethoxy-phenyl)-2-oxo-2,3-dihydro-1H-indol-3-yl]-ester dihydrochlorid
4-(4-Methyl-piperazin-1-yl)-piperidin-1-karbonsäure-[5-cyano-1-(2-fluor-benzolsulfonyl)-3-(2-ethoxy-phenyl)-2-oxo-2,3-dihydro-1H-indol-3-yl]-ester
4-(4-Methyl-piperazin-1-yl)-piperidin-1-karbonsäure-[5-cyano-1-(4-methoxy-benzolsulfonyl)-3-(2-ethoxy-phenyl)-2-oxo-2,3-dihydro-1H-indoi-3-yl]-ester
4-(4-Methyl-piperazin-1-yl)-piperidin-1-karbonsäure-[5-cyano-1-(2-methoxy-benzolsulfonyl)-3-(2-ethoxy-phenyl)-2-oxo-2,3-dihydro-1H-indol-3-yl]-ester
4-(4-Methyl-piperazin-1-yl)-piperidin-1-karbonsäure-[5-cyano-1-(2,4-dimethoxy-benzolsulfonyl)-3-(2-ethoxy-phenyl)-2-oxo-2,3-dihydro-1H-indol-3-yl]-ester-dihydrochlorid
4-(4-Proparg-3-yl-piperazin-1-yl)-piperidin-1-karbonsäure-[5-cyano-1-(4-methoxy-benzolsulfonyl)-3-(2-ethoxy-phenyl)-2-oxo-2,3-dihydro-1H-indol-3-yl]-ester
4-(4-Isopropyl-piperazin-1-yl)-piperidin-1-karbonsäure-[5-cyano-1-benzolsulfonyl-3-(2-ethoxy-phenyl)-2-oxo-2,3-dihydro-1H-indol-3-yl]-ester
4-(4-Isopropyl-piperazin-1-yl)-piperidin-1-karbonsäuie-[5-cyano-1-(4-methoxy-benzolsulfonyl)-3-(2-ethoxy-phenyl)-2-oxo-2,3-dihydro-1H-indol-3-yl]-ester
4-(4-Allyl-piperazin-1-yl)-piperidin-1-karbonsäure-[5-cyano-1-(4-methoxy-benzolsulfonyl)-3-(2-ethoxy-phenyl)-2-oxo-2,3-dihydro-1H-indol-3-yl]-ester
4-(4-Isopropyl-piperazin-1-yl)-piperidin-1-karbonsäure-[5-cyano-1-(2-methoxy-benzolsulfonyl)-3-(2-ethoxy-phenyl)-2-oxo-2,3-dihydro-1H-indol-3-yl]-ester
4-(4-Ethyl-piperazin-1-yl)-piperidin-1-karbonsäure-[5-cyano-1-benzolsulfonyl-3-(2-ethoxy-phenyl)-2-oxo-2,3-dihydro-1H-indol-3-yl]-amid
4-(4-Ethyl-piperazin-1-yl)-piperidin-1-karbonsäure-[5-cyano-1-(2-methoxy-benzolsulfonyl)-3-(2-ethoxy-phenyl)-2-oxo-2,3-dihydro-1H-indol-3-yl]-amid
4-(4-Ethyl-piperazin-1-yl)-piperidin-1-karbonsäure-[5-cyano-1-(2-fluor-benzolsulfonyl)- 3-(2-ethoxy-phenyl)-2-oxo-2,3-dihydro-1H-indol-3-yl]-amid
4-(4-Propyl-piperazin-1-yl)-piperidin-1-karbonsäure-[5-cyano-1-(2,4-dimethoxy-benzolsulfonyl)-3-(2-isopropoxy-phenyl)-2-oxo-2,3-dihydro-1H-indol-3-yl]-amid
4-(4-Propyl-piperazin-1-yl)-piperidin-1-karbonsäure-[5-cyano-1-(2-methoxy-benzolsulfonyl)-3-(2-isopropoxy-phenyl)-2-oxo-2,3-dihydro-1H-indol-3-yl]-amid
4-(4-Propy)-piperazin-1 -yl)-p)peridin-1 -karbonsäure-[5-cyano-1 -(4-methoxy-benzolsulfonyl)-3-(2-isopropoxy-phenyl)-2-oxo-2,3-dihydro-1H-indol-3-yl]-amid
4-(4-Propyl-piperazin-1-yl)-piperidin-1-karbonsäure-[5-cyano-1-(4-fluor benzolsulfonyl)-3-(2-isopropoxy-phenyl)-2-oxo-2,3-dihydro-1H-indol-3-yl]-amid
4-(4-Isopropyl-piperazin-1-yl)-piperidin-1-karbonsäure-[5-cyano-1-(2,4-dimethoxy-benzolsulfonyl)-3-(2-ethoxy-phenyl)-2-oxo-2,3-dihydro-1 H-indol-3-yl]-ester
4-(4-Isopropyl-piperazin-1-yl)piperidin-1-karbonsäure-[5-cyano-1-(2,4-difluor-benzolsulfonyl)-3-(2-ethoxy-phenyl)-2-oxo-2,3-dihydro-1H-indol-3-yl]-ester
4-(4-Isopropyl-piperazin-1-yl)-piperidin-1-karbonsäure-[5-cyano-1-(2-fluor-benzolsulfonyl)-3-(2-ethoxy-phenyl)-2-oxo-2,3-dihydro-1H-indol-3-yl]-ester
4-(4-Isopropyl-piperazin-1-yl)-piperidin-1-karbonsäure-[5-cyano-1-(4-fluor-benzolsulfonyl)-3-(2-ethoxy-phenyl)-2-oxo-2,3-dihydm-1H-indol-3-yl]-ester
4-(4-Propyl-piperazin-1-yl)-piperidin-1-karbonsäure-[5-cyano-1-(4-fluor-benzolsulfonyl)-3-(2-ethoxy-phenyl)-2-oxo-2,3-dihydro-1H-indol-3-yl]-ester
3-(2-Ethoxy-phenyl)-1-benzolsulfonyl-2-oxo-3-{2-oxo-2-[4-(4-propyl-piperazin-1-yl)-piperidin-1-yl]-ethyl}-2,3-dihydro-1H-indole-5-karbonsäurenitril
3-(2-Ethoxy-phenyl)-1-(2-methoxy-benzolsulfonyl)-2-oxo-3-{2-oxo-2-[4-(4-propyl-piperazin-1-yl)-piperidin-1-yl]-ethyl}-2,3-dihydro-1H-indol-5-karbonsäurenitril
3-(2-Ethoxy-phenyl)-1-(4-methoxy-benzolsulfonyl)-2-oxo-3-{2-oxo-2-[4-(4-propyl-piperazin-1-yl)-piperidin-1-yl]-ethyl}-2,3-dihydro-1H-indol-5-karbonsäurenitril
3-(2-Ethoxy-phenyl)-1-(2-fluoro-benzenesulfonyl)-2-oxo-3-{2-oxo-2-[4-(4-propyl-piperazin-1-yl)-piperidin-1-yl]-ethyl}-2,3-dihydro-1 H-indol-5-karbonsäurenitril
3-(2-Ethoxy-phenyl)-1-(2,4-dimethoxy-benzolulfonyl)-2-oxo-3-{2-oxo-2-[4-(4-propyl-piperazin-1-yl)-piperidin-1-yl]-ethyl}-2,3-dihydro-1H-indol-5-karbonsäurenitril
3-(2-Ethoxy-phenyl)-1-(2,4-difluor-benzolesulfonyl)-2-oxo-3-{2-oxo-2-[4-(4-propyl-piperazin-1-yl)-piperidin-1-yl]-ethyl}-2,3-dihydro-1H-indol-5-karbonsäurenitril
3-(2-Ethoxy-phenyl)-1-(4-chloro-benzolsulfonyl)-2-oxo-3-{2-oxo-2-[4-(4-propyl-piperazin-1-yl)-piperidin-1-yl]-ethyl}-2,3-dihydro-1H-indol-5-karbonsäurenitril
4-(4-Isopropyl-piperazin-1-yl)-piperidin-1-karbonsäure-[5-cyano-1-benzolsulfonyl-3-(2-ethoxy-phenyl)-2-oxo-2,3-dihydro-1H-indol-3-yl]-amid
4-(4-Isopropyl-piperazin-1-yl)-piperidin-1-karbonsäure-[5-cyano-1-(2-methoxy-benzolsulfonyl)-3-(2-ethoxy-phenyl)-2-oxo-2,3-dihydro-1H-indol-3-yl]-amid
4-(4-Isopropyl-piperazin-1-yl)-piperidin-1-karbonsäure-[5-cyano-1-(4-methoxy-benzolsulfonyl)-3-(2-ethoxy-phenyl)-2-oxo-2,3-dihydro-1H-indol-3-y1]-amid
4-(4-Isopropyl-piperazin-1-yl)-piperidin-1-karbonsäure-[5-cyano-1-(2-fluor-benzolsulfonyl)-3-(2-ethoxy-phenyl)-2-oxo-2,3-dihydro-1H-indol-3-yl]-amid
4-(4-Isopropyl-piperazin-1-yl)-piperidin-1-karbonsäure-[5-cyano-1-(2,4-difluor-benzolsulfonyl)-3-(2-ethoxy-phenyl)-2-oxo-2,3-dihydro-1H-indol-3-yl]-amid
4-(4-Ethyl-piperazin-1-yl)-piperidin-1-karbonsäure-[5-cyano-1-(4-methoxy-benzolsulfonyl)-3-(2-ethoxy-phenyl)-2-oxo-2,3-dihydro-1H-indol-3-yl]-amid
4-(4-Ethyl-piperazin-1-yl)-piperidin-1-karbonsäure-[5-cyano-1-(2,4-difluor-benzolsulfonyl)-3-(2-ethoxy-phenyl)-2-oxo-2,3-dihydro-1H-indol-3-yl]-amid
4-(4-Ethyl-piperazin-1-yl)-piperidin-1-karbonsäure-[5-cyano-1-(4-fluor-benzolsulfonyl)-3-(2-ethoxy-phenyl)-2-oxo-2,3-dihydro-1H-indol-3-yl]-amid
4-(4-Propyl-piperazin-1-yl)-piperidin-1-karbonsäure-[5-cyano-1-(4-fluor-benzolsulfonyl)-3-(2-ethoxy-phenyl)-2-oxo-2,3-dihydro-1H-indol-3-yl]-amid
4-(1-Methyl-piperidin-4-yl)-piperazin-1-karbonsäure-[1-(4-methoxy-1-benzolsulfonyl)-5-cyano-2-oxo-3-(2-ethoxyphenyl)-2,3-dihydro-1H-indol-3-yl]-ester
4-(4-Isopropyl-piperazin-1-yl)-piperidin-1-karbonsäure-[5-cyano-1-(4-fluor-benzolsulfonyl)-3-(2-ethoxy-phenyl)-2-oxo-2,3-dihydro-1H-indol-3-yl]-amid
3-(2-Ethoxy-phenyl)-1-(4-fluoro-benzolsulfonyl)-2-oxo-3-{2-oxo-2-[4-(4-propyl-piperazin-1-yl)-piperidin-1-yl]-ethyl}-2,3-dihydro-1H-indol-5-karbonsäurenitril

ihre tautomeren, enantiomeren und/oder diastereomeren Formen, sowie Nichtsalzformen und andere physiologisch verträgliche Salze der erfindungsgemäßen Verbindung oder Verbindungen.

Die erfindungsgemäße Verbindung oder Verbindungen können als Racemate oder als enantiomerenreine oder diastereomerenreine Verbindungen vorliegen. Vorzugsweise liegen die Verbindungen als enantiomerenreine oder diastereomerenreine Verbindungen vor.

Physiologisch verträgliche Salze können beispielsweise mit folgenden Anionen gebildet werden:
Chlorid, Bromid, Phosphat, Carbonat, Nitrat, Perchlorat, Sulfat, Citrat, Lactat, Tartrat, Maleat, Fumarat, Mandelat, Benzoat, Ascorbat, Cinnamat, Glycollat, Methansulfonat, Formiat, Malonat, Naphthalin-2-sulfonat Tosylate, Salicylat und/oder Acetat. Weitere geeignete Säuren sind zum Beispiel in "Fortschritte der Arzneimittelforschung", 1966, Birkhäuser Verlag, Bd.10, S.224-285 aufgelistet.

Im Sinne der vorliegenden Beschreibung umfassen die Begriffe "Alkyl" oder "Alkylen" immer unverzweigtes oder verzweigtes "Alkyl" oder "Alkylen".

C₁-C₄-Alkyl ist im Sinne der Beschreibung vorzugsweise Methyl, Ethyl, n-Propyl, i-Propyl, n-Butyl, sec-Butyl oder t-Butyl.

C₀-Alkylen oder (CH₂)₀ bezeichnen im Sinne der Beschreibung eine Einfachbindung.

C₁-C₄-Alkylen ist im Sinne der Beschreibung Methylen, Ethylen oder verzweigtes oder unverzweigtes Propylen oder Butylen.

C₁-C₆-Alkyl ist im Sinne der Beschreibung Methyl, Ethyl oder verzweigtes oder unverzweigtes Propyl, Butyl, Pentyl oder Hexyl, bevorzugt C₁-C₄-Alkyl, d.h. Methyl, Ethyl, n-Propyl, i-Propyl, n-Butyl, sec-Butyl oder t-Butyl.

C₁-C₆-Alkylen ist im Sinne der Beschreibung Methylen, Ethylen oder verzweigtes oder unverzweigtes Propylen, Butylen, Pentylen oder Hexylen, bevorzugt C₁-C₄-Alkylen, d.h. Methylen, Ethylen oder verzweigtes oder unverzweigtes Propylen oder Butylen.

Durch das Symbol 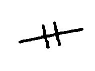 in den chemischen Formeln von Y und Z werden die Verknüpfungsstellen von Y mit X und Z und die Verknüpfungsstellen von Z mit Y angezeigt. In den Formeln von Y kann jede Verknüpfungsstelle eine Bindung zu X oder zu Z darstellen.

Die erfindungsgemäßen Verbindungen sind nach Verabreichung auf verschiedenen Wegen, insbesondere oral, wirksam.

Die erfindungsgemäßen Verbindungen zeigen gute Affinität zu Vasopressin-Rezeptoren, zum Beispiel den Vasopressin-Rezeptor-Subtypen V1a und V1b. Da die verschiedenen Vasopressin-Rezeptoren sehr unterschiedliche Effekte des Vasopressins übermitteln (M. Thibonnier, Exp.Opin. Invest. Drugs 1998, 7(5), 729-740; Serradeil-Le Gal, C, et al.; Prog Brain Res. 2002; 139:197-210), ist es von besonderer Bedeutung, Wirkungen selektiv auf zum Beispiel einen Vasopressin-Rezeptor zu erhalten, um so den gewünschten Effekt zu erzielen, ohne gleichzeitig erhebliche Nebenwirkungen zu verursachen. So vermittelt Vasopressin zum Beispiel über den Rezeptor V2, Wirkungen auf die Niere und deren Funktion und dies wäre bei einer möglichen Behandlung von CNS-Erkrankungen unerwünscht. Besondere Bedeutung hat also neben der eigentlichen Affinität am Zielrezeptor auch die Selektivität gegenüber den anderen Vasopressin-Rezeptoren. Die erfindungsgemäßen Verbindungen zeigen den Vorteil, sehr gute Affinitäten zu den gewünschten Rezeptoren wie den Vasopressin-Rezeptoren V1 b und V1 a zu haben und gleichzeitig eine verbesserte Selektivität gegenüber den anderen Rezeptoren wie V2 aufzuweisen.

Die vorliegende Erfindung stellt auch die Verwendung der erfindungsgemäßen Verbindungen zur Herstellung eines Medikaments zur Behandlung und/oder Prophylaxe von Krankheiten bereit, bei denen der Krankheitsverlauf zumindest teilweise von Vasopressin abhängt, d.h. Krankheiten, die einen erhöhten Vasopressin- oder Oxytocin-Spiegel zeigen, der mittelbar oder indirekt zum Krankheitsbild beitragen kann.

Weiterhin stellt die vorliegende Erfindung die Verwendung der erfindungsgemäßen Verbindungen zur Herstellung eines Medikaments zur Behandlung und/oder Prophylaxe von Krankheiten bereit, wie zum Beispiel Diabetes insipidus, Enuresis nocturna, Inkontinenz, Krankheiten, bei denen Blutgerinnungsstörungen auftreten und/oder zur Verzögerung der Miktion.

Die vorliegende Erfindung stellt auch die Verwendung der erfindungsgemäßen Verbindungen zur Herstellung eines Medikaments zur Behandlung und/oder Prophylaxe von folgenden Krankheiten zur Verfügung: Hypertonie, pulmonare Hypertonie, Herzinsuffizienz, Myocardinfarkt, Koronarer Spasmus, instabile Angina, PTCA (percutaneous transluminal coronary angioplasie), Ischemien des Herzens, Störungen des renalen Systems, Ödeme, renaler Vasospasmus, Nekrose des renalen Cortex, Hyponaträmie, Hypokalämie, Schwartz-Bartter Syndrom, Störungen des Gastrointestinattraktes, gastritischer Vasospasmus, Hepatozirrhose, Magen- und Darmulkus, Emesis, auftretende Emesis bei der Chemotherapie, und Reisekrankheit.

Die erfindungsgemäßen Verbindungen können auch zur Herstellung eines Medikaments zur Behandlung von verschiedenen Vasopressin-abhängigen oder Oxytocin-abhängigen Beschwerden, die zentrainervöse Ursachen oder Veränderungen in der HPA-Achse (hypothalamic pituitary adrenal axis) aufweisen, verwendet werden, zum Beispiel bei affektiven Störungen, wie depressiven Störungen und bipolaren Störungen. Dazu gehören zum Beispiel dythyme Störungen, Phobien, posttraumatische Belastungsstörungen, generelle Angststörungen, Panikstörungen, saisonale Depressionen und Schlafstörungen.

Ebenso können die erfindungsgemäßen Verbindungen zur Herstellung eines Medikaments zur Behandlung bei Angststörungen und stressabhängigen Angststörungen eingesetzt werden, wie zum Beispiel generalisierten Angststörungen, Phobien, posttraumatischen Angststörungen, panischen Angststörungen, obsessivzwanghaften Angststörungen, akuten stressabhängigen Angststörungen und Sozialphobie. Weiterhin können die erfindungsgemäßen Verbindungen auch zur Behandlung von Gedächtnisleistungsstörungen, Morbus Alzheimer, Psychosen, psychotischen Störungen, Schlafstörungen und/oder dem Cushing Syndrom eingesetzt werden.

Die vorliegende Erfindung betrifft auch pharmazeutische Zusammensetzungen, die eine wirksame Dosis einer erfindungsgemäßen Verbindung oder eines pharmazeutisch verträglichen Salzes davon und geeignete Arzneiträger enthalten.

Diese Arzneiträger werden entsprechend der pharmazeutischen Form und der gewünschten Applikationsart gewählt.

Die erfindungsgemäßen Verbindungen der allgemeinen Formel I oder gegebenenfalls geeignete Salze dieser Verbindungen können zur Herstellung von pharmazeutischen Zusammensetzungen zur oralen, sublingualen, subkutanen, intramuskulären, intravenösen, topischen, intratrachealen, intranasalen, transdermalen oder rektalen Verabreichung verwendet werden und Tieren oder Menschen in einheitlichen Verabreichungsformen, gemischt mit herkömmlichen pharmazeutischen Trägem, zur Prophylaxe oder Behandlung der obigen Störungen oder Krankheiten verabreicht werden.

Die geeigneten einheitlichen Verabreichungsformen beinhalten Formen zur oralen Verabreichung, wie Tabletten, Gelatinekapseln, Pulver, Körnchen und Lösungen oder Suspensionen zur oralen Einnahme, Formen zur sublingualen, bukkalen, intratrachealen oder intranasealen Verabreichung, Aerosole, Implantate, Formen der subkutanen, intramuskulären oder intravenösen Verabreichung und Formen der rektalen Verabreichung.

Zur topischen Verabreichung können die erfindungsgemäßen Verbindungen in Cremes, Salben oder Lotionen verwendet werden.

Um den gewünschten prophylaktischen oder therapeutischen Effekt zu erzielen, kann die Dosis des aktiven Grundbestandteils zwischen 0.01 und 50 mg pro kg Körpergewicht und pro Tag variieren.

Jede Einheitsdosis kann 0.05 bis 5000 mg, vorzugsweise 1 bis 1000 mg, des aktiven Bestandteils in Kombination mit einem pharmazeutischen Träger enthalten. Diese Einheitsdosis kann 1 bis 5 mal am Tag verabreicht werden, so dass eine tägliche Dosis von 0.5 bis 25000 mg, vorzugsweise 1 bis 5000 mg, verabreicht wird.

Falls eine feste Zusammensetzung in Form von Tabletten zubereitet wird, wird der Hauptbestandteil mit einem pharmazeutischen Träger, wie Gelatine, Stärke, Laktose, Magnesiumstearat, Talk, Siliziumdioxid oder ähnlichem, gemischt.

Die Tabletten können mit Saccharose, einem Cellulosederivat oder einer anderen geeigneten Substanz beschichtet werden oder anders behandelt werden, um eine anhaltende oder verzögerte Aktivität aufzuweisen und um eine vorbestimmte Menge des aktiven Grundbestandteils kontinuierlich freizugeben.

Eine Zubereitung in Form von Gelatinekapseln wird durch Mischen des aktiven Bestandteils mit einem Streckmittel und Aufnehmen der resultierenden Mischung in weiche oder harte Gelatinekapseln erhalten.

Eine Zubereitung in Form eines Syrups oder Elixirs oder zur Verabreichung in Form von Tropfen kann aktive Bestandteile zusammen mit einem Süßstoff, der vorzugsweise kalorienfrei ist, Methylparaben oder Propylparaben als Antiseptika, einen Aromastoff und einen geeigneten Farbstoff enthalten.

Die wasser-dispersiblen Pulver oder Körnchen können die aktiven Bestandteile, gemischt mit Dispergiermitteln, Benetzungsmitteln oder Suspensionsmitteln, wie Polyvinylpyrrolidone, sowie Süßstoffe oder Geschmackskorrigentien, enthalten.

Eine rektale Verabreichung wird durch Verwendung von Zäpfchen erreicht, die mit Bindemitteln zubereitet werden, die bei rektaler Temperatur schmelzen, zum Beispiel Kakaobutter oder Polyethylenglykole. Eine parenterale Verabreichung wird bewirkt unter Verwendung von wässrigen Suspensionen, isotonischen Salzlösungen oder sterilen und injizierbaren Lösungen, die pharmakologisch verträgliche Dispergiermittel und/oder Benetzungsmittel, zum Beispiel Propylenglykol oder Polyethylenglykol, enthalten.

Der aktive Grundbestandteil kann auch als Mikrokapseln oder Zentrosome, falls geeignet mit einem oder mehreren Trägem oder Additiven, formuliert werden.

Zusätzlich zu den Verbindungen der allgemeinen Formel (I) oder deren pharmazeutisch verträglichen Salzen können die erfindungsgemäßen Zusammensetzungen andere aktive Grundbestandteile enthalten, die zur Behandlung der oben angegebenen Störungen oder Krankheiten nützlich sein können.

Die vorliegende Erfindung betrifft somit weiterhin pharmazeutische Zusammensetzungen, in denen mehrere aktive Grundbestandteile zusammen anwesend sind, wobei mindestens einer von diesen eine erfindungsgemäße Verbindung ist.

Die erfindungsgemäßen Verbindungen stellen Antagonisten der sogenannten Rezeptoren der VasopressinOxytocin-Famile dar. Derartige Verbindungen kann man in geeigneten Tests, die die Affinität zu einem Rezeptor feststellen, untersuchen, wobei die Affinitätskonstante Ki ein Maß für die Potenz zur Bindung am Rezeptor der Verbindungen darstellt und ein kleinerer Wert eine größere Potenz darstellt. Die erfindungsgemäßen Verbindungen wurden zum Beispiel auf ihre Rezeptoraffinität im folgenden Vasopressin-Rezeptor-Subtyp V1 b-Rezeptor auf ihre Affinität geprüft.

### Vasopressin V1a Rezeptorbindungstest

Die Substanzen wurden in einer Konzentration von 10⁻² M in DMSO gelöst und in DMSO auf 10⁻³ M bis 10⁻⁹ M weiter verdünnt. Diese DMSO-Lösungen werden mit Testpuffer 1:10 verdünnt. Im Testansatz wurde die Substanzkonzentration nochmals 1:10 verdünnt.

Der Bindungstest wurde in Anlehnung an die Methode von Tahara et al. (Tahara A et al., Brit. J. Pharmacol. 125, 1463-1470 (1998)) durchgeführt. Im Testansatz (0.250 ml) wurden Membranen (50 ug Protein in Inkubationspuffer (50 mM Tris, 10 mM MgCl₂, 0.1 % BSA auf pH 7.4 mit HCl eingestellt)). von CHO-Zellen mit stabil exprimierten humanen V1a Rezeptoren (Präparation V1a Klon 5.0, mit Protease-Inhibitoren, Roche complete Mini # 1836170) mit 0,04 nM 125Jod-AVP (NEX128) in Inkubationspuffer (totale Binding) oder zusätzlich mit steigenden Konzentrationen an Testsubstanz (Verdrängungsexperiment) inkubiert. Die unspezifische Bindung wurde mit 10⁻⁶ M AVP bestimmt. Dreifachbestimmungen wurden durchgeführt.

Nach Inkubation, 60 Minuten bei Raumtemperatur, wurde der freie Radioligand mittels Vakuumfiltration (Skatron cell harvester 7000) über Wathman GF/B Glasfaserfiltermatten abfiltriert und die Filter in Szintillaüonsgefäße überführt.

Die Flüssigszintillationsmessung erfolgte in einem Tricarb-Gerät Model 2000 oder 2200CA (Packard). Die Umrechnung der gemessenen cpm in dpm wurde mit Hilfe einer Standardquenchreihe durchgeführt.

Die Bindungsparameter wurden durch nichtlineare Regression in SAS berechnet. Die Algorithmen des Programms arbeiten analog zum LIGAND Auswerteprogramm (Munson PJ und Rodbard D, Analytical Biochem. 107, 220-239 (1980)).

Für die erfindungsgemäßen Beispiele wurden in dem obigen Test die Affinitäten zu dem humanen Vasopressin-Rezeptor V1b gemessen und Affinitätskonstanten bestimmt. Dabei zeigten die Beispiele 1, 3, 4, 5, 8 und 13 Ki-Werte unter 100 nM.

### Vasopressin V1b Rezeptorbindungstest:

Die Substanzen wurden in einer Konzentration von 10⁻² M in DMSO gelöst und in DMSO auf 10⁻³ M bis 10⁻⁹ M weiter verdünnt. Diese DMSO-Lösungen wurden mit Testpuffer 1:10 verdünnt. Im Testansatz wurde die Substanzkonzentration nochmals 1:10 verdünnt.

Der Bindungstest wurde in Anlehnung an die Methode von Tahara et al. (Tahara A et al., Brit. J. Pharmacol. 125, 1463-1470 (1998)) durchgeführt. Im Testansatz (0.250 ml) wurden Membranen (58 µg Protein in Inkubationspuffer) von CHO-K1-Zellen mit stabil exprimierten humanen V1b Rezeptoren (Präparation V1b-3H2, mit Protease-Inhibitoren, Roche complete Mini # 1836170) mit 1,5 nM ³H-AVP (8-Arg-Vasopressin, NET 800) in Inkubationspuffer (50 mM Tris, 10 mM MgCl₂, 0.1 % BSA auf pH 7.4 mit HCl eingestellt) (totale Binding) oder zusätzlich mit steigenden Konzentrationen an Testsubstanz (Verdrängungsexperiment) inkubiert. Die unspezifische Bindung wurde mit 10⁻⁶ M AVP bestimmt. Dreifachbestimmungen wurden durchgeführt.

Inkubationspuffer. 50 mM Tris, 10 mM MgCl₂, 0.1 % BSA auf pH 7.4 mit HCl eingestellt.

Nach Inkubation, 60 Minuten bei Raumtemperatur, wurde der freie Radioligand mittels Vakuumfiltration (Skatron cell harvester 7000) über Wathman GF/B Glasfaserfiltermatten abfiltriert und die Filter in Szintillationsgefäße überführt.

Die Flüssigszintillationsmessung erfolgte in einem Tricarb-Gerät Model 2000 oder 2200CA (Packard). Die Umrechnung der gemessenen cpm in dpm wurde mit Hilfe einer Standardquenchreihe durchgeführt.

Die Bindungsparameter wurden durch nichtlineare Regression in SAS berechnet. Die Algorithmen des Programms arbeiten analog zum LIGAND Auswerteprogramm (Munson PJ und Rodbard D, Analytical Biochem. 107, 220-239 (1980)).

Für die erfindungsgemäßen Beispiele wurden in dem obigen Test die Affinitäten zu dem humanen Vasopressin-Rezeptor V1b gemessen und Affnitätskonstanten bestimmt. Dabei zeigten die Beispiele 2, 6, 10, 16, 17, 23, 24, 26 und 30 Ki-Werte unter 100 nM.

### Wirkung auf Vasopressin-induzierten Calcium-Anstieg in Zellen, die einen geklonten humanen Vasopressin-Rezeptor tragen

Die funktionelle Aktivität der Testsubstanzen wurde an CHO-K1 Zellen untersucht, die stabil mit dem humanen V1 b Rezeptor transfiziert wurden. Je Vertiefung einer Mikrotiterplatte mit 96 Vertiefungen wurden 50.000 Zellen ausgesät und über Nacht bei 37°C in gesättigter Wasserdampfatmosphäre mit 5% CO₂ in Kulturmedium inkubiert. Das Kulturmedium bestand aus DMEM/Nut Mix F12 mit Glutamax I (Fa. Invitrogen), 10% fötalem Kälberserum, 100 Einheiten/ml Penicillin, 100 µg/ml Streptomyzin und 800 µg/ml Geneticin. Am nächsten Tag wurden die Zellen mit Kulturmedium gewaschen und mit einem Fluoreszenzfarbstoff für Kalzium nach den Angeben des Herstellers beladen (Ca⁺⁺-Plus-Assay Kit, Molecular Devices). Die Beladung der Zellen erfolgte in Gegenwart von Probenzid (1 Vol%). Die Testsubstanzen wurden mit Kulturmedium verdünnt (Endkonzentration 10⁻¹⁰ bis 10⁻⁵M) und bei Raumtemperatur für 15 Minuten mit den mit Farbstoff beladenen Zellen inkubiert. Danach wurde Arg-Vasopressin (10⁻⁸M) zugegeben und das maximale Fluoreszenzsignal mit einem FLIPR-96 Messgerät (Molecular Devices) bestimmt. Konzentrationswirkungskurven wurden mit nichtlinearen Regressionsalgorithmen erstellt (GraphPad Prism 3.0). Kb Werte wurden aus IC50 Werten nach Cheng und Prusoff berechnet (Kb=IC50/1+L/EC50).

Im folgenden werden beispielhafte Synthesewege zur Herstellung der erfindungsgemäßen Verbindungen beschrieben.

Die Herstellung der erfindungsgemäßen Oxindole kann auf verschiedenen Wegen erfolgen und ist in den Syntheseschemata 1-4 skizziert. Bei diesen Syntheseschemata besitzen die Variablen die gleichen Bedeutungen wie in der allgemeinen Formel (I).

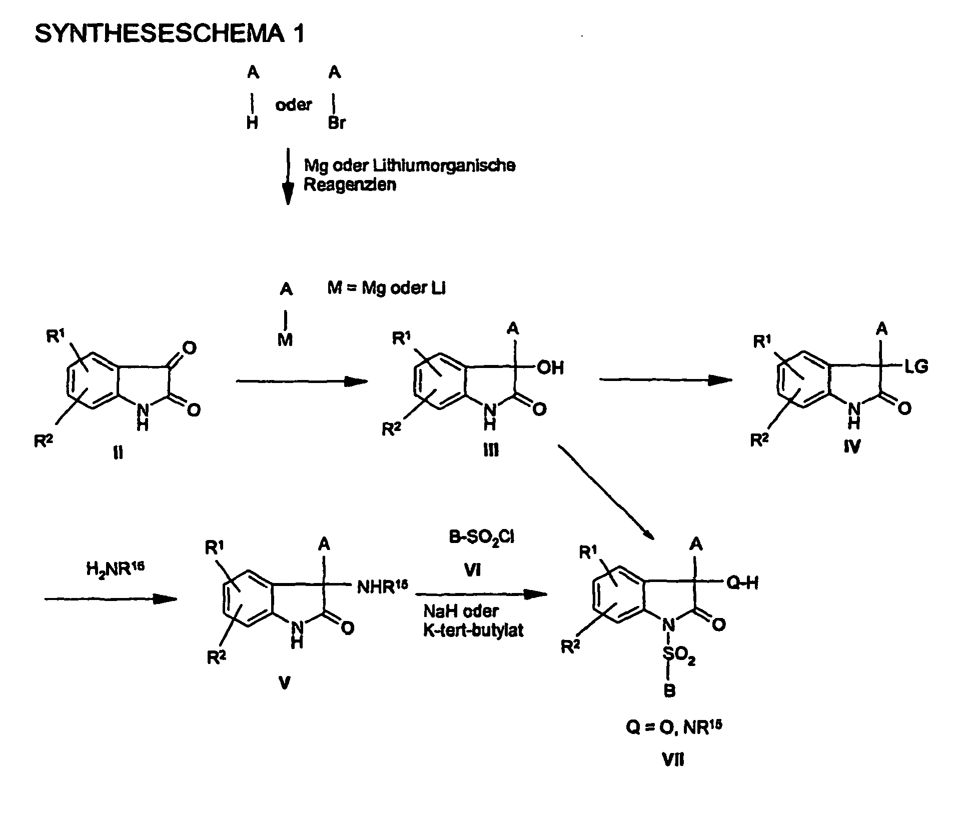

Ausgehend von Verbindungen A-H oder A-Br oder A-Cl, die in üblicher Weise metalliert werden, wie zum Beispiel als Grignard-Verbindung (Mg) oder Organyllithium-Verbindung, können durch Addition an Isatine II die 3-Hydroxy-oxindole III erhalten werden. Die metallierten Verbindungen können in üblicher Weise aus Halogen- oder Kohlenwasserstoffverbindungen erhalten werden. Beispielhafte Vorschriften sind im Houben-Weil, Methoden zur Organischen Chemie, Bd. 13, 1-2, Kap. Mg- bzw. Li-Verbindungen, enthalten. Die Isatine II sind entweder kommerziell erhältlich oder wurden in Analogie zu in der Literatur beschriebenen Methoden hergestellt (Advances in Heterocyclic Chemistry, A.R. Katritzky and A.J. Boulton, Academic Press, New York, 1975, 18, 2-58; J. Brazil. Chem. Soc. 12, 273-324, 2001).

Die 3-Hydroxy-oxindole III können in die Verbindungen IV überführt werden, welche eine Fluchtgruppe LG in 3-Stellung tragen, wobei die Fluchtgruppe LG übliche Abgangsgruppen, wie zum Beispiel Halogenide, Mesylat oder Tosylat, sein können. So kann zum Beispiel (LG = Chlor) das Zwischenprodukt IV durch Behandlung des Alkohols III mit Thionylchlorid in Gegenwart einer Base, wie zum Beispiel Pyridin, hergestellt werden. Alternativ können Alkohole III durch Überführung in das Mesylat mittels Methansulfonylchlorid in Gegenwart einer Base, wie zum Beispiel Triethylamin, erhalten werden. Die Verbindungen IV werden anschließend mit Aminen NH₂R¹⁵ umgesetzt, wobei die analogen Amine V erhalten werden. Zum Beispiel können derartige Substitutionsreaktionen mit Aminen in Gegenwart einer Base wie N,N-Diisopropylethylamin die analogen 3-Amino-oxindole V ergeben. V kann anschließend durch Behandlung mit Sulfonsäurechloriden VI nach Deprotonierung mit einer starken Base, wie zum Beispiel Kalium-*tert*-butytat oder Natriumhydrid, in DMF erfolgen und in das Produkt VII überführt werden. In analoger Weise können ausgehend von den Alkoholen III die entsprechenden Derivate VII mit Q = O erhalten werden.

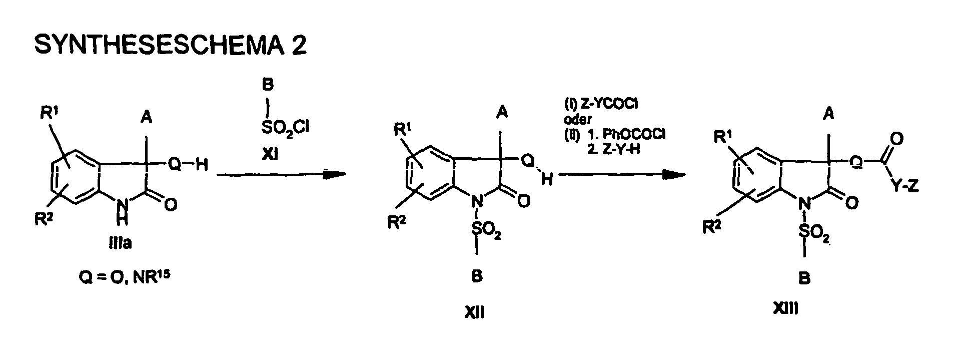

Zur Herstellung der erfindungsgemäßen Verbindungen XIII werden die Oxindole IIIa zunächst mit Sulfonsäurechloriden XI unter den bereits oben beschriebenen Bedingungen umgesetzt. Die eingesetzten Sulfonsäurechloride können entweder käuflich erworben oder in analoger Weise zu bekannten Verfahren (siehe z.B. J. Med. Chem. 40, 1149 (1997)) hergestellt werden. Die erfindungsgemäßen Verbindungen XIII werden auf verschiedenen Wegen, ausgehend von den sulfonylierten Verbindungen XII, hergestellt: (i) Umsetzung mit Carbamoylchloriden Z-Y-CO-CI in Gegenwart einer Base, wie zum Beispiel Triethylamin; (ii) Aktivierung mit Chlorameisensäure-phenylester in Gegenwart einer Base, wie zum Beispiel Pyridin und anschließende Umsetzung mit Aminen Z-Y-H, gegebenenfalls bei erhöhter Temperatur. Die Amine Z-Y-H können entweder käuflich erworben oder nach Literatur-bekannten Methoden hergestellt werden.

Die Herstellung der erfindungsgemäßen Verbindungen XXII, welche ein funktionalisiertes Stickstoffatom in der 3-Stellung tragen (z.B. Amide, Sulfonamide, Carbamate und Harnstoffe) erfolgt analog zum Syntheseschema 2: Die 3-Amino-oxindole XII (Q = NR¹⁵) werden durch Umsetzung mit Reagenzien für die Derivatisierung von Aminogruppen, wie zum Beispiel Carbonsäuren, Carbonsäurechloriden, Carbonsäureanhydriden, Sulfonsäurechloriden, Chloroformaten, Isocyanaten oder Carbamoylchloriden, in die erfindungsgemäßen Verbindungen XIII überführt, wobei im allgemeinen übliche Methoden benutzt werden (siehe J. March, Advanced Organic Chemistry, 1992, 4th edition., Wiley, New York, p. 417-421; 499; 903). Außerdem kann die 3-Aminogruppe in den Verbindungen XII (Q= NH) substituiert werden durch Behandlung mit Alkylierungsmitteln, wie zum Beispiel Alkylbromiden, -iodiden oder -mesylaten, sowie durch Umsetzung mit Aldehyden oder Ketonen in Gegenwart von Reduktionsmitteln, wie zum Beispiel Natriumcyanoborhydrid, im Sinne einer reduktiven Aminierung (J. March, Advanced Organic Chemistry, 1992, 4th edition., Wiley, New York, p. 411; 898).

Alternativ können die Bausteine XII nach dem in Syntheseschema 3 gezeigten zweistufigen Verfahren hergestellt werden.

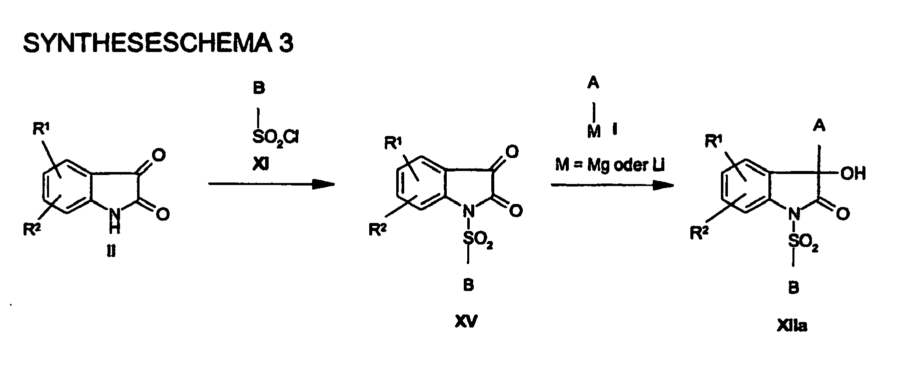

Sulfonylierte Isatine XV werden durch Deprotonierung von Isatinen II mit einer starken Base, wie zum Beispiel Natriumhydrid oder Kalium-tert.-butanolat, und anschließende Behandlung mit Sulfonsäurechloriden XI erhalten. Die Verbindungen Xlla werden im zweiten Schritt durch Addition von metallierten Verbindungen I an die 3-Ketogruppe der Sulfonyl-isatine XV erhalten. Die Vorschriften sind analog den oben beschriebenen Verfahren.

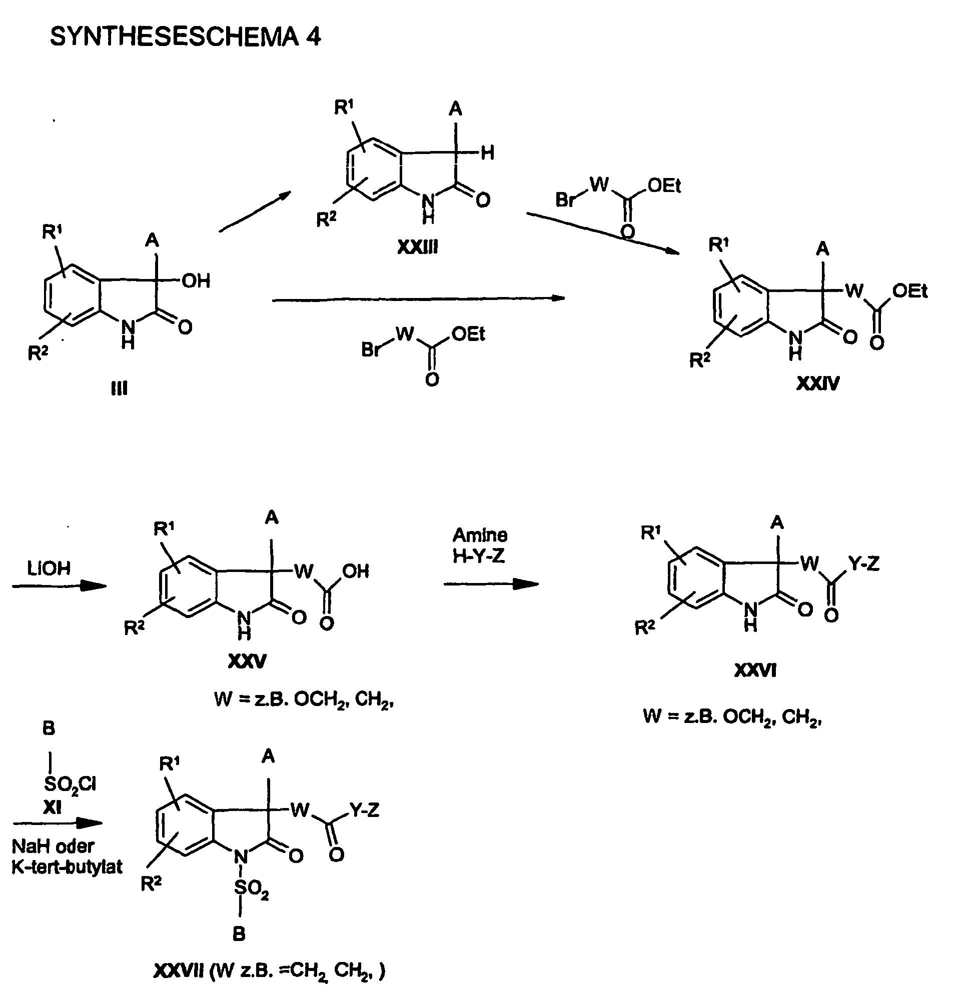

Im Syntheseschema 4 sind Wege zu Verbindungen, in denen W variiert werden kann, skizziert. Alkohole III werden mit Halogencarbonsäureestem zu den Derivaten XXIV umgesetzt, wobei bevorzugt mit Bromiden und Chloriden gearbeitet wird, aber auch analoge Mesylate oder Tosylate und ähnliche Verbindungen, in denen eine Abgangsgruppe vorhanden ist, verwendet werden können. Die Reaktionen können zum Beispiel in polaren Lösungsmitteln, wie DMF oder THF, unter Zusatz von basischen Stoffen, wie zum Beispiel NaH, Kalium-tert.-butanolat, Natriumethanolat, Trialkylaminen oder Kaliumcarbonat, bei Raumtemperatur oder erhöhten Temperaturen, wie der Siedetemperatur des Lösungsmittels, durchgeführt werden. Analog wird die Reaktion des Indol-2-ons XXIII zu XXIV durchgeführt. Die Indolone XXIII können entweder aus den analogen Alkoholen III durch Reduktion der Alkoholgruppe, zum Beispiel mit Triethylsilan oder analog Mullock, E.B. et al., J.Chem.Soc. C, 1970, 6, 829-833, Ghosal, S. et al., Ind. J.Chem., 1969m 7, 1095-1097 und US 2,759,935 synthetisch hergestellt werden. Die Ester XXIV können mit Säuren, wie HCl und H₂SO₄ oder Basen, wie NaOH, KOH oder LiOH, in die analogen Carbonsäuren XXV überführt werden, wobei üblicherweise in Lösungsmitteln, wie Alkoholen oder THF, unter Zusatz von wäßrigen Säuren oder Basen bei Raumtemperatur oder Temperaturen von 25-70°C gearbeitet wird. Diese Säuren XXV können in die Derivate XXVI überführt werden, indem die Säuren mit zum Beispiel Aminen unter Nutzung von üblichen Kupplungsbedingungen, wie sie zum Beispiel in R.C. Larock, Comprehensive Organic Transformations, Wiley 1999, Chap.9 aufgeführt sind, umgesetzt werden. Die Einführung des Sulfonsäure-Rests B-SO₂-erfolgt in analoger Weise wie oben beschrieben. Alternativ zum Schema 4 können die beiden letzten Schritte auch in umgekehrter Weise durchgeführt werden.

### EXPERIMENTELLER TEIL

### Beispiel 1

### 4-(4-Methyl-piperidin-1-yl)-piperazin-1-karbonsäure-[1-benzolsulfonyl-5-chlor-2-oxo-3-(2-methoxy-phenyl)-2,3-dihydro-1H-indol-3-yl]-ester

### 1a) 5-Chlor-3-hydroxy-3-(2-methoxyphenyl)-indol-2-on

40g (1,65 Mol) Magnesiumspäne wurden mit 100 ml Ether überschichtet, und nach Zugabe von wenig Jod wurde vorsichtig erwärmt bis die Reaktion ansprang. Zu der siedenden Lösung wurden 203 ml (1,65 Mol) Bromanisol, gelöst in 450 ml Ether, langsam so zugetropft, daß die Reaktion unter leichtem Sieden kontinuierlich ablief. Anschließend wurden unter leichter Kühlung auf 20°C 75 g (0,41 Mol) 5-Chlorisatin in 750 ml wasserfreiem Tetrahydrofuran zugetropft. Danach wurde alles noch 30 Minuten bei Raumtemperatur gerührt. Die Reaktionslösung wurde unter Rühren in eine wäßrige NH₄Cl-Lösung gegossen. Diese wäßrige Phase wurde mehrmals mit Essigester extrahiert und die vereinigten wäßrigen Phasen viermal mit Wasser gewaschen, getrocknet und im Vakuum eingeengt. Der erhaltene Rückstand wurde aus Isopropanol kristallisiert, wobei 106 g des Zwischenproduktes anfielen.

### 1b) 5-Chlor-1-(2,4-dimethoxy-benzolsulfonyl)-3-hydroxy-3-(2-2-methoxyphenyl)-indol-2-on

Zu 5g (17,3 mMol) des Zwischenproduktes 1a in 50 ml wasserfreiem Dimethylformamid wurden portionsweise 2 g (18,1 mMol) Kalium-tert.-butanolat zugegeben und alles ca. 60 Minuten gerührt. Anschließend wurden 3,2 g (18,1 mMol) Benzolsulfonsäurechlorid zügig bei 0°C zugetropft. Es wurde 2h bei 0°C und dann 16h bei Raumtemperatur gerührt. Die Reaktionslösung wurde anschließend auf 250 ml Eiswasser/K₂CO₃-Lösung gegossen, wobei ein Niederschlag anfiel, der in Methylenchlorid gelöst wurde. Diese organische Phase wurde mit NaCl-Lösung gewaschen, getrocknet und im Vakuum eingeengt. Der erhaltene Rückstand wurde aus Ethanol kristallisiert, wobei 2,8 g des Zwischenproduktes erhalten wurden.

### 1c) Kohlensäure-[5-chlor-1-(2,4-dimethoxy-benzolsulfonyl)-3-(2-methoxyphenyl)-2-oxo-2,3-dihydro-1-H-indol-3-yl]-ester-phenylester

3,3 g (7,7 mMol) des Zwischenproduktes 1b und 4,65 g (46 mMol) Triethylamin wurden in 30 ml Methylenchlorid gelöst. Bei 0°C wurden 4,2 g (26,9 mMol) Chlorameisensäurephenylester zügig zugetropft. Es wurde noch 15 Minuten gerührt, und anschließend wurde die Reaktionslösung in ein 5%ige Kaliumkarbonat-Lösung/Eiswasser-Gemisch gegossen. Die wäßrige Lösung wurde dreimal mit Methylchlorid extrahiert. Die vereinigten organischen Phasen wurden noch mit wäßriger Kaliumcarbonat-Lösung und eine NaCl-Lösung gewaschen, über MgSO₄ getrocknet und im Vakuum eingeengt. Der erhaltene Rückstand wurde mit wenig Methanol behandelt, wobei ein Feststoff ausfiel, der isoliert und getrocknet wurde. Es wurden 3,2 g des Zwischenproduktes erhalten.

### 1d) 4-(4-Methyl-piperidin-1-yl)-piperazin-1-karbonsäure-[1-benzolsulfonyl-5-chlor-2-oxo-3-(2-methoxy-phenyl)-2,3-dihydro-1 H-indol-3-yl]-ester

0,15 g (0,27 mmol) des Zwischenproduktes 1c und 204 mg (1,1 mmol) 1-Methylpiperidin-4-yl)-piperazin wurden für 16h bei Raumtemperatur in 5 ml Tetrahyrofuran gerührt. Anschließend wurde das Lösungsmittel im Vakuum entfernt. Der Rückstand wurde aus 5 ml Methanol kristallisiert, wobei 95 mg des Produktes erhalten wurden.
¹H-NMR (D₆-DMSO): δ = 1.35(2H), 1.6(2H), 1.8(2H), 2.1(3H), 2.15(1H), 2.3(2H), 2.75(2H), 3.05(2H), 3.3(3H), 3.4-3.7(1H), 6.9(1H), 7.1(1H), 7.15(1H), 7.35(1H), 7.45(1H), 7.65(2H), 7.7-7.9(3H) und 8.1 (2H) ppm.

### Beispiel 2

### 4-(1-Methyl-piperidin-4-yl)-piperazin-1-karbonsäure-[1-(2,4-dimethoxy-benzolsulfonyl)-5-methoxy-2-oxo-3-(2-propoxyphenyl)-2,3-dihydro-1H-indol-3-yl]-ester

### 2a) 5-Chlor 3-hydroxy-3-(2-propoxyphenyl)-indol-2-on

3.1 g (0.13 Mol) Magnesiumspäne wurden mit 20 ml Ether überschichtet, und nach Zugabe von wenig Jod wurde vorsichtig erwärmt bis die Reaktion ansprang. Zu der siedenden Lösung wurden 27,3 g (0,13 Mol) 2-Propoxy-1-brombenzol gelöst in 100 ml Ether langsam so zugetropft, daß die Reaktion unter leichtem Sieden kontinuierlich ablief. Anschließend wurden unter leichter Kühlung auf 20°C 7,5 g (42 mMol) 5-Methoxyisatin in 150 ml wasserfreiem Tetrahydrofuran zugetropft. Danach wurde alles noch 30 Minuten bei Raumtemperatur gerührt. Die Reaktionslösung wurde unter Rühren in eine wäßrige NH₄Cl-Lösung gegossen. Diese wäßrige Phase wurde mehrmals mit Essigester extrahiert und die vereinigten wäßrigen Phasen viermal mit Wasser gewaschen, getrocknet und im Vakuum eingeengt. Der erhaltene Rückstand wurde aus wenig Essigester kristallisiert, wobei 8,3 g des Zwischenproduktes anfielen.

### 2b) Kohlensäure-[5-methoxy 2-oxo-3-(2-propoxy-phenyl)-2,3-dihydro-1-H-indol-3-yl]-ester-phenyl ester

Zu 3 g (9,6 mMol) des Zwischenproduktes 2a in 50 ml Pyridin wurden bei 0°C 1,26 ml (10,1 mMol) Chlorameisensäurephenylester zügig zugetropft. Es wurde 16h bei Raumtemperatur gerührt. Anschließend wurde alles in Eiswasser gegossen und mehrmals mit Essigester extrahiert. Die vereinigten organischen Phasen wurden noch mehrmals mit Wasser gewaschen, über MgSO₄ getrocknet und im Vakuum eingeengt. Der erhaltene Rückstand wurde mit wenig Ether behandelt, wobei ein Feststoff ausfiel, der isoliert und getrocknet wurde. Es wurden 3,4 g des Zwischenproduktes erhalten.

### 2c) 4-(4-Methyl-piperidin-1-yl)-piperazin-1-karbonsäure-[5-methoxy-2-oxo-3-(2-propoxy-phenyl)-2,3-dihydro-1H-indol-3-yl]-ester

3,3g (7,6 mMol) des Zwischenproduktes 2b und 5,6g (30,5 mMol) 1-Methylpiperidin-4-yl)-piperazin wurden für 16h bei Raumtemperatur in 100 ml Tetrahyrofuran gerührt. Anschließend wurde das Lösungsmittel im Vakuum entfernt. Der Rückstand wurde zwischen Wasser und Essigester verteilt. Die Wasserphase wurde noch zweimal mit Essigester gewaschen. Die vereinigten Essigesterphasen wurden nochmals mit Wasser gewaschen, getrocknet und im Vakuum eingeengt. Der Rückstand wurde mit Ether verrührt, wobei ein Feststoff entstand, der isoliert wurde. Es wurden 2,9 g des Zwischenproduktes erhalten.

### 2d) 4-(1-Methyl-piperidin-4-yl)-piperazin-1-karbonsäure-[1-(2,4-dimethoxy-benzolsulfonyl)-5-methoxy-2-oxo-3-(2-propoxyphenyl)-2,3-dihydro-1H-indol-3-yl]-ester

Zu 200 mg (0,38 mMol) des Zwischenproduktes 2c in 5 ml wasserfreiem Dimethylformamid wurden portionsweise 54 mg (0,48 mMol) Kalium-tert.-butanolat zugegeben und alles ca. 60 Minuten gerührt. Anschließend wurden 113 mg (0,48 mMol) 2,4-Dimethoxybenzolsulfonsäurechlorid zügig bei 0°C zugetropft. Es wurde alles 16h bei Raumtemperatur gerührt. Die Reaktionslösung wurde anschließend auf 1 M NaOH gegossen, wobei ein Niederschlag anfiel, der isoliert wurde. Dieser Niederschlag wurde in 1 ml Methanol gelöst und mit 1 ml etherischem HCl versetzt. Diese Lösung wurde über Nacht bei 0°C aufbewahrt, wobei ein Niederschlag anfiel, der isoliert wurde. Es wurden 213 mg des Produktes als Dihydrochlorid erhalten.
¹H-NMR (D₆-DMSO): δ = 0.7(3H), 1.5(2H), 2.0(2H), 2.3(2H), 2.7(3H), 2.8-3.7(18H), 3.8(2H), 3.85(3H), 4.3(1H), 6.6(3H), 6.0-7.1(3H), 7.35(1H), 7.7(2H), 7.85(1H), und 10.4-10.8(N⁺H, breit) ppm.

### Beispiel 3

### 4-(1-Methyl-piperidin-4-yl)-piperazin-1-karbonsäure-[1-(2,4-dimethoxy-benzolsulfonyl)-5-chlor-2-oxo-3-(2-methoxyphenyl)-2,3-dihydro-1H-indol-3-yl]-amid-dihydrochlorid

### 3a) 3,5 Dichlor-3-(2-methoxyphenyl)-indol-2-on

Zu 100 g (0,345 Mol) des Zwischenproduktes 1a, 56 ml (0,695 Mol) Pyridin in 1 L Methylenchlorid bei 0°C wurden langsam 38 ml (0,518 Mol) Thionylchlorid zugetropft und anschließend noch ca. 30 Minuten gerührt. Dann wurde das Reaktionsgemisch auf Eiswasser gegossen und die organische Phase abgetrennt. Diese organische Phase wurde noch mit Wasser gewaschen, getrocknet und im Vakuum eingeengt. Der Rückstand wurde mehrmals mit Toluol behandelt und das organische Lösungsmittel jeweils im Vakuum entfernt. Es wurden 79 g des Rohproduktes erhalten, das ohne weitere Reinigung weiter umgesetzt wurde.

### 3b) 3-Amino-5-chlor-3-(2-methoxyphenyl)-indol-2-on

10 g (32,45 mMol) des Zwischenproduktes 2a wurden in 100 ml Methylenchlorid suspendiert. Nach der Zugabe von 100 ml 2-molarer ethanolische Ammoniak-Lösung wurde das Reaktionsgemisch 16h gerührt. Danach wurde alles auf Eiswasser gegossen und die organische Phase abgetrennt. Die wäßrige Phase wurde gekühlt, wobei sich langsam ein weisses Kristallisat bildete, das isoliert wurde. Es wurden 5,7g des Produktes erhalten.

### 3c) Kohtensäure-[5-chlor-2-oxo-3-(2-methoxy-phenyl)-2,3-dihydro-1-H-indol-3-yl]-ester phenyl-amid

Zu 0,8 g (2,8 mMol) des Zwischenproduktes 3b in 20 ml Pyridin wurden bei 0°C 0,38 ml (3,1 mMol) Chlorameisensäureethylester gegeben und anschließend alles für 16h bei Raumtemperatur gerührt. Dann wurde der Ansatz auf Eiswasser gegossen und mit Essigester extrahiert. Die organische Phase wurde mit Wasser gewaschen, getrocknet und im Vakuum eingeengt. Der so erhaltene Rückstand wurde in wenig Ether gelöst und durch vorsichtige Zugabe von n-Pentan wurde das Produkt ausgefällt. Es wurden 1,1g erhalten.

### 3d) 4-(4-Methyl-piperidin-1-yl)-piperazin-1-karbonsäure-(5-chlor-2-oxo-3-phenyl-2,3-dihydro-1 H-indol-3-yl)-amid

1 g (2,4 mMol) des Zwischenproduktes 3c und 1.8 g (9,8 mMol) 1(1-Methylpiperidin-4-yl)piperazin wurden in 35ml wasserfreiem Tetrahydrofuran für 3h unter Rückfluß gekocht. Anschließend wurde das Lösungsmittel im Vakuum entfernt. Der erhaltene Rückstand wurde zwischen Wasser und Essigester verteilt, die organische Phase abgetrennt, mit Wasser gewaschen, getrocknet und im Vakuum eingeengt. Der Rückstand wurde mit Ether/Pentan behandelt, wonach das Produkt ais Feststoff anfiel. Es wurden 0,76 g erhalten.

### 3e) 4-(1-Methyl-piperidin-4-yl)-piperazin-1-karbonsäure-[1-(2,4-dimethoxy-benzolsulfonyl)-5-chlor-2-oxo-3-(2-methoxyphenyl)-2,3-dihydro-1 H-indol-3-yl]-amid-dihydrochlorid

Zu 0,21 g (0,42 mmol) des Zwischenproduktes 3d in 2 ml Dimethylformamid wurden bei 0°C 0,052 mg (0,47 mMol) Kalium-tert.-butanolat gegeben. Alles wurde für 1h bei 0°C gerührt. Danach wurden 0,11 g (0,47 mMol) 2,4-Dimethoxybenzolsulfonsäurechlorid zugegeben. Anschließend wurde das Reaktionsgemisch für 16 bei Raumtemperatur gerührt. Nun wurde das Gemisch in 5%ige Kaliumkarbonat-Lösung gegossen, wonach sich langsam ein Niederschlag bildete. Dieser Niederschlag wurde isoliert und chromatographisch an Kieselgel (Fließmittel: Methylenchlorid/Methanol = 1/1) gereinigt. Es wurde 0,1g des Produktes erhalten.
¹H-NMR (D₆-DMSO): δ = 2.0(2H), 2.3(2H), 2.7(3H), 2.8-3.1(4H), 3.1-3.3(1H), 3.25-3.7(2H), 3.3-3.6(9H), 3.7(3H), 3.85(3H), 3.9-4.1(1H), 6.7(2H), 6.95(1H), 7.05(1 H), 7.3(1 H), 7.35(3H), 7.7(1 H), 7.9(2H) und 10.5(N⁺H, breit) ppm.

Folgende Verbindungen wurden in analoger Weise zu den in den Beispielen 1, 2, 3 und 192 beschriebenen methodischen Verfahren hergestellt:

### Beispiel 4

### 4-(1-Methyl-piperidin-4-yl)-piperazin-1-karbonsäure-[1-benzolsulfonyl-5-chlor-2-oxo-3-phenyl-2,3-dihydro-1 H-indol-3-yl]-ester-dihydrochlorid

¹H-NMR (D₆-DMSO): δ = 2.0(2H), 2.3(2H), 2.7(3H), 2.85-3.1(4H), 3.2-3.7(8H), 4.25(1H), 7.15(2H), 7.35(3H), 7.55(1H), 7.6(3H), 7.8(1H), 7.9(1H), 8.0(1 H) und 10.5(N⁺H, breit) ppm.

### Beispiel 5

### 4-(4-Methyl-piperazin-1-yl)-piperidin-1-karbonsäure-1-benzolsulfonyl-5-chlor-2-oxo-3-phenyl-2,3-dihydro-1H-indol-3-yl]-ester

¹H-NMR (D₆-DMSO): δ = 1.05(1H), 1.4(1H), 1.65(1H), 1.8(1H), 2.1(3H), 2.2-2.5(8H), 2.65(1H), 3.0(1H), 3.5(2H), 4.1(1H), 7.1(2H), 7.35(3H), 7.45(1H), 7.55-7.7(3H), 7.8(1 H), 7.9(1H) und 7.95(2H) ppm.

### Beispiel 6

### 4-(1-Methyl-piperidin-4-yl)-piperazin-1-karbonsäure-[1-(2,4-dimethoxy-benzolsulfonyl)-5-methoxy-2-oxo-3-(2-methoxyphenyl)-2,3-dihydro-1H-indol-3-yl]-ester-dihydrochlorid

¹H-NMR (D₆-DMSO): δ = 2.05(2H), 2.7(3H), 2.9-3.1(4H), 3.1-3.3(2H), 3.3-3.8(15H), 3.85(3H), 4.1-4.4(1H), 6.75(3H), 6.95(2H), 7.05(1H), 7.35(1H), 7.65(1 H), 7.75(1 H), 7.85(1H) und 10.5-11(N⁺H, breit) ppm.

### Beispiel 7

### 4-(4-Methyl-piperazin-1-yl)-piperidin-1-karbonsäure-[1-benzolsulfonyl-5-methyl-2-oxo-3-phenyl-2,3-dihydro-1H-indol-3-yl]-ester-dihydrochlorid

¹H-NMR (D₆-DMSO): δ = 1.2-1.7(4H), 1.9-2.2(2H), 2.3(3H), 2.6-2.8(5H), 3.0(2H), 3.2-3.8(6H), 4.2(1 H), 7.1(3H), 7.3(4H), 7.6(2H), 7.7(2H) und 7.9(2H) ppm.

### Beispiel 8

### 4-(1-Methyl-piperidin-4-yl)-piperazin-1-karbonsäure-[1-(2,4-dimethoxy-benzolsulfonyl)-5-methoxy-2-oxo-3-(2-methylphenyl)-2,3-dihydro-1H-indol-3-yl]-ester-dihydrochlorid

¹H-NMR (D₆-DMSO): δ = 2.05(2H), 2.3(2H), 2.45(3H), 2.7(3H), 2.9-3.1(4H), 3.1-3.25(1H), 3.25-3.7(10H), 3.75(3H), 3.85(3H), 4.1-4.4(1H), 6.6(1H), 6.7(2H), 6.85(1 H), 7.1(2H), 7.25(2H), 7.65(1 H), 7.8(1 H) und 10.5(N⁺H, breit) ppm.

### Beispiel 9

### 4-(Piperidin-4-yl)-piperazin-1-karbonsäure-[1-(2,4-dimethoxy-benzol sulfonyl)-5-methoxy-2-oxo-3-(2-methoxyphenyl)-2,3-dihydro-1H-indol-3-yl]-ester

¹H-NMR (D₆-DMSO): δ = 1.4(2H), 1.7(2H), 2.25-2.4(3H), 2.55-2.7(2H), 3.05-3.2(4H), 3.25-3.4(2H), 3.45-3.6(8H), 3.65(3H), 3.85(3H), 6.55(1 H), 6.65(1 H), 6.7(1H), 6.9(1H), 6.95(1H), 7.05(1H), 7.35(1H), 7.65(1H), 7.7(1H) und 7.85(1 H) ppm.

### Beispiel 10

### 4-(1-Methyl-piperidin-4-yl)-piperazin-1-kearbonsäure-[1-(2,4-dimethoxy-benzolsulfonyl)-5-fluoro-2-oxo-3-(2-methoxyphenyl)-2,3-dihydro-1H-indol-3-yl]-ester

¹H-NMR (D₆-DMSO): δ = 1.2-1.5(4H), 1.65(2H), 1.85(2H), 2.1(3H), 2,15(1H), 2,3(2H), 2.75(2H), 3.2(2H), 3.5(2H), 3,55(3H), 3,6(3H), 3,85(3H), 6,65(1H), 6,7(1H), 6.95(1H), 7,0(1H), 7,1(1H), 7.2(1 H), 7.4(1H), 7.7(1H), 7.75(1H) und 7,85(1 H) ppm.

### Beispiel 11

### 4-(1-Methyl-piperidin-4-yl)-piperazin-1-karbonsäure-[1-(2-cyano-benzol-sulfonyl)-5-methoxy-2-oxo-3-(2-methylphenyl)-2,3-dihydro-1H-indol-3-yl]-ester-dihydrochlorid

¹H-NMR (D₆-DMSO): δ = 2.0(2H), 2.3(2H), 2.4(3H), 2.7(3H), 2.8-3.1(4H), 3.2-3.7(8H), 3.8(3H), 4.1-4.4(1H), 6.8(1H), 6.9(1H), 7.1(2H), 7.2-7.4(3H), 7.8-8.0(3H), 8.1(1H), 8.2(1H) und 10.5(N⁺H, breit) ppm.

### Beispiel 12

### 4-(1-Methyl-piperidin-4-yl)-piperazin-1-karbonsäure-[1-(4-brom-2-methyl-benzolsulfonyl)-5-methoxy-2-oxo-3-(2-methylphenyl)-2,3-dihydro-1H-indol-3-yl]-ester-dihydrochlorid

¹H-NMR (D₆-DMSO): δ = 2.0(2H), 2.3(2H), 2.5(6H), 2.7(3H), 2.9-3.1(4H), 3.1-3.25(1H), 3.25-3.7(6H), 3.75(3H), 3.8(1H), 4.1-4.4(1 H), 6.7(1H), 6.9(1H), 7.15(2H), 7.25(1H), 7.1-7.3(3H), 7.95(1 H) und 10.5(N⁺H, breit) ppm.

### Beispiel 13

### 4-(1-Benzyl-piperidin-4-yl)-piperazin-1-karbonsäure-[1-(2,4-dimethoxy-benzolsulfonyl)-5-methoxy-2-oxo-3-(2-methoxyphenyl)-2,3-dihydro-1H-indol-3-yl]-ester

¹H-NMR (D₆-DMSO): δ = 1.4(2H), 1.65(2H), 1.9(2H), 2.1.2.4(3H), 2.8(2H), 3.2(2H), 3.25-3.4(2H), 3.4(2H), 3.45-3.6(8H), 3.65(3H), 3.85(3H),6.6(1H), 6.65(2H), 6.9(1H), 6.95(1H), 7.05(1H), 7.2-7.4(6H), 7.6(1H), 7.65(1H) und 7.85(1 H) ppm.

### Beispiel 14

### 4-(1-Methyl-piperidin-4-yl)-piperazin-1-karbonsäure-[1-(2,4-dimethoxy-benzolsuffonyl)-5-methyl-2-oxo-3-(2-methylphenyl)-2,3-dihydro-1H-indol-3-yl]-ester-dihydrochlorid

¹H-NMR (D₂O): δ = 2.0(2H), 2.25(3H), 2.3(3H), 2.4(2H), 2.85(3H), 3.1 (2H), 3.25(3H), 3.25-3.5(5H), 3.55(2H), 3.65(2H), 3.8(3H), 3.8-4.1(2H), 6.45(1H), 6.65(1H), 6.75(1H), 7.05(1H), 7.1(1H), 7.2-7.4(4H), 7.6(1 H) und 7.95(1 H) ppm.

### Beispiel 15

### 4-(1-Methyl-piperidin-4-yl)-piperazin-1-karbonsäure-[1-(2,4-dimethoxy-benzolsulfonyl)-5-cyano-2-oxo-3-(2-methylphenyl)-2,3-dihydro-1H-indol-3-yl]-ester-dihydrochlorid

¹H-NMR (D₂O): δ = 1.9(2H), 2.3(3H), 2.4(2H), 2.8(3H), 3.1 (2H), 3.3(3H), 3.3-3.5(4H), 3.5-3.8(6H), 3.8(3H), 3.8-4.1(1H), 6.45(1H), 6.65(1h), 6.7(1H), 7.1(1H), 7.2-7.4(2H), 7.7(1H) und 7.9-8.0(3H) ppm.

### Beispiel 16

### 4-(1-Methyl-piperidin-4-yl)-piperazin-1-karbonsäure-[1-(2,4-dimethoxy-benzolsulfonyl)-5-methoxy-2-oxo-3-(2-methoxyphenyl)-2,3-dihydro-1H-indol-3-yl]-amid-dihydrochlorid

¹H-NMR (D₂O): δ = 1.95(2H), 2.4(2H), 2.85(3H), 3.1(2H), 3.2-3.4(4H), 3.4(3H), 3.55(2H), 3.6(3H), 3.6-3.8(8H), 3.85(3H), 6.6(1 H), 6.7(1H), 6.8-6.95(4H), 7.0(1 H), 7.35(1 H), 7.6(1 H) und 8.0(1 H) ppm.

### Beispiel 17

### 4-(1-Methyl-piperidin-4-yl)-piperazin-1-karbonsäure-[1-(2,4-dimethoxy benzolsulfonyl)-5-methyl-2-oxo-3-(2-methoxyphenyl)-2,3-dihydro-1H-indol-3-yl]-ester dihydrochlorid

¹H-NMR (D₂O): δ = 1.9(2H), 2.1(3H), 2.4(2H), 2.8(3H), 3.1(2H), 3.25(2H), 3.35(2H), 3.4(3H), 3.5(2H), 3.7(3H), 3.75(1H), 3.8(3H), 3.9-4.1(2H), 6.65(1H), 6.7(1H), 6.95(1H), 7.0(1 H), 7.1(1H), 7.2(1 H), 7.4(1H), 7.6(1H), 7.8(1 H) und 8.0(1 H) ppm.

### Beispiel 18

### 4-(1-Methyl-piperidin-4-yl)-piperazin-1-karbonsäure-[1-(2,4-dimethoxy-benzolsulfonyl)-5-methoxy-2-oxo-3-(2-isopropylphenyl)-2,3-dihydro-1 H-indol-3-yl]-ester-dihydrochlorid

¹H-NMR (D₆-DMSO): δ = 1.1(3H), 1.2(3H), 2.05(2H), 2.3(2H), 2.7(3H), 2.8-3.1(4H), 3.2-4.0(15H), 4.1-4.4(1 H), 6.4(1 H), 6.5(1 H), 6.65(1H), 6.85-7.0(1H), 7.1 (1 H), 7.35(1 H), 7.5(1 H), 7.6(2H), 7.85(1 H) und 10.5(N⁺H, breit) ppm.

### Beispiel 19

### 4-(1-Methyl-piperidin-4-yl)-piperazin-1-karbonsäure-[1-(4-methoxy-benzolsulfonyl)-5-methoxy-2-oxo-3-(2-Isopropylphenyl)-2,3-dihydro-1 H-indol-3-yl]-ester-dihydrochlorid

¹H-NMR (D₆-DMSO): δ = 1.1(3H), 1.2(3H), 2.0(2H), 2.3(2H), 2.7(3H), 2.8-3.1(4H), 3.1-3.7(8H), 3.7(1H), 3.75(1H), 3.8(3H), 4.1-4.4(1H), 6.45(1H), 6.9(1H), 6.95-7.15(4H), 7.35(1H), 7.5(1H), 7.75(1H), 7.8(2H) und 10.5(N⁺H, breit) ppm.

### Beispiel 20

### 4-(1-Methyl-piperidin-4-yl)-piperazin-1-karbonsäure-[1-(3,4-dimethoxy-benzol-sulfonyl)-5-methoxy-2-oxo-3-(2-isopropylphenyl)-2,3-dihydro-1 H-indol-3-yl]-ester-dihydrochlorid

¹H-NMR (D₆-DMSO): δ = 1.2(3H), 1.25(3H), 2.0(2H), 2.3(2H), 2.7(3H), 2.8-3.1(4H), 3.2-3.7(8H), 3.65(3H), 3.7(3H), 3.8(3H), 3.9(1H) 4.1-4.4(1H), 6.35(1H), 6.9(1H), 7.0(1H), 7.1(2H), 7.25(1H), 7.35(1H), 7.5(2H), 7.7(1H), und 10.4(N⁺H ,breit) ppm.

### Beispiel 21

### 4-(1-Methyl-piperidin-4-yl)-piperazin-1-karbonsäure-[1-(4-isopropyl-benzol-sulfonyl)-5-methoxy-2-oxo-3-(2-isopropylphenyl)-2,3-dihydro-1H-indol-3-yl]-ester-dihydrochlorid

¹H-NMR (D₆-DMSO): δ = 1.1(6H), 1.2(6H), 2.0(2H), 2.3(2H), 2.7(3H), 2.8-3.1(4H), 3.2-3.70(13H), 3.8(3H), 4.1-4.3(1H), 6.45(1H), 6.9(1H), 7.0(1H), 7.1(1H), 7.3-7.5(3H), 7.5(1H), 7.7-7.9(3H) und 10.6(N⁺H,breit) ppm.

### Beispiel 22

### 4-(1-Methyl-piperidin-4-yl)-piperazin-1-karbonsäure-[1-(2-cyano-benzol-sulfonyl)-5-methoxy-2-oxo-3-(2-isopropylphenyl)-2,3-dihydro-1H-indol-3-yl]-ester-dihydrochlorid

¹H-NMR (D₆-DMSO): δ = 1.05(3H), 1.2(3H), 2.0(2H), 2.3(2H), 2.7(3H), 2.8-3.1(4H), 3.2-3.75(9H), 3.7(3H), 3.8(3H), 4.1-4.4(1H), 6.6(1H), 6.9(1H), 7.05(1H), 7.15(1H), 7.35(1H), 7.45(1H), 7.9(3H), 8.0(1H), 8,15(1H) und 10.5(N⁺H, breit) ppm.

### Beispiel 23

### 4-(1-Methyl-piperidin-4-yl)-piperazin-1-karbonsäure-[1-(2,4-dimethoxy-benzolsulfonyl)-2-oxo-3-(2-methoxyphenyl)-2,3-dihydro-1H-indol-3-yl]-ester dihydrochlorid

¹H-NMR (D₂O): δ = 1.9(2H), 2.4(2H), 2.8(3H), 3.1(2H), 3.25(2H), 3.4(3H), 3.4-3.6(4H), 3.6(3H), 3.7(1H), 3.85(3H), 3.9-4.1 (2H), 6.65(1H), 6.7(1H), 6.9(1H), 7.1(3H), 7.4(2H), 7.7(1H), 7.8(1H) und 8.0(1 H) ppm.

### Beispiel 24

### 4-(1-Methyl-piperidin-4-yl)-piperazin-1-karbonsäure-[1-(2,4-dimethoxy-benzolsulfonyl)-5-methoxy-2-oxo-3-(2-isopropoxyphenyl)-2,3-dihydro-1H-indol-3-yl]-ester-dihydrochlorid

¹H-NMR (D₆-DMSO): δ = 0.75(3H), 1.2(3H), 2.0(2H), 2.3(2H), 2.7(3H), 2.8-3.1(6H), 3.1-3.3(6H), 3.4-3.65(9H), 3.7(3H), 3.8(3H), 4.3(1H), 4.6(1H), 6.6(1H), 6.65(2H), 7.35(1H), 7.0(2H), 7.3(1H), 7.7(2H), 7.8(1H), 10.5-11 (N⁺H, breit) ppm.

### Beispiel 25

### 4-(1-Methyl-piperidin-4-yl)piperazin-1-karbonsäure-[1(2-cyano-benzol-sulfonyl)-5-methoxy-2-oxo-3-(2-isopropoxyphenyl)-2,3-dihydro-1H-indol-3-yl]-ester-dihydrochlorid

¹H-NMR (D₆-DMSO): δ = 0.7(3H), 1.2(3H), 2.0(2H), 2.25(2H), 2.7(3H), 2.9-3.2(6H), 3.2-3.6(6H), 3.65(3H), 4.3(1H), 4.6(1 H), 6.6(1H), 7.0(3H), 7.35(1 H), 7.75(1 H), 7.8-8.0(3H), 8.1 (1 H), 8.2(1 H), 10.5-11 (N⁺H, breit) ppm.

### Beispiel 26

### 4-(1-Methyl-piperidin-4-yl)-piperazin-1-karbonsäure-[1-(2-methoxy-4-methylbenzol)-sulfonyl-5-methoxy-2-oxo-3-(2-isopropoxyphenyl)-2,3-dihydro-1H-indol-3-yl]-ester-dihydrochlorid

¹H-NMR (D₆-DMSO): δ = 0.7(3H), 1.2(3H), 2.0(2H), 2.3(2H), 2.4(3H), 2.7(3H), 2.8-3.0(4H), 3.0-3.3(4H), 3.3-3.6(9H), 3.7(3H), 4.3(1H), 4.5(1H), 6.55(1H). 6.9(1 H), 7.0(4H), 7.35(1H). 7.7-7.8(3H), 10.5-11 (N⁺H, breit) ppm.

### Beispiel 27

### 4-(1-Methyl-piperidin-4-yl)-piperazin-1-karbonsäure-[1-(2-methyl-benzol-sulfonyl)-5-methoxy-2-oxo-3-(2-isopropylphenyl)-2,3-dihydro-1H-indol-3-yl]-ester-dihydrochlorid

¹H-NMR (D₆-DMSO): δ = 1.1(3H), 1.2(3H), 2.0(2H), 2.3(2H), 2.4(3H), 2.7(3H), 2.9-4.1(4H), 3.1-3.3(2H), 3.3-3.9(12H), 4.1-4.3(1H), 6.5(1H), 6.95(1H), 7.0(1H), 7.05(1 H), 7.3-7.45(3H), 7.5(1H), 7.6(1H), 7.7(1 H), 8.05(1 H), 10.5-11 (N⁺H), breit) ppm.

### Beispiel 28

### 4-(1-Methyl-piperidin-4-yl)-piperazin-1-karbonsäure-[1-(2-methyl-benzol-sulfonyl)-5-methoxy-2-oxo-3-(2-isopropoxyphenyl)-2,3-dihydro-1H-indol-3-yl]-ester-dihydrochlorid

¹H-NMR (D₆-DMSO): δ = 0.7(3H), 1.2(3H), 2.0(2H), 2.3(2H), 2.6(3H), 2.7(3H), 2.9-3.1(4H), 3.1-3.3(2H), 3.3-3.7(6H), 3.7(3H), 4.3(1H), 4.6(1H), 6.6(1 H), 7.0(3H), 7.3(1H), 7.4(2H), 7.6(1H), 7.75(2H), 8.1(1H), 10.5-11 (N⁺H, breit) ppm.

### Beispiel 29

### 4-(1-Methyl-piperidin-4-yl)-piperazin-1-karbonsäure-[1-(2-methyl-benzol-sulfonyl)-5-methoxy-2-oxo-3-(2-ethoxyphenyl)-2,3-dihydro-1H-indol-3-yl]-ester-dihydrochlorid

¹H-NMR (D₆-DMSO): δ = 1.0(3H), 2.0(2H), 2.3(2H), 2.6(3H), 2.7(3H), 2.95(2H), 3.0-3.3(2H), 3.3-3.7(8H), 3.7(3H), 3.75(1H), 3.9(1H), 4.3(1H), 6.7(1 H), 7.0(2H), 7.05(1 H), 7.35(1 H), 7.45(2H), 7.60(1 H), 7.65(1 H), 7.7(1 H), 8.1(1 H), 10.3-10.8(N⁺H, breit) ppm.

### Beispiel 30

### 4-(1-Methyl-piperidin-4-yl)-piperazin-1-karbonsäure-[1-(2,4-dimethoxy-benzolsulfonyl)-5-methoxy-2-oxo-3-(2-ethoxyphenyl)-2,3-dihydro-1H-indol-3-yl]-ester-dihydrochlorid

¹H-NMR (D₆-DMSO): δ = 1.05(3H), 2.0(2H), 2.3(2H), 2.7(3H), 2.8-3.25(6H), 3.3-3.7(9H), 3.7(3H), 3.75(1H), 3.85(3H), 3.9(1H), 4.3(1H), 6.6-6.7(3H), 6.95(2H), 7.05(1H), 7.35(1H), 7.7(2H), 7.75(1H) und 10.3-10.8(N⁺H, breit) ppm.

### Beispiel 31

### 4-(1-Methyl-piperidin-4-yl)-piperazin-1-karbonsäure-[1-(2-methyl-benzol-sulfonyl)-5-methoxy-2-oxo-3-(2-methoxyphenyl)-2,3-dihydro-1H-indol-3-yl]-ester-dihydrochlorid

¹H-NMR (D₂O): δ = 1.9(2H), 2.4(2H), 2.6(3H), 2.85(3H), 3.1(2H), 3.2-3.7(15H), 3.9(1H), 4.1(1H), 6.7(1H), 6.95(2H), 7.1(1H), 7.4(1H), 7.45(2H), 7.65(1 H), 7.7(1H), 7.8(1 H) und 8.2(1 H) ppm.

### Beispiel 32

### 4-(1-Methyl-piperidin-4-yl)-piperazin-1-karbonsäure-[1-(3-cyano-benzol-sulfonyl)-5-methoxy-2-oxo-3-(2-ethoxyphenyl)-2,3-dihydro-1H-indol-3-yl]-ester-dihydrochlorid

¹H-NMR (D₆DMSO): δ = 1.1(3H), 2.1(2H), 2.3(2H), 2.7(3H), 2.8-3.1(4H), 3.1-3.3(2H), 3.33.65(6H), 3.65(3H), 3.8(1H), 3.9(1H), 4.3(1H), 6.65(1H), 6.95(2H), 7.05(1H), 7.35(1H), 7.75(1H), 7.8(1H), 7.85(1H), 8.25(1H), 8.3(1 H), 8.35(1 H) und 1.0.5-11(N⁺H, breit) ppm.

### Beispiel 33

### 4-(1-Methyl-piperidin-4-yl)-piperazin-1-karbonsäure-[1-(4-ethyl-benzol-sulfonyl)-5-methoxy-2-oxo-3-(2-ethoxyphenyl)-2,3-dihydro-1H-indol-3-yl]-ester-dihydrochlorid

¹H-NMR (D₆-DMSO): δ = 1.0(3H), 1.2(3H), 2.0(2H), 2.3(2H), 2.7(3H), 2.9-3.1(4H), 3.1-3.3(2H),3.3-3.9(13H), 4.3(1H), 6.6(1H), 6.95(2H), 7.05(1H), 7.4(1 h), 7.5(2H), 7.7(1 H), 7.8(1 H), 7.9(2H) und 10.5-11 (N⁺H, breit) ppm.

### Beispiel 34

### 4-(1-Methyl-piperidin-4-yl)-piperazin-1-karbonsäure-[1-(2-methyl-benzo)-sulfonyl)-5-methoxy-2-oxo-3-(2-propoxyphenyl)-2,3-dihydro-1H-indol-3-yl-ester-dihydrochlorid

¹H-NMR (D₆-DMSO): δ = 0.7(3H), 1.9(2H), 2.05(2H), 2.3(2H), 2.6(3H), 2.7(3H), 2.7-3.7(15H), 3.7-3.9(2H), 4.3(1H), 6.7(1H), 6.95(2H), 7.05(1H), 7.35(1H), 7.4(2H), 7.6(1H), 7.65(1H), 7.75(1H), 8.1(1), und 10.5-11(N⁺H, breit) ppm.

### Beispiel 35

### 4-(1-Methyl-piperidin-4-yl)-piperazin-1-karbonsäure-[1-(2-methoxy-4-methyl-benzolsulfonyl)-5-methoxy-2-oxo-3-(2-propoxyphenyl)-2,3-dihydro-1 H-indol-3-yl]-ester-dihydrochlorid

¹H-NMR (D₆-DMSO): δ = 0.7(3H), 1.5(2H), 2.0(2H), 2.3(2H), 2.4(3H), 2.7(3H), 2.9-3.7(19H), 3.8(1H), 4.3(1H), 6.6(1H), 6.7-7.1 (5H), 7.35(1H), 7.7(2H), 7.8(1 H) und 10.5-11 (N⁺H, breit) ppm.

### Beispiel 36

### 4-(1-Methyl-piperidin-4-yl)-piperazin-1-karbonsäure-[1-benzolsulfonyl-5-methyl-2-oxo-3-phenyl-2,3-dihydro-1H-indol-3-yl]-ester-dihydrochlorid

¹H-NMR (D₆-DMSO): δ = 2.0(2H), 2.2-2.4(2H), 2.3(3H), 2.7(1H), 2.75(3H), 2.9-3.8(11H), 4.2(1H), 7.1 (2H), 7.15(1H), 7.3-7.5(4H), 7.6(2H), 7.75(2H) und 8.0(2H) ppm.

### Beispiel 37

### 4-(1-Methyl-piperidin-4-yl)-piperazin-1-karbonsäure-[1-(2,4-dimethoxy-benzolsulfonyl)-5-methoxy-2-oxo-3-(2-ethylphenyl)-2,3-dihydro-1 H-indol-3-yl]-ester-dihydrochlorid

¹H-NMR (D₂O): δ = 1.1(3H), 1.95(2H), 2.4(2H), 2.75(1H), 2.8-3.0(4H), 3.1(2H), 3.15(3H), 3.2-3.5(4H), 3.5-3.65(4H), 3.65-3.75(8H), 3.8-4.1 (3H), 6.35(1H), 6.5(1H), 6.6(1H), 6.7(1H), 6.9(1H), 7.1(1H), 7.3(1H), 7.4(1H), 7.6(1 H) und 7.9(1 H) ppm.

### Beispiel 38

### 4-(1-Methyl-piperidin-4-yl)-piperazin-1-karbonsäure-[1-(2,4-dimethoxy-benzolsulfonyl)-5-methoxy-2-oxo-3-(2,5-dimethoxyphenyl)-2,3-dihydro-1H-indol-3-yl]-ester-dihydrochlorid

¹H-NMR (D₂O) δ = 1.9-2.1(2H). 2.4(2H), 2.9(3H), 3.1(2H), 3.2-3.6(11H), 3.65(3H), 3.7(3H), 3.8(3H), 3.9(3H), 3.9-4.2(3H), 6.65(1H), 6.7(1H), 6.75(1 H), 6.95(1 H), 7.0(2H), 7.3(1 H), 7.6(1 H) und 8.0(1 H) ppm.

### Beispiel 39

### 4-(1-Methyl-piperidin-4-yl)-piperazin-1-karbonsäure-[1-(2-methyl-benzol-sulfonyl)-5-methoxy-2-oxo-3-(2,5-dimethoxyphenyl)-2,3-dihydro-1H-indol-3-yl]-ester dihydrochlorid

¹H-NMR (D₂O): δ = 1.9-2.1(2H), 2.4-2.6(2H), 2.7(3H), 2.9(3H), 3.1-3.3(2H), 3.3(1H), 3.3-3.6(7H), 3.6-3.75(4H), 3.8(3H), 3.9(3H), 3.9-4.2(2H), 6.9(1H), 7.0(1 H), 7.1(2H), 7.45(1 H), 7.5(2H), 7.7(1 H), 7.8(1 H) und 8.3 ppm.

### Beispiel 40

### 4-(1-Methyl-piperidin-4-yl)-piperazin-1-karbonsäure-[1-(benzolsulfonyl]-5-methoxy-2-oxo-3-(2,5-dimethoxyphenyl)-2,3-dihydro-1H-indol-3-yl]-ester-dihydrochlorid

¹H-NMR (D₂O): δ = 1.95(2H), 2.4(2H), 2.85(3H), 3.1(2H), 3.2(3H), 3.2-3.6(7H), 3.65(3H), 3.7(2H), 3.75(3H), 3.8-4.2(2H), 6.75(1H), 6.8(1H), 6.95(2H), 7.3(1 H), 7.6(2H), 7.75(2H) und 8.1(2H) ppm.

### Beispiel 41

### 4-(1-Methyl-piperidin-4-yl)-piperazin-1-karbonsäure-[1-(4-cyano-1-benzolsulfonyl)-5-chlor-2-oxo-3-(2-ethoxyphenyl)-2,3-dihydro-1H-indol-3-yl]-ester

¹H-NMR (CDCl₃): δ = 1.3(3H), 1.5-1.7(2H), 1.7(2H), 1.9(2H), 2.2-2.35(4H), 2.4(2H), 2.5(2H), 2.8-3.05(4H), 3.55(2H), 3.8(1H), 4.0(1H), 6.8(1H), 6.95(1 H), 7.0(1 H), 7.25-7.35(2H), 7.7(1 H), 7.75(2H), 7.9(1 H) und 8.2(1 H) ppm.

### Beispiel 42

### 4-(1-Methyl-piperidin-4-yl)-piperazin-1-karbonsäure-[1-(2-methoxy-1-benzolsulfonyl)-5-chlor-2-oxo-3-(2-ethoxyphenyl)-2,3-dihydro-1H-indol-3-yl]-ester

¹H-NMR (CDCl₃): δ = 1.25(3H), 1.55(2H), 1.7(2H), 1.9(2H), 2.2-2.9(6H), 2.5(2H), 2.9(2H), 3.05(2H), 3.55(5H), 3.8(1H), 4.05(1H), 6.8(1H), 6.9-7.1 (4H), 7.25-7.35(2H), 7.5(1 H), 7.65(1H), 7.95(1H) und 8.15(1 H) ppm.

### Beispiel 43

### 4-(1-Methyl-piperidin-4-yl)piperazin-1-karbonsäure-[1-(2-methoxy-4-methyl-1-benzolsulfonyl)-5-chlor-2-oxo-3-(2-ethoxyphenyl)-2,3-dihydro-1H-indol-3-yl]-ester

¹H-NMR (CDCl₃): δ = 1.25(3H), 1.55(2H), 1.7(2H), 1.9(2H), 2.2-2.6(11H), 2.9(2H), 3.1(2H), 3.6(5H), 3.8(1H), 4.05(1H), 6.7(1H), 6.8(1H), 6.85(1H), 6.95(2H), 7.3(2H), 7.65(1 H), 7.95(1 H) und 8.0(1 H) ppm.

### Beispiel 44

### 4-(1-Methyl-piperidin-4-yl)-piperazin-1-karbonsäure-[1-benzolsulfonyl-5-chlor-2-oxo-3-(2-ethoxyphenyl)-2,3-dihydro-1H-indol-3-yl]-ester

¹H-NMR (CDCl₃): δ = 1.25(3H), 1.55(2H), 1.75(2H), 1.9(2H), 2.25(4H), 2.3(2H), 2.5(2H), 2.9(2H), 3.0(2H), 3.55(2H), 3.8(1H), 4.0(1H), 6.8(1H), 6.9(1H), 7.0(1H), 7.3(2H), 7.5(2H), 7.6(1H), 7.7(1H), 7.9(1H) und 8.1(1H) ppm.

### Beispiel 45

### 4-(1-Methyl-piperidin-4-yl)-piperazin-1-karbonsäure-[1-(2,4-dimethoxy-1-benzolsulfonyl)-5-chlor-2-oxo-3-(2-ethoxyphenyl)-2,3-dihydro-1H-indol-3-yl]-ester

¹H-NMR (CDCl₃): δ = 1.25(3H), 1.6(2H), 1.7(2H), 1.95(2H), 2.3(4H), 2.3(2H), 2.35(2H), 2.55(2H), 2.9(2H), 3.1(2H), 3.5-3.65(5H), 3.8(1H), 3.85(3H), 4.05(1H), 6.4(1H), 6.5(2H), 6.8(1 H), 6.9-7.0(2H), 7.3(2H), 7.65(1H), 7.9(1H) und 8.05(1 H) ppm.

### Beispiel 46

### 4-(1-Methyl-piperidin-4-yl)-piperazin-1-karbonsäure-[1-(2,4-dimethoxy-1-benzolsulfonyl)-5-chlor-2-oxo-3-(2-methoxyphenyl)-2,3-dihydro-1H-indol-3-yl]-ester-dihydrochlorid

¹H-NMR (D₆-DMSO): δ = 2.05(2H), 2.3(2H), 2.65-2.8(3H), 2.9-3.7(18H), 3.9(3H), 4.3(1H), 6.7(2H), 7.0(1H), 7.1(1H), 7.1(1H), 7.4(1H), 7.45(1H), 7.8(1 H), 7.9(1 H), 10.6(N⁺-H) und 11.7(N⁺-H) ppm.

### Beispiel 47

### 4-(1 -Methyl-piperidin-4-yl)-piperazin-1-karbonsäure-[1-(2-methoxy-4-methyl-1-benzolsulfonyl)-5-chlor-2-oxo-3-(2-ethoxyphenyl)-2,3-dihydro-1H-indol-3-yl]-amid

¹H-NMR (CDCl₃): δ = 1.5(3H), 1.6(2H), 1.75(2H), 1.95(2H), 2.25(4H), 2.35(3H), 2.45(4H), 2.9(2H), 3.2(4H), 3.55(3H), 4.1-4.3(2H), 6.7(1H), 6.8-7.0(5H), 7.25(2H), 7.3(1 H), 7.85(1H) und 8.0(1 H) ppm.

### Beispiel 48

### 4-(1-Methyl-piperidin-4-yl)-piperazin-1-karbonsäure-[1-(2,4,6-trimethyl-1-benzolsulfonyl)-5-chlor-2-oxo-3-(2-ethoxyphenyl)-2,3-dihydro-1H-indol-3-yl]-ester

¹H-NMR (CDCl₃): δ = 1.15(3H), 1.6(2H), 1.7(2H), 1.9(2H), 2.2-2.3(7H), 2.4(2H), 2.5(2H), 2.7(6H), 2.9(2H), 3.1(1H), 3.35(1H), 3.55(1H), 3.6(1H), 3.85(1H), 4.05(1H), 6.8(1H), 6.9(2H), 7.0(2H), 7.2.-7.3(2H), 7.65(1H) und 7.95(1 H) ppm.

### Beispiel 49

### 4-(1-Methyl-piperidin-4-yl)-piperazin-1-karbonsäure-[1-(2,4-dichlor-1-benzolsulfonyl)-5-methoxy-2-oxo-3-(2-ethoxyphenyl)-2,3-dihydro-1H-indol-3-yl]-ester

¹H-NMR (CDCl₃): δ = 1.2(3H), 1.6(2H), 1.75(2H), 1.9(2H), 2.2-2.4(4H), 2.4-2.6(3H), 2.9(2H), 3.1 (2H), 3.6(2H), 3.75(3H), 3.8(1H), 4.05(1H), 6.55(1H), 6.75(1H), 6.85(1H), 7.0(1H), 7.3(1H), 7.35(1H), 7.45(1H), 7.65(1H), 7.9(1H) und 8.3(1H) ppm.

### Beispiel 50

### 4-(1-Methyl-piperidin-4-yl)-piperazin-1-karbonsäure-[1-(2-trifluormethoxy-1-benzolsulfonyl)-5-methoxy-2-oxo-3-(2-ethoxyphenyl)-2,3-dihydro-1H-indol-3-yl]-ester

¹H-NMR (CDCl₃): δ = 1.15(3H), 1.4-1.8(4H), 1.9(2H), 2.2-2.7(8H), 2.8-3.0(3H), 3.05(1 H), 3.55(1 H), 3.7(3H), 3.8(1 H), 4.05(1 H), 6.55(1 H), 6.75(1 H), 6.85(1H), 6.95(1H), 7.2-7.45(3H), 7.55(1 H), 7.65(1 H), 7.9(1 H) und 8.3(1 H) ppm.

### Beispiel 51

### 4-(1-Methyl-piperidin-4-yl)-piperazin-1-karbonsäure-[1-(2-methoxy-1-benzolsulfonyl)-5-methoxy-2-oxo-3-(2-ethoxyphenyl)-2,3-dihydro-1 H-indol-3-yl]-ester

¹H-NMR (CDCl₃): δ = 1.25(3H), 1.4-1.8(4H), 1.9(2H), 2.2-2.6(8H), 2.9(2H), 3.05(2H), 3.5-3.7(4H), 3.75(3H), 3.8(1 H), 4.05(1H), 6.55(1 H), 6.75(1 H), 6.8-7.1(4H), 7.25(1H), 7.5(1H), 7.7(1H), 7.9(1H) und 8.15(1H) ppm.

### Beispiel 52

### 4-(1-Methyl-piperidin-4-yl)-piperazin-1-karbonsäure-[1-benzolsulfonyl-5-methoxy-2-oxo-3-(2-ethoxyphenyl)-2,3-dihydro-1H-indol-3-yl]-ester

¹H-NMR (CDCl₃): δ = 1.25(3H), 1.5-1.8(4H), 1.9(2H), 2.2-2.4(6H), 2.5(2H), 2.9(2H), 3.0(2H), 3.55(2H), 3.7(3H), 3.75(1H), 4.0(1H), 6.5(1H), 6.75(1H), 6.8(1H), 7.0(1H), 7.25(1H), 7.45(1H), 7.6(1H), 7.7(1H), 7.85(1H) und 8.15(1H) ppm.

### Beispiel 53

### 1-(2,4-Dimethoxy-1-benzolsulfonyl]-5-methoxy-3-(2-methoxyphenyl)-3{2[4-methyl-piperidin-1-yl)-piperazin-1-yl]-2-oxo-ethoxy}-1,3-dihydroindolon-dihydrochlorid

¹H-NMR (D₆-DMSO): δ = 2.1(2H), 2.3(2H), 2.7(3H), 2.8-3.3(6H), 3.3-3.7(8H), 3.7-4.0(12H), 4.2(1H), 6.5(1H), 6.8(2H), 6.95(1H), 7.1-7.15(2H), 7.35(1H), 7.7(1 H), 7.75(1 H), 7.95(1 H), 10.6(N⁺-H) und 11.8(N⁺-H) ppm.

### Beispiel 54

### 1-(2,4-Dimethoxy-1-benzolsulfonyl)-5-methoxy-3-(2-methoxyphenyl)-3{2[4-methyl-piperazin-1-yl)-pipeddin-1-yl]-2-oxo-ethoxy}-1,3-dihydroindolon

¹H-NMR (D₆-DMSO): δ = 1.5(2H), 2.1(2H), 2.8(3H), 2.9(1H), 3.25-3.8(22H), 3.9(3H), 4.3(1 H), 6.5(1 H), 6.75(2H), 6.95(1 H), 7.05(1 H), 7.15(1 H), 7.35(1 H), 7.75(1 H), 7.8(1 H) und 7.95(1 H) ppm.

### Beispiel 55

### 4-(4-Methyl-piperazin-1-yl)-piperidin-1-karbonsäure-[1-(2,4-dimethoxy-1-benzolsulfonyl]-5-methoxy-2-oxo-3-(2-methoxyphenyl)-2,3-dihydro-1H-indol-3-yl]-ester-dihydrochlorid

¹H-NMR (D₆-DMSO): δ = 1.4(1H), 1.6(1H), 2.1(2H), 2.7(1H), 2.8(3H), 3.0(1 H), 3.25-3.8(19H), 3.9(3H), 4.3(1H), 6.6(1H), 6.65(1H), 6.9-7.0(2H), 7.05(1 H), 7.35(1 H), 7.65(1 H), 7.7(1 H) und 7.9(1H) ppm.

### Beispiel 56

### 4-(1-Methyl-piperidin-4-yl)-piperazin-1-karbonsäure-[1-(2-methyl-1-benzolsulfonyl]-5-methoxy-2-oxo-3-[2-(2-methoxyethyl)phenyl]-2,3-dihydro-1 H-indol-3-yl]-ester-dihydrochlorid

¹H-NMR (D₆-DMSO): δ = 2.15(2H), 2.3(2H), 2.4(3H), 2.7(3H), 2.9-3.2(5H), 3.25-3.7(10H), 3.8(3H), 3.85(1H), 4.25(1H), 6.55(1H), 7.0(1H), 7.1(2H), 7.30-7.5(4H), 7.6(1 H), 7.7(1 H) und 8.05(1 H) ppm.

### Beispiel 57

### 4-(1-Methyl-pipeddin-4-yl)-piperazin-1-karbonsäure-[1-(2-methoxy-4-methyl-1-benzolsulfonyl]-5-methoxy-2-oxo-3-[2-(2-methoxyethyl)phenyl]-2,3-dihydro-1 H-indol-3-yl]-ester-dihydrochlorid

¹H-NMR (D₆-DMSO): δ = 2.1 (2H), 2.3(2H), 2.35(3H), 2.7(3H), 2.9-3.2(5H), 3.25-3.7(16H), 3.8(3H), 3.85(1H), 4.25(1H), 6.5(1H), 6.8-7.0(3H), 7.1 (2H), 7.35(1 H), 7.4(1 H), 7.65(1 H), 7.75(1 H), 10.6(N⁺-H) und 11.8(N⁺-H) ppm.

### Beispiel 58

### 4-(1-Methyl-piperidin-4-yl)-piperazin-1-karbonsäure-[1-(2,4-dimethoxy-1-benzolsulfonyl]-5-methoxy-2-oxo-3-[2-(2-methoxyethyl)phenyl]-2,3-dihydro-1 H-indol-3-yl]-ester-dihydrochlorid

¹H-NMR (D₆-DMSO): δ = 2.1(2H), 2.3(2H), 2.7(3H), 2.8-3.2(5H), 3.25-3.7(16H), 3.75(3H), 3.8-3.9(4H), 4.25(1H), 6.45(1H), 6.55(1H), 6.65(1H), 6.9(1H), 7.1(2H), 7.35(1H), 7.4(1H), 7.65(1H), 7.75(1H), 10.6(N⁺-H) und 11.8(N⁺-H) ppm.

### Beispiel 59

### 4-(1-Methyl-piperidin-4-yl)-piperazin-1-karbonsäure-[5-chloro-1-(2,4-dimethoxy-benzolsulfonyl)-3-(2-methoxymethyl-phenyl)-2-oxo-2,3-dihydro-1H-indol3-yl]-ester

¹H-NMR (CDCl₃): δ = 1.6(2H), 1.75(2H), 1.95(2H), 2.2-2.3(4H), 2.35-2.6(4H), 2.9(2H), 3.25(2H), 3.4(6H), 3.55(2H), 3.85(3H), 4.7(1H), 5.0(1H), 6.35(1H), 6.55(2H), 7.05-7.15(1H), 7.35(1H), 7.4(1H), 7.65(1H), 7.9(1H) und 8.0(1H) ppm.

### Beispiel 60

### 4-(1-Methyl-piperidin-4-yl)-piperazin-1-karbonsäure-[1-(2,4-dimethoxy-benzolsulfonyl)-5-methoxy-2-oxo-3-(2-ethoxyphenyl)-2,3-dihydro-1H-indol-3-yl]-amid

¹H-NMR (CDCl₃): δ = 1.5-1.7(5H), 1.75(2H), 1.9(2H), 2.15-2.35(4H), 2.45(4H), 2.9(2H), 3.25(4H), 3.55(3H), 3.75(3H), 3.85(3H), 4.2(2H), 6.4(1H), 6.55(1 H), 6.75-6.95(5H), 7.05(1 H), 7.2(1 H), 7.8(1 H) und 8.1 (1 H) ppm.

### Beispiel 61

### 4-(1-Methyl-piperidin-4-yl)-piperazin-1-karbonsäure-[1-benzolsulfonyl-5-methoxy-2-oxo-3-(2-ethoxyphenyl)-2,3-dihydro-1H-indol-3-yl]-amid dihydrochlorid

¹H-NMR (D₆-DMSO): δ = 1.25(3H), 2.0(2H), 2.3(2H), 2.75(3H), 2.75-3.0(4H), 3.15(2H), 3.3-3.6(4H), 3.65(3H), 3.75-4.1(4H), 6.85(2H), 6.85-7.0(2H), 7.25-7.45(2H), 7.6(3H), 7.75(1H), 7.9(1H), 8.05(1H), 10.4(N⁺-H) und 11.1(N⁺-H) ppm.

### Beispiel 62

### 4-(1-Methyl-piperidin-4-yl)-piperazin-1-karbonsäure-[1-benzolsulfonyl-5-cyano-2-oxo-3-(2-ethoxyphenyl)-2,3-dihydro-1H-indol-3-yl]-ester

¹H-NMR (CDCl₃): δ = 1.2(3H), 1.4-2.1(6H), 2.3-2.7(8H), 2.9(2H), 3.15(2H), 3.6(2H), 3.75(1 H), 4.0(1 H), 6.8(1 H), 7.0(1 H), 7.25(1 H), 7.3(1 H), 7.5(2H), 7.6-7.75(3H) und 8.1(3H) ppm.

### Beispiel 63

### 4-(1-Methyl-piperidin-4-yl)-piperazin-1-karbonsäure-[1-(2,4-dimethoxy-1 benzolsulfonyl)-5-cyano-2-oxo-3-(2-ethoxyphenyl)-2,3-dihydro-1H-indol-3-yl]-ester

¹H-NMR (CDCl₃): δ = 1.25(3H), 1.5-1.9(6H), 2.1(1 H), 2.25-2.5(4H), 2.55(2H), 2.95-3.2(4H), 3.55(3H), 3.6(2H), 3.8(1 H), 3.85(3H), 4.05(1H), 6.4(1H), 6.55(1H), 6.8(1 H), 7.0(1H), 7.25(2H), 7.65(2H), 8.05(1H) und 8.15(1H) ppm.

### Beispiel 64

### 4-(1-Methyl-piperidin-4-yl)-piperazin-1-karbonsäure-[1-(2,4-dichlor-1-benzolsulfonyl)-5-cyano-2-oxo-3-(2-ethoxyphenyl)-2,3-dihydro-1H-indol-3-yl]-ester

### Beispiel 65

### 4-(1-Methyl-piperidin-4-yl)-piperazin-1-karbonsäure-[1-(2-methoxy-1-benzolsulfonyl)-5-cyano-2-oxo-3-(2-ethoxyphenyl)-2,3-dihydro-1H-indol-3-yl]-ester

¹H-NMR (CDCl₃): δ = 1.25(3H), 1.5-1.9(6H), 2.1(1 H), 2.25-2.5(4H), 2.55(2H), 2.95-3.2(4H), 3.55(3H), 3.6(2H), 3.8(1H), 3.85(3H), 4.05(1H), 6.4(1H), 6.55(1 H), 6.8(1 H), 7.0(1 H), 7.25(2H), 7.65(2H), 8.05(1 H) und 8.15(1 H) ppm.

### Beispiel 66

### 4-(1-Methyl-piperidin-4-yl)-piperazin-1-karbonsäure-[1-(2,4-dimethoxy-benzolsulfonyl)-5-chlor-2-oxo-3-(2-ethoxyphenyl)-2,3-dihydro-1H-indol-3-yl]-amid dihydrochlorid

¹H-NMR (D₆-DMSO): δ = 1.2(3H), 2.1(2H), 2.3(2H), 2.7(3H), 2.9(4H), 3.2(2H), 3.3-3.7(8H), 3.8-4.0(5H), 4.05(2H), 6.65(2H), 6.9(1 H), 7.0(1H), 7.2(1 H), 7.25-7.4(3H), 7.7(1H), 7.9(2H), 10.5(N⁺-H) und 11.3(N⁺-H) ppm.

### Beispiel 67

### 4-(1-Methyl-piperidin-4-yl)-piperazin-1-karbonsäure-[1-benzolsulfonyl-5-chlor-2-oxo-3-(2-ethoxyphenyl)-2,3-dihydro-1H-indol-3-yl]-amid dihydrochlorid

¹H-NMR (D₆-DMSO): δ = 1.1(3H), 2.1(2H), 2.3(2H), 2.7(3H), 2.8-3.0(4H), 3.2(2H), 3.3-3.7(4H), 3.8-4.0(4H), 6.95(2H), 7.2(1H), 7.35(2H), 7.55(1H), 7.65(2H), 7.7-7.85(2H), 7.95(1H), 8.05(1H), 10.5(N⁺-H) und 11.3(N⁺-H) ppm.

### Beispiel 68

### 4-(1-Methyl-piperidin-4-yl)-piperazin-1-karbonsäure-[1-(2,4,6-trimethyl-1-benzolsulfonyl)-5-chlor-2-oxo-3-(2-ethoxyphenyl)-2,3-dihydro-1H-indol-3-yl]-amid dihydrochlorid

¹H-NMR (D₆-DMSO): δ = 1.2(3H), 2.05(2H), 2.3(6H), 2.6(3H), 2.85-3.05(4H), 3.2(2H), 3.4-3.7(4H), 3.95(2H), 4.05(2H), 6.85(2H), 7.0(1H), 7.1(3H), 7.3(1 H), 7.35(1H), 7.45(1H), 7.75(1H), 7.95(1H), 10.5(N⁺-H) und 11.2(N⁺-H) ppm.

### Beispiel 69

### 4-(1-Methyl-piperidin-4-yl)-piperazin-1-karbonsäure-[1-(4-isopropyl-1-benzolsulfonyl)-5-chlor-2-oxo-3-(2-ethoxyphenyl)-2,3-dihydro-1H-indol-3-yl]-amid

### Beispiel 70

### 4-(1-Methyl-piperidin-4-yl)-piperazin-1-karbonsäure-[1-(4-cyano-1-benzolsulfonyl)-5-chlor-2-oxo-3-(2-ethoxyphenyl)-2,3-dihydro-1H-indol-3-yl]-amid

¹H-NMR (CDCl₃): δ = 1.5(3H), 1.6(2H), 1.75(2H), 1.95(2H), 2.2-2.3(4H), 2.45(4H), 2.9(2H), 3.05-3.25(4H), 4.1(1H), 4.2(1 H), 6.5(1H). 6.9(2H), 7.1(1H), 7.15(1 H), 7.25-7.35(2H), 7.75(2H), 7.85(1 H) und 8.2(1 H) ppm.

### Beispiel 71

### 4-(1-Methyl-piperidin-4-yl)-piperazin-1-karbonsäure-[5-chlor-3-(2-ethoxyphenyl)-1-(2-methoxy-benzolsulfonyl)-2-oxo-2,3-dihydro-1H-indol-3-yl]-amid

¹H-NMR (CDCl₃): δ = 1.5(3H), 1.6(2H), 1.75(2H), 1.95(2H), 2.2-2.35(4H), 2.45(4H), 2.9(2H), 3.25(4H), 3.6(3H), 4.1-4.25(2H), 6.75-6.85(2H), 6.9(3H), 7.1(1H), 7.25(2H), 7.35(1H), 7.55(2H), 7.9(1H) und 8.15(1H) ppm.

### Beispiel 72

### 4-(1-Methyl-piperidin-4-yl)-piperazin-1-karbonsäure-[1-(2,4-dimethoxy-benzolsulfonyl)-3-(2-ethoxy-phenyl)-5-fluoro-2-oxo-2,3-dihydro-1H-indol-3-yl]-ester

¹H-NMR (CDCl₃): δ = 1.25(3H), 1.65(2H), 1.75(2H), 2.05(2H), 2.2-2.7(8H), 2.95(2H), 3.1(2H), 3.6(5H), 3.75-3.9(4H), 4.05(1H), 6.4(1H), 6.55(1H), 6.7(1 H), 6.8(1H), 6.9-7.1 (2H), 7.3(1 H), 7.65(1 H), 7.95(1 H) und 8.1(1H) ppm.

### Beispiel 73

### 4-(1-Methyl-piperidin-4-yl)-piperazin-1-karbonsäure-[1-benzolsulfonyl-3-(2-ethoxy-phenyl)-5-fluoro-2-oxo-2,3-dihydro-1H-indol-3-yl]-ester

¹H-NMR (CDCl₃): δ = 1.25(3H), 1.65(2H), 1.75(2H), 2.0(2H), 2.2-2.7(8H), 2.85-3.15(4H), 3.6(2H), 3.8(1 H), 4.0(1 H), 6.7(1 H), 6.8(1 H), 7.0(2H), 7.3(1 H), 7.5(2H), 7.6(1 H), 7.7(1 H), 7.9(1 H) und 8.15(2H) ppm.

### Beispiel 74

### 4-(1-Methyl-piperidin-4-yl)-piperazin-1-karbonsäure-[3-(2-ethoxy-phenyl)-5-methoxy-1-(4-methoxy-benzolsulfonyl)-2-oxo-2,3-dihydro-1H-indol-3-yl]-ester

¹H-NMR (CDCl₃): δ = 1.25(3H), 1.4-1.9(6H), 2.2(2H), 2.25-2.7(6H), 2.9-3.2(4H), 3.6(2H), 3.8(1H), 3.85(3H), 4.0(1 H), 6.8(1H), 6.95(2H), 7.05(1H), 7.3(1 H), 7.35(1 H), 7.65(1 H), 7.7(1 H) und 8.05(3H) ppm.

### Beispiel 75

### 4-(1-Methyl-piperidin-4-yl)-piperazin-1-karbonsäure-[3-(2-isopropoxy-phenyl)-5-methoxy-1-(4-methoxy-benzolsulfonyl)-2-oxo-2,3-dihydro-1H-indol-3-yl]-ester

¹H-NMR (CDCl₃): δ = 0.9(3H), 1.35(3H), 1.5-1.7(4H), 1.95(2H), 2.2-2.4(6H), 2.5(2H), 2.9(2H), 3.0(2H), 3.6(2H), 3.7(3H), 3.8(3H), 4.5(1H), 6.5(1H), 6.8(1H), 6.85(1H), 6.9-7.0(3H), 7.25(1H), 7.7(1H), 7.85(2H) und 8.05(2H) ppm.

### Beispiel 76

### 4-(1-Methyl-piperidin-4-yl)-piperazin-1-karbonsäure-[5-methoxy-1-(4-methoxy-benzolsulfonyl)-2-oxo-3-(2-propoxy-phenyl)-2,3-dihydro-1H-indol-3-yl]-ester

¹H-NMR (CDCl₃): δ = 0.8(3H), 1.5-1.85(6H), 1.9(2H), 2.2-2.3(4H), 2.35(2H), 2.5(2H), 2.9(2H), 3.05(2H), 3.6(2H), 3.65-3.75(4H), 3.85(3H), 3.9(1H), 6.55 (1H), 6.8(2H), 6.9(2H), 7.0(1 H), 7.25(1H), 7.7(1H), 7.85(1H) und 8.15(2H) ppm.

### Beispiel 77

### 4-(-1-Methyl-piperidin-4-yl)-piperazin-1-karbonsäure-[5-chloro-3-(2-ethoxy phenyl)-1-(4-methoxy-benzolsulfonyl)-2-oxo-2,3-dihydro-1H-indot-3-yl]-amid

¹H-NMR (CDCl₃): δ = 1.5-1.75(5H), 1.75(2H), 1.9(2H), 2.2-2.35(4H), 2.4(4H), 2.9(2H), 3.15-3.3(4H), 3.85(3H), 4.15(1 H), 4.2(1 H), 6.65(1H), 6.85-7.05(5H), 7.2-7.3(3H), 7.8(1 H) und 8.15(2H) ppm.

### Beispiel 78

### 4-(1-Methyl-piperidin-4-yl)-piperazin-1-karbonsäure-{5-methoxy-1-(2-methoxy-benzolsulfonyl)-3-[2-(2-methoxy-ethyl)-phenyl]-2-oxo-2,3-dihydro-1H-indol-3-yl}-amid dihydrochlorid

¹H-NMR (D₆-DMSO): δ = 2.05(2H), 2.3(2H), 2.7(3H), 2.85-3.2(5H), 3.25-3.7(16H), 3.75(3H), 3.85(1H), 4.25(1H), 6.5(1H), 6.9(1H), 7.25(1H), 7.4(1H), 7.75(2H), 7.9(1 H), 10.S(N⁺-H) und 11.7(N⁺-H) ppm.

### Beispiel 79

### 4-(1-Methyl-piperidin-4-yl)-piperazin-1-karbonsäure-[5-methoxy-1-(2-methoxy-benzolsulfonyl)-2-oxo-3-(2-propoxy-phenyl)-2,3-dihydro-1H-indol-3-yl]-ester

¹H-NMR (CDCl₃): δ = 0.8(3H), 1.4-1.85(6H), 1.9(2H), 2.2-2.4(6H), 2.5(2H), 2.9(2H), 3.1(2H), 3.55(2H), 3.6(3H), 3.7(4H), 3.95(1H), 6.55(1H), 6.65-7.05(5H), 7.25(1H), 7.5(1H), 7.65(1H), 7.9(1 H) und 8.1(1H) ppm.

### Beispiel 80

### 4-(1-Methyl-piperidin-4-yl)-piperazin-1-karbonsäure-[3-(2-isopropoxy-phenyl)-5-methoxy-1-(2-methoxy-benzolsulfonyl)-2-oxo-2,3-dihydro-1H-indol-3-yl]-ester

¹H-NMR (CDCl₃): δ = 0.9(3H), 1.4(3H), 1.5-1.8(4H), 1.95(2H), 2.2-2.45(6H), 2.5(2H), 2.9(2H), 3.05(2H), 3.45-3.65(5H), 3.7(3H), 4.5(1H). 6.5(1H), 6.8(1H), 6.85-6.95(3H), 7.0(1H), 7.25(1H), 7.5(1H), 7.7(1H), 7.9(1H) und 8.15(1H) ppm.

### Beispiel 81

### 4-(1-Methyl-piperidin-4-yl)-piperazin-1-karbonsäure-[5-methoxy-1-(4-methoxy-benzolsulfonyl)-3-(2-methoxymethyl-phenyl)-2-oxo-2,3-dihydro-1H-indol-3-yl]-ester

¹H-NMR (CDCl₃): δ = 1.5-1.9(4H), 2.0(2H), 2.2-2.6(8H), 2.95(2H), 3.2(2H), 3.3-3.7(9H), 3.75(3H), 3.85(3H), 6.6(1 H), 6.7(1H), 6.85-7.05(4H), 7.25(1 H), 7.35(1 H), 7.85(1H) und 8.20(1 H) ppm.

### Beispiel 82

### 4-(1-Methyl-piperidin-4-yl)-piperazin-1-karbonsäure-[1-benzolsulfonyl-5-chloro-3-(2-methoxymethyl-phenyl)-2-oxo-2,3-dihydro-1H-indol-3-yl]-ester

¹H-NMR (CDCl₃): δ = 1.4-1.8(6H), 1.9(2H), 2.2-2.3(4H), 2.35(1H), 2.4(2H), 2.55(1H), 2.9(2H), 3.15(2H), 3.4(3H), 3.55(2H), 4.75(1H), 5.0(1H), 6.55(1H), 7.05-7.15(2H), 7.3-7.55(4H), 7.6-7.7(2H), 7.95(1 H) und 8.05(1 H) ppm.

### Beispiel 83

### 4-(4-Methyl-piperazin-1-yl)-piperidin-1-karbonsäure-[3-(2-ethoxy-phenyl)-5-methoxy-1-(2-methoxy-benzolsulfonyl)-2-oxo-2,3-dihydro-1 H-indol-3-yl]-ester

¹H-NMR (CDCl₃): δ = 1.1(1H), 1.25(3H), 1.35-1.9(5H), 2.25-2.7(11H), 2.9(1H), 3.6(3H), 3.65(1 H), 3.7(3H), 3.75(1 H), 4.05(1 H), 4.25(1 H), 6.55(1 H), 6.75(1 H), 6.8-7.1 (4H), 7.3(1 H), 7.5(1 H), 7.7(1 H), 7.9(1 H) und 8.15(1 H) ppm.

### Beispiel 84

### 4-(4-Methyl-piperazin-1-yl)-piperidin-1-karbonsäure-[1-(2,4-dimethoxy-benzolsulfonyl)-3-(2-ethoxy-phenyl)-5-methoxy-2-oxo-2,3-dihydro-1H-indol-3-yl]-ester

¹H-NMR (CDCl₃): δ = 1.2(1H), 1.25(3H), 1.35-1.9(5H), 2.25-2.7(11H), 2.9(1H), 3.55(3H), 3.65-3.7(4H), 3.8(1H), 3.85(3H), 4.05(1H), 4.25(1H), 6.4(1 H), 6.55(2H), 6.75(1 H), 6.85(1 H), 6.95(1 H), 7.25(1 H), 7.7(1 H), 7.85(1 H) und 8.05(1 H) ppm.

### Beispiel 85

### 4-(4-Methyl-piperazin-1-yl)-piperidin-1-karbonsäure-[3-(2-ethoxy-phenyl)-5-methoxy-1-(4-methoxy-benzolsulfonyl)-2-oxo-2,3-dihydro-1H-indol-3-yl]-ester

¹H-NMR (CDCl₃): δ = 1.25(3H), 1.35-1.9(6H), 2.25-2.7(11H), 2.9(1H), 3.6(1H), 3.7(3H), 3.8(1H), 3.85(3H), 4.0(1H), 4.25(1H), 6.5(1H), 6.75(1H), 6.8(1H), 6.95(2H), 7.0(1 H), 7.25(1H), 7.7(1 H), 7.85(1 H) und 8.1(2H) ppm.

### Beispiel 86

### 4-(4-Methyl-piperazin-1-yl)-piperidin-1-karbonsäure-[3-(2-isopropoxy-phenyl)-5-methoxy-1-(2-methoxy-benzolsulfonyl)-2-oxo-2,3-dihydro-1H-indol-3-yl]-ester

¹H-NMR (CDCl₃): δ = 0.85(3H), 1.1(1H), 1.25-1.9(11H), 2.2-2.7(11H), 2.85(1H), 3.5-3.65(4H), 3.7(3H), 4.2(1H), 4.5(1H), 6.5(1H), 6.75(1H), 6.8-7.1(5H), 7.25(1 H), 7.7(1 H), 7.9(1 H) und 8.15(2H) ppm.

### Beispiel 87

### 4-(4-Methyl-piperazin-1-yl)-piperidin-1-karbonsäure-[1-(2,4-dimethoxy-benzolsulfonyl)-3-(2-isopropoxy-phenyl)-5-methoxy-2-oxo-2,3-dihydro-1H-indol-3-yl]-ester

¹H-NMR (CDCl₃): δ = 0.85(3H), 1.1-1.9(9H), 2.2-2.75(11H), 2.9(1 H), 3.6(4H), 3.65-3.75(4H), 3.8(3H), 4.25(1H), 4.5(1H), 6.4(1H), 6.5(2H), 6.75(1H), 6.85(1 H), 6.9(1 H), 7.25(1 H), 7.7(1 H), 7.9(1 H) und 8.1 (1 H) ppm.

### Beispiel 88

### 4-(4-Methyl-piperazin-1-yl)-piperidin-1-karbonsäure-[5-chlor-3-(2-ethoxyphenyl)-1-(4-methoxy-benzolsulfonyl)-2-oxo-2,3-dihydro-1H-indol-3-yl]-ester

¹H-NMR (CDCl₃): δ = 1.25(3H), 1.35-2.0(6H), 2.2-2.75(10H), 2.9(1H), 3.6(1H), 3.75-3.9(4H), 4.0(1H), 4.25(1H), 6.8(1H), 6.95(3H), 7.0(1H), 7.3(2H), 7.7(1 H), 7.85(1 H) und 8.05(1H) ppm.

### Beispiel 89

### 4-(4-Methyl-piperazin-1-yl)-piperidin-1-karbonsäure-[3-(2-isopropoxy-phenyl)-5-methoxy-1-(4-methoxy-benzolsulfonyl)-2-oxo-2,3-dihydro-1H-indol-3-yl]-ester .

¹H-NMR (CDCl₃): δ = 0.85(1H). 1.1-1.9(9H), 2.2-2.7(11 H), 2.9(1 H), 3.6(1 H), 3.7(3H), 3.85(3H), 4.25(1 H), 4.5(1 H), 6.5(1H), 6.8(1H), 6.85(1 H), 6.85-7.0(3H), 7.3(1 H), 7.7(1 H), 7.85(1 H) und 8.05(2H) ppm.

### Beispiel 90

### 4-(4-Methyl-piperazin-1-yl)-piperidin-1-karbonsäure-[5-chlor-3-(2-ethoxyphenyl)-1-(2-methoxy-benzolsulfonyl)-2-oxo-2 ,3-dihydro-1H-indol-3-yl]-ester

¹H-NMR (CDCl₃): δ = 1.1 (1H), 1.25(3H), 1.35-1.9(5H), 2.2-2.7(11H), 2.9(1 H), 3.5-3.7(4H), 3.8(1H), 4.05(1H), 4.25(1 H), 6.8(1 H), 6.9-7.1 (4H), 7.3(2H), 7.5(1 H), 7.65(1 H), 7.95(1 H) und 8.15(1 H) ppm.

### Beispiel 91

### 4-(4-Methyl-piperazin-1-yl)-piperidin-1-karbonsäure-[5-chlor-1-(2,4-dimethoxy-benzolsulfonyl)-3-(2-ethoxy-phenyl)-2-oxo-2,3-dihydro-1H-indol-3-yl]-ester

¹H-NMR (CDCl₃): δ = 0.85(3H), 1.0-1.9(6H), 2.25(3H), 2.25-2.7(8H), 2.9(1H), 3.55(3H), 3.65(1H), 3.75-3.85(4H), 4.05(1H), 4.2(1H), 6.4(1H), 6.5(1H), 6.75(1 H), 6.9-7.1(2H), 7.3(2H), 7.7(1 H), 7.9(1 H) und 8.05(1 H) ppm.

### Beispiel 92

### 4-(1-Methyl-piperidin-4-yl)-piperazin-1-karbonsäure-[1-(2-methoxy-benzolsulfonyl)-3-(2-methoxy-phenyl)-2-oxo-6-trifluoromethyl-2,3-dihydro-1H-indol-3-yl]-ester

### Beispiel 93

### 4-(1-Methyl-piperidin-4-yl)-piperazin-1-karbonsäure-[3-(2-ethoxy-phenyl)-5-fluoro-1-(2-methoxy-benzolsulfonyl)-2-oxo-2,3-dihydro-1H-indol-3-yl]-ester

¹H-NMR (CDCl₃): δ = 1.25(3H), 1.5-1.8(4H), 1.9(2H), 2.2-2.6(8H), 2.9(2H), 3.1(2H), 3.5-3.7(5H), 3.8(3H), 4.05(3H), 6.7(1H), 6.8(1H), 6.9-7.1(4H), 7.25(1 H), 7.5(1 H), 7.65(1H), 7.95(1 H) und 8.150(1 H) ppm.

### Beispiel 94

### 4-(1-Methyl-piperidin-4-yl)-piperazin-1-karbonsäure-[3-(2-ethoxy-phenyl)-5-fluoro-1-(4-methoxy-benzolsulfonyl)-2-oxo-2,3-dihydro-1H-indol-3-yl]-ester dihydrochlorid

¹H-NMR (D₆-DMSO): δ = 1.0(3H), 2.05(2H), 2.3(2H), 2.7(3H), 2.8-3.9(17H), 4.3(1H), 6.95-7.05(2H), 7.1(2H), 7.25(1H), 7.35(1H), 7.75(2H), 7.95(2H), 10.5(NH⁺) und 11.6(NH⁺) ppm.

### Beispiel 95

### 4-(1-Methyl-piperidin-4-yl)-piperazin-1-karbonsäure-[1-(4-cyano-benzolsulfonyl)-3-(2-ethoxy-phenyl)-5-fluoro-2-oxo-2,3-dihydro-1H-indol-3-yl]-ester

¹H-NMR (CDCl₃): δ = 1.3(3H), 1.5-1.8(4H), 1.9(2H), 2.2-2.6(8H), 2.8-3.05(4H), 3.55(2H), 3.8(1 H), 4.05(3H), 6.7(1 H), 6.8(1 H), 7.05(2H), 7.3(1 H), 7.7(1 H), 7.8(2H), 7.9(1 H) und 8.2(1 H) ppm.

### Beispiel 96

### 4-(1-Methyl-piperidin-4-yl)-piperazin-1-karbonsäure-[5-chlor-3-(2-ethoxyphenyl)-1-(4-methoxy-benzolsulfonyl)-2-oxo-2,3-dihydro-1H-indol-3-yl]-ester dihydrochlorid

¹H-NMR (D₆-DMSO): δ = 2.1(2H), 2.3(2H), 2.7(3H), 2.9(2H), 3.0-3.8(13H), 3.85(3H), 4.25(1H), 4.7(1H), 4.8(1H), 6.5(1H), 7.1(2H), 7.2(1H), 7.4(1H), 7.5(1 H), 7.6(2H), 7.8-7.9(3H), 10.5(NH⁺) und 11.7(NH⁺) ppm.

### Beispiel 97

### 4-(1-Methyl-piperidin-4-yl)-piperazin-1-karbonsäure-[5-chlor-1-(2-methoxy-benzolsulfonyl)-3-(2-methoxymethyl-phenyl)-2-oxo-2,3-dihydro-1H-indol-3-yl]-ester

¹H-NMR (CDCl₃): δ = 1.6(2H), 1.7(2H), 1.9(2H), 2.5(4H), 2.4-2.6(4H), 2.9(2H), 3.25(2H), 3.4(3H), 3.5(3H), 3.6(2H), 4.3(1H), 5.0(1H), 6.55(1H), 6.85(1 H), 7.0-7.2(3H), 7.35(1 H), 7.45(1 H), 7.55(1 H), 7.65(1 H), 7.95(1 H) und 8.1(1H)ppm.

### Beispiel 98

### 4-(1-Methyl-piperidin-4-yl)-piperazin-1-karbonsäure-[3-(2-ethoxy-phenyl)-5-methoxy-1-(2-methoxy-benzolsulfonyl)-2-oxo-2,3-dihydro-1H-indol-3-yl]-amide

¹H-NMR (CDCl₃): δ = 1.4-1.65(5H), 1.75(2H), 1.9(2H), 2.15-2.35(4H), 2.45(4H), 2.9(2H), 3.25(4H), 3.55(3H), 3.75(3H), 4.15(2H), 6.75-6.85(3H), 6.9(3H), 7.0(1 H), 7.05(1 H), 7.2(1 H), 7.5(1 H), 7.8(1 H) und 8.15(1 H) ppm.

### Beispiel 99

### 4-(1-Methyl-piperidin-4-yl)-piperazin-1-karbonsäure-[3-(2-ethoxy-phenyl)-5-methoxy-1-(4-methoxy-benzolsulfonyl)-2-oxo-2,3-dihydro-1H-indol-3-yl]-amid

¹H-NMR (CDCl₃): δ = 1.4-1.6(5H), 1.75(2H), 1.9(2H), 2.15-2.35(4H), 2.35-2.5(4H), 2.9(2H), 3.15-3.35(4H), 3.7(3H), 3.85(3H), 4.1-4.3(2H), 6.7-6.9(8H), 7.25(1 H), 7.75(1 H) und 8.05(1 H) ppm.

### Beispiel 100

### 4-(1-Methyl-piperidin-4-yl)-piperazin-1-karbonsäure-[1-(2,4-dimethoxy-benzolsulfonyl)-3-(2-methoxy-phenyl)-2-oxo-6-trifluoromethyl-2,3-dihydro-1H-indol-3-yl]-ester

### Beispiel 101

### 4-(4-Methyl-piperazin-1-yl)-piperidin-1-karbonsäure-[5-chlor-3-(2-ethoxyphenyl)-1-(4-methoxy-benzolsulfonyl)-2-oxo-2,3-dihydro-1H-indol-3-yl]-amid dihydrochlorid

¹H-NMR (D₆-DMSO): δ = 1.15(3H), 1.3-1.5(2H), 1.9-2.1 (2H), 2.5-2.7(2H), 2.8(3H), 3.2-3.8(9H), 3.8-4.1(7H), 6.95(2H), 7.15(2H), 7.25(1H), 7.3(2H), 7.4(1H), 7.7(1 H), 7.75(1 H) und 7.9(2H) ppm.

### Beispiel 102

### 4-(4-Methyl-piperazin-1-yl)-piperidin-1-karbonsäure-[5-chlor-1-(2,4-dimethoxy-benzolsulfonyl)-3-(2-ethoxy-phenyl)-2-oxo-2,3-dihydro-1H-indol-3-yl]-amid-dihydrochlorid

¹H-NMR (D₆-DMSO): δ = 1.2(3H), 1.3-1.5(2H), 1.9-2.1(2H), 2.65(2H), 2.8(3H), 3.2-3.8(12H), 3.85(3H), 3.95(2H), 4.05(2H), 6.6-6.7(2H), 6.85(1 H), 7.0(1 H), 7.1(1H), 7.25-7.4(3H), 7.7(2H) und 7.9(1 H) ppm.

### Beispiel 103

### 4-(4-Methyl-piperazin-1-yl)-piperidin-1-karbonsäure-[5-chlor-3-(2-ethoxyphenyl)-1-(2-methoxy-benzolsulfonyl)-2-oxo-2,3-dihydro-1H-indol-3-yl]-amid

¹H-NMR (CDCl₃): δ = 1.2-1.4(2H), 1.55(3H), 1.75(2H), 2.2-2.8(14H), 3.55(3H), 3.75(2H), 4.2(2H), 6.8(1 H), 6.85(1H), 6.9-7.0(3H), 7.O5(1H), 7.25(2H), 7.3(1 H), 7.55(1 H), 7.9(1 H) und 8.15(1 H) ppm.

### Beispiel 104

### 4-(4-Methyl-piperidin-1-yl)-piperazin-1-karbonsäure-[5-cyano-3-(2-ethoxyphenyl)-2-oxo-1-(toluene-2-sulfonyl)-2,3-dihydro-1H-indol-3-yl]-ester

¹H-NMR (CDCl₃): δ = 1.2(3H), 1.6(2H), 1.75(2H), 1.95(2H), 2.2-2.45(6H), 2.55(2H), 2.65(3H), 2.9(2H), 3.05(1H), 3.15(1H), 3.55(2H), 3.85(1H), 4.05(1H), 6.8(1 H), 7.0(1 H), 7.3(1 H), 7.35(1H), 7.45(1H), 7.65(1H), 8.05(1 H) und 8.25(1 H) ppm.

### Beispiel 105

### 4-(1-Methyl-piperidin-4-yl)piperazin-1-karbonsäure-[5-cyano-3-(2-ethoxyphenyl)-2-oxo-1-(toluene-4-sulfonyl)-2,3-dihydro-1H-indol-3-yl]-ester

¹H-NMR (CDCl₃): δ = 1.2(3H), 1.6(2H), 1.75(2H), 2.05(2H), 2.2-2.45(9H), 2.5(1H), 2.6(1H), 2.9-3.2(4H), 3.6(2H), 3.8(1H), 4.0(1 H), 6.8(1H), 7.05(1H), 7.2-7.4(4H), 7.65(1 H), 7.7(1 H), 8.0(2H) und 8.05(1 H) ppm.

### Beispiel 106

### 4-(1-Methyl-piperidin-4-yl)-piperazin-1-karbonsäure-[1-(2-chlor-benzolsulfonyl)-5-cyano-3-(2-ethoxy-phenyl)-2-oxo-2,3-dihydro-1H-indol-3-yl]-ester

¹H-NMR (CDCl₃): δ = 1.2(3H), 1.6(2H), 1.75(2H), 1.9(2H), 2.2-2.3(4H), 2.35(2H), 2.5(1H), 2.6(1H), 2.8(2H), 3.0(1H), 3.15(1H), 3.6(2H), 3.8(1H), 4.05(1H), 6.8(1H), 7.0(1H), 7.25-7.35(2H), 7.4(1H), 7.5(2H), 7.65(2H), 8.15(1 H) und 8.4(1 H) ppm.

### Beispiel 107

### 4-(1-Methyl-piperidin-4-yl)-piperazin-1-karbonsäure-[5-cyano-1-(2,5-dimethoxy-benzolsulfonyl)-3-(2-ethoxy-phenyl)-2-oxo-2,3-dihydro-1H-indol-3-yl]-ester

¹H-NMR (CDCl₃): δ = 1.25(3H), 1.6(2H), 1.75(2H), 1.9(2H), 2.2-2.3(4H), 2.35(2H), 2.55(2H), 2.9(2H), 3.1(2H), 3.5-3.7(5H), 3.8(4H), 4.05(1H), 6.8(1H), 6.85(1H), 7.0(1H), 7.1(1H), 7.25-7.35(2H), 7.6-7.75(3H) und 8.15(1 H) ppm.

### Beispiel 108

### 4-(1-Methy)-piperidin-4-yl)-piperazin-1-karbonsäure-[5-cyano-1-(2-cyano-benzolsulfonyl)-3-(2-ethoxy-phenyl)-2-oxo-2,3-dihydro-1H-indol-3-yl]-ester

¹H-NMR (CDCl₃): δ = 1.25(3H), 1.6(2H), 1.7(2H), 1.95(2H), 2.2-2.4(6H), 2.45(1H), 2.6(1 H), 2.7-3.1(4H), 3.5(1H), 3.6(1 H), 3.8(1H), 4.05(1 H), 6.8(1H), 7.05(1H), 7.2(1H), 7.35(1H), 7.6-7.8(4H), 7.85(1H), 8.3(1H) und 8.4(1H) ppm.

### Beispiel 109

### 4-(1-Methyl-piperidin-4-yl)-piperazin-1-karbonsäure-[5-cyano-1-(2,4-difluoro-benzolsulfonyl)-3-(2-ethoxy-phenyl)-2-oxo-2,3-dihydro-1H-indol-3-yl]-ester

¹H-NMR (CDCl₃): δ = 1.25(3H), 1.6(2H), 1.7(2H), 1.95(2H), 2.2-2.45(6H), 2.55(2H), 2.8-3.2(4H), 3.6(2H), 3.8(1 H), 4.05 (1H), 6.8(1H), 6.95-7.1 (2H), 7.25(1 H), 7.3(1 H), 7.7(2H), 8.1(1H) und 8.15(1 H) ppm.

### Beispiel 110

### 4-(1-Methyl-piperidin-4-yl)-piperazin-1-karbonsäure-[5-cyano-1-(4-fluoro-benzolsulfonyl)-3-(2-ethoxy-phenyl)-2-oxo-2,3-dihydro-1H-indol-3-yl]-ester

¹H-NMR (CDCl₃): δ = 1.25(3H), 1.55(2H), 1.75(2H), 1.95(2H), 2.2-2.3(4H), 2.35(2H), 2.55(2H), 2.9(2H), 3.0(2H), 3.6(2H), 3.8(1 H), 4.0(1 H), 6.8(1H), 7.05(1H), 7.15(2H), 7.25(1H), 7.3(1H), 7.65(1H), 7.7(1H), 8.05(1H) und 8.15(1 H) ppm.

### Beispiel 111

### 4-(1-Methyl-piperidin-4-yl)-piperazin-1-karbonsäure-[5-cyano-3-(2-ethoxyphenyl)-1-(4-isopropyl-benzolsulfonyl)-2-oxo-2,3-dihydro-1H-indol-3-yl]-ester

¹H-NMR (CDCl₃): δ = 1.2-1.3(9H), 1.55(2H), 1.75(2H), 1.95(2H), 2.2-2.4(6H), 2.45(1H), 2.6(1H), 2.8-3.1(5H), 3.6(2H), 3.75(1H), 4.0(1 H), 6.8(1H), 7.05(1H), 7.25(1H), 7.3-7.4(1H). 7.65(1H), 7.7(1H), 8.0(1 H) und 8.1(1H) ppm.

### Beispiel 112

### 4-(1-Methyl-piperidin-4-yl)-piperazin-1-karbonsäure-[5-cyano-3-(2-ethoxyphenyl)-1-(2-fluoro-benzolsulfonyl)-2-oxo-2,3-dihydro-1 H-indol-3-yl]-ester

¹H-NMR (CDCl₃): δ = 1.25(3H), 1.6(2H), 1.75(2H), 1.95(2H), 2.2-2.4(6H), 2.5(1H), 2.6(1 H), 2.85-3.0(3H), 3.1(1H), 3.6(2H), 3.8(1H), 4.05(1H), 6.8(1 H), 7.05(1H), 7.15(1H), 7.2-7.4(3H), 7.6(1 H), 7.7(2H) und 8.15(1 H) ppm.

### Beispiel 113

### 4-(1-Methyl-piperidin-4-yl)-piperazin-1-karbonsäure-[1-(5-chlor-2-methoxy benzolsulfonyl)-5-cyano-3-(2-ethoxy-phenyl)-2-oxo-2,3-:dihydro-1H-indol-3-yl]-ester

¹H-NMR (CDCl₃): δ = 1.25(3H), 1.6(2H), 1.75(2H), 1.95(2H), 2.2-2.45(5H), 2.55(2H), 2.9(2H), 3.05(2H), 3.5-3.7(5H), 3.8(1H), 4.05(1H), 6.8(1H), 6.85(1 H), 7.0(1 H), 7.2-7.4(2H), 7.45(1 H), 7.7(2H) und 8.1 (2H) ppm.

### Beispiel 114

### 4-(1-Methyl-piperidin-4-yl)-piperazin-1-karbonsäure-[5-cyano-3-(2-Athoxy-phenyl)-1-(2-methoxy-5-methyl-benzolsulfonyl)-2-oxo-2,3-dihydro-1H-indol-3-yl]-ester

¹H-NMR (CDCl₃): δ = 1.25(3H), 1.55(2H), 1.7(2H), 1.9(2H), 2.2-2.3(4H), 2.3-2.45(5H), 2.55(2H), 2.9(2H), 3.1(2H), 3.5-3.7(5H), 3.8(1H), 4.05(1H), 6.8(2H), 7.0(1 H), 7.2-7.4(3H), 7.65(2H), 7.9(1 H) und 8.1(1 H) ppm.

### Beispiel 115

### 4-(-Methyl-piperidin-4-yl)-piperazin-1-karbonsäure-[5-cyano-3-(2-ethoxyphenyl)-1-(2-methoxy-4-methyl-benzolsulfonyl)-2-oxo-2,3-dihydro-1H-indol-3-yl]-ester

¹H-NMR (CDCl₃): δ = 1.25(3H), 1.55(2H), 1.7(2H), 1.9(2H), 2.2-2.3(4H), 2.3-2.45(5H), 2.55(2H), 2.9(2H), 3.1(2H), 3.5-3.7(5H), 3.8(1H), 4.05(1H), 6.7(1H), 6.8(1 H), 6.85(1H), 7.0(1 H), 7.3(2H), 7.65(2H), 8.0(1 H) und 8.15(1H) ppm.

### Beispiel 116

### 4-(1-Methyl-piperidin-4-yl)-piperazin-1-karbonsäure-[1-benzolsulfonyl-5-cyano-3-(2-ethoxy-phenyl)-2-oxo-2,3-dihydro-1H-indol-3-yl]amid-dihydrochlorid

¹H-NMR (D₂O): δ = 0.95(3H), 1.85(2H), 2.3(2H), 2.8(3H), 3.05(2H), 3.1-3.55(9H), 3.6(2H), 3.7(1H), 3.8(1 H), 6.85(1H), 6.95(1H), 7.25(1H), 7.45(1H), 7.5(3H), 7.6-7.7(2H) und 7.8-8.0(3H) ppm.

### Beispiel 117

### 4-(1-Methyl-piperidin-4-yl)-piperazin-1-karbonsäure-[5-cyano-3-(2-ethoxyphenyl)-1-(4-methoxy-benzolsulfonyl)-2-oxo-2,3-dihydro-1H-indol-3-yl]-amid

¹H-NMR (CDCl₃): δ = 1.4-1.8(7H), 1.95(2H), 2.25(4H), 2.45(4H), 2.9(2H), 3.1-3.3(4H), 3.85(3H), 4.1(1H), 4.2(1H), 6.5(1H), 6.85-7.0(4H), 7.1(1H), 7.3(1 H), 7.5(1 H), 7.6(1 H) und 7.95-8.1(3H) ppm.

### Beispiel 118

### 4-(1-Methyl-piperidin-4-yl)-piperazin-1-karbonsäure-[5-cyano-3-(2-ethoxyphenyl)-1-(2-methoxy-benzolsulfonyl)-2-oxo-2,3-dihydro-1 H-indol-3-yl]-amid-dihydrochlorid

¹H-NMR (D₂O): δ = 0.75(3H), 1.95(2H), 2.4(2H), 2.85(3H), 3.05(2H), 3.2-3.4(4H), 3.4(3H), 3.5-3.9(9H), 6.95(2H), 7.1-7.2(2H), 7.3-7.4(2H), 7.6(1H), 7.7-7.8(2H), 7.8(1 H) und 8.1(3H) ppm.

### Beispiel 119

### 4-(1-Methyl-piperidin-4-yl)-piperazin-1-karbonsäure-[5-cyano-3-(2-ethoxyphenyl)-1-(2,4-dimethoxy-benzolsulfonyl)-2-oxo-2,3-dihydro-1H-indol-3-yl]-amid

¹H-NMR (CDCl₃): δ = 1.5-1.9(7H), 2.05(2H), 2.25-2.35(4H), 2.45(4H), 3.0(2H). 3.15-3.3(4H), 3.5(3H), 3.85(3H), 4.15(1H), 4.25(1H), 6.4(1H), 6.55(1H), 6.7(1H), 6.85(1 H), 6.9(2H), 7.25(1 H), 7.6(2H) und 8.2(2H) ppm.

### Beispiel 120

### 4-(1-Methyl-piperidin-4-yl)-piperazin-1-karbonsäure-[1-(4-acetylamino-benzolsulfonyl)-5-cyano-3-(2-ethoxy-phenyl)-2-oxo-2,3-dihydro-1H-indol-3-yl]-ester

¹H-NMR (CDCl₃): δ = 1.25(3H), 1.6(2H), 2.0(2H), 2.15-2.25(4H), 2.25-2.4(5H), 2.5(2H), 2.9-3.1 (4H), 3.5(1H), 3.6(1H), 3.8(1H), 4.0(1H), 6.8(1H), 7.0(1 H), 7.25(1 H), 7.3(1 H), 7.6-7.75(4H), 7.8 (1H) und 8.052(3H) ppm.

### Beispiel 121

### 4-(1-Methyl-piperidin-4-yl)-piperazin-1-karbonsäure-[1-(2,4-dimethoxy-benzolsulfonyl)-3-(2-ethoxy-phenyl)-5-fluoro-2-oxo-2,3-dihydro-1H-indol-3-yl]-amid

¹H-NMR (CDCl₃): δ = 1.35(2H), 1.5-1.9(5H), 2.25-2.35(4H), 2.3(4H), 2.4-2.8(10H), 3.5(3H), 3.75-3.9(5H), 4.1-4.3(2H), 6.4(1 H), 6.55(1H), 6.7-7.0(3H), 7.05(1H), 7.25(1H), 7.9(2H) und 8.1(1H) ppm.

### Beispiel 122

### 4-(1-Methyl-piperidin-4-yl)-piperazin-1-karbonsäure-[1-(2-methoxy-benzolsulfonyl)-3-(2-ethoxy-phenyl)-5-fluoro-2-oxo-2,3-dihydro-1 H-indol-3-yl]-amid

¹H-NMR (CDCl₃): δ = 1.2-1.4(2H), 1.55(3H), 1.6-1.9(2H), 2.25-2.4(4H), 2.4-2.8(10H), 3.55(3H), 3.8(2H), 4.1-4.3(2H), 6.8-7.1(8H), 7.25(1H), 7.5(1H), 7.9(1 H) und 8.15(1H) ppm.

### Beispiel 123

### 4-(1-Methyl-piperidin-4-yl)-piperazin-1-karbonsäure-[1-(4-methoxy-benzolsulfonyl)-3-(2-ethoxy-phenyl)-5-fluoro-2-oxo-2,3-dihydro-1H-indol-3-yl]-amid-dihydrochlorid

¹H-NMR (D₆-DMSO): δ = 1.15(3H), 1.5(2H), 2.05(2H), 2.6(2H), 2.8(3H), 3.2-3.8(11 H), 3.85(4H), 3.95(1 H), 6.95(2H), 7.1(2H), 7.15(2H), 7.3(1 H), 7.4(1 H), 7.7(1 H), 7.75(1 H), und 7.95(2H) ppm.

### Beispiel 124

### 4-(1-Methyl-piperidin-4-yl)-piperazin-1-karbonsäure-[3-(2-ethoxy-phenyl)-5-isopropyl-1-(2-methoxy-benzolsulfonyl)-2-oxo-2,3-dihydro-1H-indol-3-yl]-ester

¹H-NMR (CDCl₃): δ = 1.1-1.3(9H), 1.55(2H), 1.7(2H), 1.9(2H), 2.15-2.4(6H), 2.5(2H), 2.8(1H), 2.9(1H), 3.1(1H), 3.6(2H), 3.8(1H), 4.05(1H), 6.75(1H), 6.8(1H), 6.9(1H), 6.95-7.05(2H), 7.15(1H), 7.25(1H), 7.5(1H), 7.7(1H), 7.85(1H) und 8.15(1 H) ppm.

### Beispiel 125

### 4-(1-Methyl-piperazin-4-yl)-piperidin-1-karbonsäure-[5-cyano-3-(2-ethoxyphenyl)-1-(2-methoxy-benzolsulfonyl)-2-oxo-2,3-dihydro-1 H-indol.-3-yij-amid

¹H-NMR (CDCl₃): δ = 1.25-1.45(2H), 1.45-1.7(5H), 1.75(2H), 2.25-2.75(12H), 3.55(3H), 3.75(2H), 4.15(1H), 4.25(1H), 6.65(1H), 6.85(1H), 6.9(1 H), 6.95(1 H); 7.05(1 H), 7.25(1 H), 7.55(1 H), 7.6(2H), 8.1 (1 H) und 8.15(1 H) ppm.

### Beispiel 126

### 4-(1-Methyl-piperidin-4-yl)-piperazin-1-karbonsäure-[3-(2-ethoxy-phenyl)-5-fluoro-1-(2-methoxy-benzolsulfonyl)-2-oxo-2,3-dihydro-1H-indol-3-yl]-amid

¹H-NMR (CDCl₃): δ = 1.4-1.6(5H), 1.7(2H), 1.9(2H), 2.2-2.3(4H), 2.45(4H), 3.55(3H), 4.1-4.3(2H), 6.75-7.15(7H), 7.25(1H), 7.5(1H), 7.9(1H) und 8.15(1 H) ppm.

### Beispiel 127

### 4-(1-Methyl-piperidin-4-yl)-piperazin-1-karbonsäure-[3-(2-ethoxy-phenyl)-5-isopropyl-1-(4-methoxy-benzolsulfonyl)-2-oxo-2,3-dihydro-1H-indol-3-yl]-ester

¹H-NMR (CDCl₃): δ = 1.15(6H), 1.2(3H), 1.55(2H), 1.75(2H), 2.0(2H), 2.2-2.45(6H), 2.45(1H), 2.55(1H), 2.75(1H), 2.95(2H), 3.05(2H), 3.6(2H), 3.75(1H), 3.85(3H), 4.0(1H), 6.75(2H), 6.95(2H), 7.0(1H), 7.15(1H), 7.25(1 H), 7.7(1 H), 7.8(1H) und 8.1(2H) ppm.

### Beispiel 128

### 4-(1-Methyl-piperazin-4-yl)-piperidin-1-karbonsäure-[5-cyano-3-(2-ethoxyphenyl)-1-(2,4-dimethoxy-benzolsulfonyl)-2-oxo-2,3-dihydro-1H-indol-3-yl]-amid

¹H-NMR (CDCl₃): δ = 1.2(3H), 1.35(2H), 1.55(2H), 1.75(2H), 2.2-2.8(12H), 3.5(3H), 3.75(2H), 3.85(3H), 4.15(1H), 4.2(1H), 6.4(1H), 6.55(1H), 6.7(1H), 6.85(1 H), 6.9(1 H), 6.95(1 H), 7.25(1 H), 7.6(2H) und 8.1(2H) ppm.

### Beispiel 129

### (-)-4-(1-Methyl-piperidin-4-yl)-piperazin-1-karbonsäure-[5-cyano-3-(2-ethoxyphenyl)-1-(2-methoxy-benzolsulfonyl)-2-oxo-2,3-dihydro-1H-indol-3-yl]-ester

¹H-NMR (CDCl₃): δ = 1.25(3H), 1.55(2H), 1.75(2H), 1.9(2H), 2.2-2.45(6H), 2.55(2H), 2.9(2H), 3.1(2H), 3.55-3.7(5H), 3.8(1H), 4.1(1H), 6.8(1H), 6.95(1H), 7.0-7.1(3H), 7.3(1H), 7.6(1H), 7.7(2H) und 8.15(2H) ppm.

### Beispiel 130

### (+)-4-(1-Methyl-piperidin-4-yl)-piperazin-1-karbonsäure-[5-cyano-3-(2-ethoxy-phenyl)-1-(2-methoxy-benzolsulfonyl)-2-oxo-2,3-dihydro-1 H-indol-3-yl]-ester

¹H-NMR (CDCl₃): δ = 1.25(3H), 1.55(2H), 1.75(2H), 1.9(2H), 2.2-2.45(6H), 2.55(2H), 2.9(2H), 3.1(2H), 3.55-3.7(5H), 3.8(1H), 4.1(1H), 6.8(1H), 6.95(1 H), 7.0-7.1(3H), 7.3(1 H), 7.6(1 H), 7.7(2H) und 8.15(2H) ppm.

### Beispiel 131

### (-)-4-(1-Methyl-piperidin-4-yl)-piperazin-1-karbonsäure-[5-cyano-3-(2-ethoxyphenyl)-1-(2,4-dimethoxy-benzolsulfonyl)-2-oxo-2,3-dihydro-1H-indol-3-yl]-ester

¹H-NMR (CDCl₃): δ = 1.25(3H), 1.55(2H), 1.75(2H), 1.9(2H), 2.2-2.45(6H), 2.55(2H), 2.95(2H), 3.1(2H), 3.5-3.7(5H), 3.8(1H), 3.85(3H), 4.05(1H), 6.4(1 H), 6.55(1 H), 6.8(1 H), 7.0(1 H), 7.3(2H), 7.65(2H), 8.05(1 H) und 8.1(1 H) ppm.

### Beispiel 132

### (+)-4-(1-Methyl-piperidin-4-yl)-piperazin-1-karbonsäure-[5-cyano-3-(2-ethoxy-phenyl)-1-(2,4-dimethoxy-benzolsulfonyl)2-oxo-2,3-dihydro-1H-indol-3-yl]-ester

¹H-NMR (CDCl₃): δ = 1.25(3H), 1.55(2H), 1.75(2H), 1.9(2H), 2.2-2.45(6H), 2.55(2H), 2.95(2H), 3.1(2H), 3.5-3.7(5H), 3.8(1H), 3.85(3H), 4.05(1H), 6.4(1H), 6.55(1H), 6.8(1H), 7.0(1H), 7.3(2H), 7.65(2H), 8.05(1H) und 8.1(1H)ppm.

### Beispiel 133

### 4-(4-Methyl-piperazin-1-yl)-piperidin-1-karbonsäure-[1-benzolsulfonyl-5-cyano-3-(2-ethoxy-phenyl)-2-oxo-2,3-dihydro-1H-indol-3-yl]-amid

¹H-NMR (CDCl₃): δ = 1.35(2H), 1.5(3H), 1.85(2H), 2.25-2.8(14H), 3.7(2H), 4.1(1H), 4.2(1H), 6.5(1 H), 6.9(2H), 7.05(1H), 7.3(1H), 7.45-7.7(5H), 8.05(1 H) und 8.15(1 H)ppm.

### Beispiel 134

### 4-(1-Methyl-piperidin-4-yl)-piperazin-1-karbonsäure-[1-benzolsulfonyl-3-(2-ethoxy-phenyl)-5-isopropyl-2-oxo-2,3-dihydro-1H-indol-3-yl]-ester

¹H-NMR (CDCl₃): δ = 1.15(6H), 1.2(3H), 1.55(2H), 1.7(2H), 1.95(2H), 2.2-2.4(5H), 2.45(1H), 2.55(1H), 2.75(1H), 2.9(2H), 3.05(2H), 3.6(2H), 3.75(1H), 3.95(1H), 6.8(2H), 7.0(1 H), 7.2(1H), 7.3(2H), 7.5(2H), 7.6(1 H), 7.75(1H), 7.8(1H) und 8.15(2H)ppm.

### Beispiel 135

### 4-(1-Methyl-piperidin-4-yl)-piperazin-1-karbonsäure-[4-chlor-3-(2-methoxyphenyl)-1-(2,4-dimethoxy-benzolsulfonyl)-2-oxo-2,3-dihydro-1H-indol-3-yl]-ester

¹H NMR (CDCl₃) δ 1.43-1.94(7H), 2.24-2.66(7H), 2.86-3.30(3H), 3.60(3H), 3.68(3H), 3.84(1 H), 6.40(1 H), 6.52(1 H), 6.78(1 H), 6.93(1 H), 7.00(1 H), 7.33-7.20(m), 7.80(1 H), 7.91 (1 H), 8.06(1 H).

### Beispiel 136

### 4-(1-Methyl-piperidin-4-yl)-piperazin-1-karbonsäure-[4-chlor-3-(2-methoxyphenyl)-1-(4-methoxy-benzolsulfonyl)-2-oxo-2,3-dihydro-1H-indol-3-yl]-ester

¹H NMR (CDCl₃) δ 1.43-2.01(5H), 2.26-2.56(6H), 2.63(1 H), 3.10(2H), 3.25(1 H), 3.50-3.65(4H), 3.73(1 H), 3.84(3H), 6.76(1 H), 6.91-7.07(4H), 7.22-7.37(m), 7.81 (2H), 8.08(2H).

### Beispiel 137

### 4-(1-Methyl-piperazin-4-yl)-piperidin-1-karbonsäure-[3-(2-ethoxy-phenyl)-5-methoxy-1-(2-Methoxy-benzolsulfonyl)-2-oxo-2,3-dihydro-1H-indol-3-yl]-amid-dihydrochlorid

¹H-NMR (D₆-DMSO): δ = 1.3(3H), 1.45(2H), 2.05(2H), 2.6(2H), 2.85(3H), 3.2-3.9(15H), 3.9-4.2(4H), 6.8(2H), 7.0(3H), 7.15(1H), 7.2(1 H), 7.3(1H), 7.6(1H), 7.7(2H) und 8.0(1H) ppm.

### Beispiel 138

### 4-(1-Methyl-piperazin-4-yl)-piperidin-1-karbonsäure-[3-(2-ethoxy-phenyl)-5-methoxy-1-(2,4-dimethoxy-benzolsulfonyl)-2-oxo-2,3-dihydro-1H-indol-3-yl]-amid-dihydrochlorid

¹H-NMR (D₆-DMSO): δ = 1.3(3H), 1.3-1.6(2H), 2.05(2H), 2.65(2H), 2.8(3H), 3.2-3.8(15H), 3.85(3H), 3.9-4.2(4H), 6.7(2H), 6.8(2H), 6.95(1H), 7.0(2H), 7.3(1 H), 7.6(1 H), 7.7(2H) und 7.9(1 H) ppm.

### Beispiel 139

### 4-(1-Methyl-piperazin-4-yl)-piperidin-1-karbonsäure-[3-(2-ethoxy-phenyl)-5-methoxy-1-(4-methoxy-benzolsulfonyl)-2-oxo-2,3-dihydro-1H-indol-3-yl]-amid-dihydrochlorid

¹H-NMR (D₆-DMSO): δ = 1.2(3H), 1.35-1.6(2H), 1.9-2.1(2H), 2.6(2H), 2.8(3H), 3.2-3.8(12H), 3.8-4.1(7H), 6.8-6.95(3H), 6.95(1H), 7.15(2H), 7.2-7.3(2H), 7.6(1 H), 7.7(1 H) und 7.9(2H) ppm.

### Beispiel 140

### 4-(4-Methyl-piperazin-1-yl)-piperidin-1-karbonsäure-[1-benzolsulfonyl-3-(2-ethoxy-phenyl)-5-methoxy-2-oxo-2,3-dihydro-1H-indol-3-yl]-amid-dihydrochlorid

¹H-NMR (D₆-DMSO): δ = 1.2(3H), 1.3-1.6(2H), 1.9-2.1(2H), 2.6(2H), 2.8(3H), 3.2-3.8(14H), 3.85(1H), 3.95(1H), 6.8(2H), 6.9(1H), 6.95(1H), 7.3(2H), 7.6-7.8(5H) und 8.0(2H) ppm.

### Beispiel 141

### 4-(1-Methyl-piperidin-4-yl)-piperazin-1-karbonsäure-[6-chlor-3-(2-methoxyphenyl)-1-(4-methoxy-benzolsulfonyl)-2-oxo-2,3-dihydro-1H-indol-3-yl]-ester

### Beispiel 142

### 4-(1-Methyl-piperidin-4-yl)-piperazin-1-karbonsäure-[1-(2,4-dimethoxy-benzolsulfonyl)-3-(2-ethoxy-phenyl)-5-isopropyl-2-oxo-2,3-dihydro-1 H-indol-3-yl]-ester

¹H-NMR (D₆-DMSO): δ = 0.95(3H), 1.1 (6H), 1.4(2H), 1.65(2H), 2.0(2H), 2.15-2.35(4H), 2.4(2H), 2.55(2H), 2.85(2H), 2.95(1H), 3.15(1H), 3.4(1H), 3.45(3H), 3.65(1 H), 3.7(1 H), 3.85(3H), 3.9(1 H), 6.6(1 H), 6.65(1 H), 6.85(1 H), 6.95(1 H), 7.05(1 H), 7.25(1 H), 7.3(1 H), 7.65(1 H), 7.7(1 H) und 7.85(1 H) ppm.

### Beispiel 143

### 4-Piperidin-4-yl-piperazin-1-karbonsäure-[5-cyano-1-(2,4-dimethoxy-benzolsulfonyl)-3-(2-ethoxy-phenyl)-2-oxo-2,3-dihydro-1H-indol-3-yl]-ester-dihydrochlorid

¹H-NMR (D₆-DMSO): δ = 1.0(3H), 1.9(2H), 2.25(2H), 2.8-3.7(15H), 3.75(1H), 3.85(3H), 3.95(1H), 4.3(1H), 6.6-6.7(2H), 7.0(1 H), 7.1(2H), 7.35(1H), 7.65(1H), 7.8(1H), 7.85(1H), 7.95(2H), 9.0(NH⁺) und 11.8(NH⁺) ppm.

### Beispiel 144

### 4-(4-Methyl-piperazin-1-yl)-piperidin-1-karbonsäure-[5-cyano-3-(2-ethoxy phenyl)-1-(4-methoxy-benzolsulfonyl)-2-oxo-2,3-dihydro-1H-indol-3-yl]-amid

¹H-NMR (CDCl₃): δ = 1.35(2H), 1.5(3H), 1.55(2H), 1.75(2H), 2.3-2.5(4H), 2.5-2.8(8H), 3.7(2H), 3.85(3H), 4.1(1H), 4.2(1H), 6.5(1H), 6.8-7.0(4H), 7.05(1H), 7.25(1H), 7.45(1 H), 7.6(1H) und 8.0-8.1(3H) ppm.

### Beispiel 145

### 4-(4-Ethyl-piperidin-1-yl)-piperazin-1-karbonsäure-[5-cyano-3-(2-ethoxyphenyl)-1-(2,4-dimethoxy-benzolsulfonyl)-2-oxo-2,3-dihydro-1H-indol-3-yl]-ester

¹H-NMR (CDCl₃): δ = 1.1(3H), 1.25(3H), 1.55(2H), 1.55(2H), 1.9(2H), 2.3(1H), 2.4(4H), 2.55(2H), 2.9-3.2(4H), 3.55(3H), 3.6(2H), 3.8(1H), 3.85(3H), 4.05(1H), 6.4(1H), 6.55(1H), 6.8(1H), 7.00(1 H), 7.2-7.4(2H), 7.65(2H), 8.05(1H), und 8.1(1H) ppm.

### Beispiel 146

### 4-(4-Propyl-piperidin-1-yl)-piperazin-1-karbonsäure-[5-cyano-3-(2-ethoxyphenyl)-1-(2,4-dimethoxy-benzolsulfonyl)-2-oxo-2,3-dihydro-1H-indol-3-yl]-ester

¹H-NMR (CDCl₃): δ = 0.9(3H), 1.25(3H), 1.4-1.6(4H), 1.7(2H), 1.9(2H), 2.25(3H), 2.35(2H), 2.55(2H), 2.95(2H), 3.15(2H), 3.55(3H), 3.6(2H), 3.8(1H), 3.85(3H), 4.05(1H), 6.4(1H), 6.55(1H), 6.8(1H), 7.00(1H), 7.2-7.4(2H), 7.6-7.75(2H), 8.05(1 H), und 8.15(1 H) ppm.

### Beispiel 147

### 4-(4-Isopropyl-piperidin-1-yl)-piperazin-1-karbonsäure-[5-cyano-3-(2-ethoxyphenyl)-1-(2,4-dimethoxy-benzolsulfonyl)-2-oxo-2,3-dihydro-1H-indol-3-yl]-ester

¹H-NMR (CDCl₃): δ = 1.25(3H), 1.45(6H), 1.6(2H), 1.9(2H), 2.3-2.8(8H). 3.1-3.3(2H), 3.4-3.8(7H), 3.8(1H), 3.85(3H), 4.05(1H), 6.4(1H), 6.55(1H), 6.8(1 H), 7.0(1 H), 7.2-7.4(2H), 7.6-7.75(2H), 8.05(1 H), und 8.15(1H) ppm.

### Beispiel 148

### 4-(4-Methyl-piperidin-1-yl)-piperazin-1-karbonsäure-[4-methyl-3-(2-ethoxyphenyl)-1-(2,4-dimethoxy-benzolsulfonyl)-2-oxo-2,3-dihydro-1H-indol-3-yl]-ester

### Beispiel 149

### 4-(4-Methyl-piperidin-1-yl)-piperazin-1-karbonsäure-[5-cyano-3-(2-ethoxyphenyl)-1-(3,4-dibrom-benzolsulfonyl)-2-oxo-2,3-dihydro-1H-indol-3-yl]-ester

¹H-NMR (CDCl₃): δ = 1.25(3H), 1.55(2H), 1.75(2H), 1.95(2H), 2.2-2.6(8H), 2.9(2H), 3.0(2H), 3.5 (1H), 3.65(1 H), 3.8(1 H), 4,0 (1H), 6.8(1H), 7.05(1H), 7.25(1 H), 7.35(1 H), 7.6-7.8(3H), 7.85(1 H), 8.0(1 H) und 8.3(1 H) ppm.

### Beispiel 150

### 4-(4-Methyl-piperidin-1-yl)-piperazin-1-karbonsäure-[4-methoxy-3-(2-methoxy-phenyl)-1-(2-methoxy-benzolsulfonyl)-2-oxo-2,3-dihydro-1H-indol-3-yl]-ester

### Beispiel 151

### 4-(4-Methyl-piperidin-1-yl)-piperazin-1-karbonsäure-[4-methoxy-3-(2-methoxy-phenyl)-1-(2,4-dimethoxy-benzolsulfonyl)-2-oxo-2,3-dihydro-1H-indol-3-yl]-ester

### Beispiel 152

### 4-(4-Methyl-piperidin-1-yl)-piperazin-1-karbonsäure-[5-methoxy-3-(2-ethoxyphenyl)-1-(2,4-dimethoxy-benzolsulfonyl)-2-oxo-2,3-dihydro-1H-indol-3-yl]-ester

¹H-NMR (CDCl₃): δ = 1.25(3H), 1.55(2H), 1.7(2H), 1.9(2H), 2.2-2.3(4H), 2.4(2H), 2.5(2H), 2.9(2H), 3.1(2H), 3.6(4H), 3.7(3H), 3.75(1H), 3.8(3H), 4.05(1H), 6.4(1H), 6.5-6.6(2H), 6.75(1H), 6.8(1H), 6.95(1H), 7.25(1H), 7.65(1 H), 7.85(1 H), und 8.05(1H) ppm.

### Beispiel 153

### 4-Piperazin-1-yl-piperidin-1-karbonsäure-[5-cyano-1-(2,4-dimethoxy-benzolsulfonyl)-3-(2-ethoxy-phenyl)-2-oxo-2,3-dihydro-1H-indol-3-yl]-amid-dihydrochiorid

¹H-NMR (D₆-DMSO): δ = 1.25(3H), 1.45(2H), 2.0(2H), 2.65(2H), 3.2-3.8(13H), 3.8-4.1 (6H), 6.6-6.75(2H), 6.9(1H), 7.0(1H), 7.25-7.4(2H), 7.7(1 H), 7.8(2H), 7.9(2H), 9.4(NH⁺), 9.6(NH⁺) und 11.9(NH⁺) ppm.

### Beispiel 154

### 4-(4-Ethyl-piperazin-1-yl)-piperidin-1-karbonsäure-[5-cyano-1-(2,4-dimethoxy-benzolsulfonyl)-3-(2-ethoxy-phenyl)-2-oxo-2,3-dihydro-1H-indol-3-yl]-amid

¹H-NMR (CDCl₃): δ = 1.2-1.4(5H), 1.5(3H), 1.75(2H), 2.4(1H), 2.5-2.8(12H), 3.5(3H), 3.75(2H), 3.85(3H), 4.15(1H), 4.2(1H), 6.4(1H), 6.55 (1H), 6.7(1H), 6.85(1H), 6.9(1H), 6.95(2H), 7.25(1H), 7.6(2H) und 8.1(2H) ppm.

### Beispiel 155

### 4-(4-Propyl-piperazin-1-yl)-piperidin-1-karbonsäure-[5-cyano-1-(2,4-dimethoxy-benzolsulfonyl)-3-(2-ethoxy-phenyl)-2-oxo-2,3-dihydro-1H-indol-3-yl]-amid

¹H-NMR (D₆-DMSO): δ = 0.9(3H), 1.15(3H), 1.45(2H), 1.7(2H), 2.0(2H), 2.65(2H), 3.05(2H), 3.25-3.8(12H), 3.8-4.1(7H), 6.7(2H), 6.9(1H), 6.95(1H), 7.3(2H), 7.7(1 H), 7.8(2H) und 7.9(2H) ppm.

### Beispiel 156

### 4-(4-Propyl-piperazin-1-yl-piperidin-1-karbonsäure-[5-cyano-1-benzolsulfonyl-3-(2-ethoxy-phenyl)-2-oxo-2,3-dihydro-1 H-indol-3-yl]-amid

¹H-NMR (CDCl₃): δ = 0.9(3H), 1.35(2H), 1.45-1.55(5H), 1.75(2H), 2.3(3H), 2.4-2.8(10H), 3.7(2H), 4.1(1H), 4.2(1H), 6.45(1H), 6.9(2H), 7.1(1H), 7.3(1H), 7.5(3H), 7.55-7.7(2H), 8.0(1H) und 8.1(2H) ppm.

### Beispiel 157

### 4-(4-Propyl-piperazin-1-yl)-piperidin-1-karbonsäure-[5-cyano-1-(2-methoxy-benzolsulfonyl)-3-(2-ethoxy-phenyl)-2-oxo-2,3-dihydro-1H-indol-3-yl]-amid

¹H-NMR (CDCl₃): δ = 0.9(3H), 1.35(2H), 1.45-1.6(5H), 1.75(2H), 2.25-2.4(3H), 2.4-2.65(8H), 2.65(2H), 3.5(3H), 3.75(2H), 4.15(1H), 4.2(1H), 6.65(1H), 6.85(1H), 6.9(1H), 6.95(1H), 7.05(1H), 7.25(1H), 7.55(1H), 7.6(2H), 8.1(1H) und 8.15(1H) ppm.

### Beispiel 158

### 4-(4-Isopropyl-piperazin-1-yl)-piperidin-1-karbonsäure-[5-cyano-1-(2,4-dimethoxy-benzolsulfonyl)-3-(2-ethoxy-phenyl)-2-oxo-2,3-dihydro-1H-indol-3-yl]-amid dihydrochlorid

¹H-NMR (D₆-DMSO): δ = 1.1(3H), 1.25(6H), 1.45(2H), 2.0(2H), 2.65(2H), 3.3-3.8(13H), 3.8-4.1(7H), 6.7(2H), 6.95(1H), 7.0(1H), 7.3(2H), 7.7(1H), 7.8(2H) und 7.9(2H) ppm.

### Beispiel 159

### 4-(4-Propyl-piperazin-1-yl)-pipeddin-1-karbonsäure-[5-cyano-1-(2,4-difluoro-benzolsulfonyl)-3-(2-ethoxy-phenyl)-oxo-2,3-dihydro-1H-indol-3-yl]-ester

¹H-NMR (CDCl₃): δ = 0.9(3H), 1.1(1H), 1.25(3H), 1.35(1H), 1.4-1.65(4H), 1.7(1H), 1.85(1H), 2.25-2.75(10H), 2.95(1H), 3.55(1H), 3.8(1H), 4.05(1H), 4.2(1H), 6.8(1H), 6.9(1H), 7.0(2H), 7.25(1H), 7.3(1H), 7.7(2H) und 8.1-8.25(2H) ppm.

### Beispiel 160

### 4-(4-Propyl-piperazin-1-yl)-piperidin-1-karbonsäure-[5-cyano-1-(4-methoxy-benzolsulfonyl)-3-(2-ethoxy-phenyl)-2-oxo-2,3-dihydro-1H-indol-3-yl]-ester

¹H-NMR (CDCl₃): δ = 0.9(3H), 1.1-1.35(4H), 1.4-1.75(6H), 1.85(1H), 2.25-2.75(10H), 2.95(1H), 3.55(1H), 3.8(1 H), 3.85(3H), 4.0(1 H), 4.2(1 H), 6.8(1 H), 6.95(2H), 7.05(1H), 7.25(1H), 7.3(1H), 7.65(1H), 7.7(1H) und 8.05(3H) ppm.

### Beispiel 161

### 4-(4-Propyl-piperazin-1-yl)-piperidin-1-karbonsäure-[5-cyano-1-(2-fluoro-benzolsulfonyl)-3-(2-ethoxy-phenyl)-2-oxo-2,3-dihydro-1H-indol-3-yl]-ester

¹H-NMR (CDCl₃): δ = 0.9(3H), 1.1(1H), 1.3(3H), 1.35-1.75(4H), 1.75-1.95(1H), 2.25-2.75(12H), 2.9(1H), 3.55(1H), 3.8(1H), 4.0(1 H), 4.2(1H), 6.8(1 H), 7.0(1H), 7.15(1H), 7.2-7.4(3H), 7.6(1H), 7.7(2H) und 8.15(2H) ppm.

### Beispiel 162

### 4-(4-Propyl-piperazin-1-yl)-piperidin-1-karbonsäure-[5-cyano-1-benzolsulfonyl-3-(2-ethoxy-phenyl)2-oxo-2,3-dihydro-1H-indol-3-yl]-ester

¹H-NMR (CDCl₃): δ = 0.9(3H), 1.2(1 H), 1.25(3H), 1.35-1.75(6H), 1.85(1H), 2.25-2.75(10H), 2.95(1H), 3.5(1H), 3.8(1H), 4.0(1H), 4.2(1 H), 6.8(1H), 7.0(1H), 7.25(1H), 7.3(1H), 7.5(2H), 7.65(2H), 7.7(1H) und 8.05-8.2(3H) ppm.

### Beispiel 163

### 4-(4-Propyl-piperazin-1-yl)-piperidin-1-karbonsäure-[5-cyano-1-(4-methoxy-benzolsulfonyl)-3-(2-ethoxy-phenyl)-2-oxo-2,3-dihydro-1H-indol-3-yl]-amid

¹H-NMR (CDCl₃): δ = 0.9(3H), 1.35(2H), 1.45-1.6(5H), 1.75(2H), 2.3(2H), 2.35(1H), 2.4-2.8(10H), 3.7(2H), 3.85(3H), 4.1(1H), 4.2(1H), 6.5(1H), 6.85-7.0(4H), 7.1(1H), 7.3(1H), 7.5(1 H), 7.6(1 H), 8.05(1 H) und 8.1(2H) ppm.

### Beispiel 164

### 4-(4-Propyl-piperazin-1-yl)-piperidin-1-karbonsäure-[5-cyano-1-(2-fluoro-benzolsulfonyl)-3-(2-ethoxy-phenyl)-2-oxo-2,3-dihydro-1H-indol-3-yl]-amid

¹H-NMR(CDCl₃): δ = 0.9(3H), 1.25(1H), 1.35(1H), 1.45-1.65(5H), 1.75(2H), 2.2-2.35(3H), 2.35-2.8(10H), 3.7(2H), 4.1(1H), 4.2(1H), 6.45(1H), 6.9(2H), 7.1(1H), 7.15(1H), 7.3(2H), 7.5(1H), 7.55-7.65(2H), 8.1(1H) und 8.15(1H) ppm.

### Beispiel 165

### 4-(4-Propyl-piperazin-1-yl)-piperidin-1-karbonsäure-[5-cyano-1-(2,4-difluoro-benzolsulfonyl)-3-(2-ethoxy-phenyl)-2-oxo-2,3-dihydro-1H-indol-3-yl]-amid

¹H-NMR (CDCl₃): δ = 0.9(3H), 1.25(1H), 1.35(1H), 1.45-1.65(5H), 1.75(2H), 2.2-2.35(3H), 2.4-2.8(10H), 3.7(2H), 4.1(1H), 4.2(1H), 6.4(1H), 6.8-7.05(4H), 7.15(1H), 7.3(1H), 7.5(1H), 7.6(1H), 8.1(1H) und 8.15(1H) ppm.

### Beispiel 166

### 4-(4-Propyl-piperazin-1-yl)-piperidin-1-karbonsäure-[5-cyano-1-(2-methoxy-benzolsulfonyl)-3-(2-ethoxy-phenyl)-2-oxo-2,3-dihydro-1H-indol-3-yl]-ester

¹H-NMR (CDCl₃): δ = 0.9(3H), 1.1(1H), 1.25(3H), 1.4-1.95(10H), 2.25-2.75(11H), 2.95(1H), 3.55(4H), 3.8(1H), 4.05(1H), 4.2(1H), 6.8(1H), 6.95(1H), 7.05(2H), 7.25(1H), 7.3(1H), 7.55(1H), 7.65(2H) und 8.1(2H) ppm.

### Beispiel 167

### 4-(4-Methyl-piperazin-1-yl)-piperidin-1-karbonsäure-[5-cyano-1-benzolsulfonyl-3-(2-ethoxy-phenyl)-2-oxo-2,3-dihydro-1H-indol-3-yl]-ester dihydrochlorid

¹H-NMR (D₆-DMSO): δ = 0.95(3H), 1.35(1H), 1.6(1H), 1.95(1H), 2.1(2H), 2.6(2H), 2.8(3H), 3.0(1H), 3.2-3.9(12H), 4.25(1H), 6.95(9H), 7.1(1H), 7.4(1H), 7.6-7.75(3H), 7.8(2H), 7.9(2H), 8.0(1H) und 8.05(1H) ppm.

### Beispiel 168

### 4-(4-Methyl-piperazin-1-yl)-piperidin-1-karbonsäure-[5-cyano-1-(2,4-difluoro-benzolsulfonyl)-3-(2-ethoxy-phenyl)-2-oxo-2,3-dihydro-1 H-indol-3-yl]-ester-dihydrochiorid

¹H-NMR (D₆-DMSO): δ = 1.2(3H), 1.35-1.7(2H), 1.85-2.2(2H), 2.6(1H), 2.8(3H), 3.0(1H), 3.2-3.8(11H), 3.95(1H), 4.25(1H), 7.0(1H), 7.1(1H), 7.3-7.5(2H), 7.5-7.7(2H), 7.8(1 H), 7.95(2H) und 8.1(1H) ppm.

### Beispiel 169

### 4-(4-Methyl-piperazin-1-yl)-piperidin-1-karbonsäure-[5-cyano-1-(2-fluoro-benzolsulfonyl)-3-(2-ethoxy-phenyl)-2-oxo-2,3-dihydro-1H-indol-3-yl]-ester

¹H-NMR (CDCl₃): δ = 1.25(3H), 1.3-1.9(5H), 2.25-2.7(12H), 2.9(1H), 3.55(1H), 3.8(1H), 4.05(1H), 4.2(1H), 6.8(1H), 7.05(1H), 7.15(1H), 7.2-7.4(3H), 7.6(1 H), 7.7(2H) und 8.15(2H) ppm.

### Beispiel 170

### 4-(4-Methyl-piperazin-1-yl)-piperidin-1-karbonsäure-[5-cyano-1-(4-methoxy-benzolsuffonyl)-3-(2-ethoxy-phenyl)-2-oxo-2,3-dihydro-1 H-indol-3-yl]-ester

¹H-NMR (CDCl₃): δ = 1.15-1.3(4H), 1.4-1.9(4H), 2.3-2.75(12H), 2.95(1H), 3.55(1 H), 3.8(1 H), 3.85(3H), 3.95(1 H), 4.2(1 H), 6.8(1 H), 6.95(2H), 7.05(1 H), 7.25(1 H), 7.3(1 H), 7.65(1 H), 7.7(1 H) und 8.05(3H) ppm.

### Beispiel 171

### 4-(4-Methyl-piperazin-1-yl)-piperidin-1-karbonsäure-[5-cyano-1-(2-methoxy-benzolsulfonyl)-3-(2-ethoxy-phenyl)-2-oxo-2,3-dihydro-1H-indol-3-yl]-ester

¹H-NMR (CDCl₃): δ = 1.1(1H), 1.25(3H), 1.3-1.9(4H), 2.25-3.2(12H), 2.95(1H), 3.5-3.65(4H), 3.8(1H), 4.05(1H), 4.2(1H), 6.8(1H), 6.9(1H), 7.0-7.1(2H), 7.3(1 H), 7.55(1H), 7.65(2H) und 8.1(2H) ppm.

### Beispiel 172

### 4-(4-Methy)-piperazin-1-yl)-piperidin-1-karbonsäure-[5-cyano-1-(2,4-dimethoxy-benzolsulfonyl)-3-(2-ethoxy-phenyl)-2-oxo-2,3-dihydro-1H-indol-3-yl]-ester-dihydrochlorid

¹H-NMR (CF₃COOD): δ = 0.5(3H), 1.8(2H), 2.0(2H), 2.5(3H), 2.75(2H), 3.05(3H), 3.1-3.6(16H), 6.0(1H), 6.1(1H), 6.2(1H), 6.45(1H), 6.75(1H), 7.2(1 H), 7.35(1 H), 7.4(1 H) und 7.5(1 H) ppm.

### Beispiel 173

### 4-(4-Proparg-3-yl-piperazin-1-yl)-piperidin-1-karbonsäure-[5-cyano-1-(4-methoxy-benzolsulfonyl)-3-(2-ethoxy-phenyl)-2-oxo-2,3-dihydro-1H-indol-3-yl]-ester

¹H-NMR (CDCl₃): δ = 1.3-1.5(2H), 1.5(3H), 1.7-1.85(2H), 2.25(1H), 2.5-2.8(10H), 3.3(2H), 3.5(3H), 3.8(2H), 3.85(3H), 4.1-4.3(2H), 6.4(1H), 6.55(1H), 6.7(1 H), 6.85(1 H), 6.9-7.0(2H), 7.3(1 H), 7.6(2H) und 8.1 (2H) ppm.

### Beispiel 174

### 4-(4-Isopropyl-piperazin-1-yl)-piperidin-1-karbonsäure-[5-cyano-1-benzolsulfonyl-3-(2-ethoxy-phenyl)-2-oxo-2,3-dihydro-1 H-indol-3-yl]-ester

¹H-NMR (CDCl₃): δ = 1.1(6H), 1.2(3H), 1.35-1.75(5H), 1.75-1.95(1H), 2.25-2.8(9H), 2.95(1H), 3.5(2H), 3.8(1H), 4.0(1H), 4.2(1H), 6.8(1H), 7.05(1H), 7.2(1 H), 7.35(1H), 7.5(1H), 7.6-7.7(3H) und 8.05-8.15(3H) ppm.

### Beispiel 175

### 4-(4-Isopropyl-piperazin-1-yl)-piperidin-1-karbonsäure-[5-cyano-1-(4-methoxy-benzolsulfonyl)-3-(2-ethoxy-phenyl)-2-oxo-2,3-dihydro-1H-indol-3-yl]-ester

¹H-NMR (CDCl₃): δ = 1.1(6H), 1.25(3H), 1.35-1.75(5H), 1.75-1.95(1H), 2.35(1H), 2.4-2.8(8H), 2.95(1H), 3.55(1H), 3.75(1H), 3.85(3H), 4.0(1H), 4.2(1H), 6.8(1H), 6.95(2H), 7.05(1H), 7.2(1 H), 7.3(1H), 7.65(1H), 7.7(1H) und 8.05-8.15(3H) ppm.

### Beispiel 176

### 4-(4-Allyl-piperazin-1-yl)-piperidin-1-karbonsäure-[5-cyano-1-(4-methoxy-benzolsulfonyl)-3-(2-ethoxy-phenyl)-2-oxo-2,3-dihydro-1H-indol-3-yl]-ester

¹H-NMR (CDCl₃): δ = 1.3-1.5(2H), 1.5-1.7(5H), 1.7-1.85(2H), 2.3-2.8(9H), 3.05(2H), 3.5(3H), 3.75(2H), 3.85(3H), 4.1-4.3(2H), 5.1-5.3(2H), 5.9(1H), 6.4(1H), 6.55(1H), 6.7(1H), 6.85(1H), 6.9(1H), 6.95(1H), 7.25(1H), 7.6(2H) und 8.1 (2H) ppm.

### Beispiel 177

### 4-(4-Isopropyl-piperazin-1-yl)-piperidin-1-karbonsäure-[5-cyano-1-(2-methoxy-benzolsulfonyl)-3-(2-ethoxy-phenyl)-2-oxo-2,3-dihydro-1H-indol-3-yl]-ester

¹H-NMR (CDCl₃): δ = 1.1(6H), 1.25(3H), 1.35-1.95(6H), 2.35(1H), 2.4-2.8(9H), 2.95(1H), 3.55(3H), 3.8(1H), 4.05(1H), 4.2(1H), 6.8(1H), 6.95(1H), 7.0-7.1 (2H), 7.25(1 H), 7.3(1 H), 7.55(1 H), 7.7(2H) und 8.1(2H) ppm.

### Beispiel 178

### 4-(4-Ethyl-piperazin-1-yl)-piperidin-1-karbonsäure-[5-cyano-1-benzolsulfonyl-3-(2-ethoxy-phenyl)-2-oxo-2,3-dihydro-1 H-indol-3-yl]-amid

¹H-NMR (CDCl₃): δ = 1.1 (3H), 1.35(2H), 1.5(3H), 1.75(2H), 2.25-2.8(13H), 3.8(2H), 4.1(1H), 4.2(1 H), 6.45(1 H), 6.9(2H), 7.1(2H), 7.3(1 H), 7.5(3H). 7.55-7.7(2H), 8.0(1H) und 8.15(2H) ppm.

### Beispiel 179

### 4-(4-Ethyl-piperazin-1-yl)-piperidin-1-karbonsäure-[5-cyano-1-(2-methoxy-benzolsulfonyl)-3-(2-ethoxy-phenyl)-2-oxo-2,3-dihydro-1H-indol-3-yl]-amid

¹H-NMR (CDCl₃): δ = 1.1(3H), 1.35(2H), 1.5(3H), 1.75(2H), 2.25-2.8(13H), 3.55(3H), 3.75(2H), 4.15(1H), 4.25(1H), 6.65(1H), 6.85(1H), 6.9(2H), 6.95(1H), 7.05(1H), 7.3(1H), 7.55(1H), 7.6(2H), 8.1(1H) und 8.15(1H) ppm.

### Beispiel 180

### 4-(4-Ethyl-piperazin-1-yl)-piperidin-1-karbonsäure-[5-cyano-1-(2 fluoro-benzolsulfonyl)-3-(2-ethoxy-phenyl)-2-oxo-2,3-dihydro-1H-indol-3-yl]-amid

¹H-NMR (CDCl₃): δ = 1.1 (3H), 1.25(1H), 1.35(1H), 1.5(3H), 1.55(2H), 1.75(2H), 2.25-2.8(13H), 3.5(3H), 3.7(2H), 4.1(1H), 4.2(1H), 6.45(1H), 6.9(2H), 7.1(1H), 7.15(1H), 7.3(2H), 7.5(1H), 7.6(2H), 8.1(1H) und 8.15(1H) ppm.

### Beispiel 181

### 4-(4-Propy)-piperazin-1-yl)-piperidin-1-karbonsäure-[5-cyano-1-(2,4-dimethoxy-benzolsulfonyl)-3-(2-isopropoxy-phenyl)-2-oxo-2,3-dihydro-1H-indol-3-yl]-amid

¹H-NMR (CDCl₃): δ = 0.9(3H), 1.25-1.4(5H), 1.45-1.65(5H), 1.75(2H), 2.25-2.8(13H), 3.5(3H), 3.7(2H), 3.9(3H); 4.75(1H), 6.4(1H), 6.55(1 H), 6.65(1H), 6.85(1 H), 6.9(1 H), 7.0(1 H), 7.25(1 H), 7.6(1 H), 7.65(1 H) und 8.1(2H) ppm.

### Beispiel 182

### 4-(4-Propyl-piperazin-1-yl)-pipendin-1-karbonsäure-[5-cyano-1-(2-methoxy-benzolsulfonyl)-3-(2-isopropoxy-phenyl)-2-oxo-2,3-dihydro-1H-indol-3-yl]-amid

¹H-NMR (CDCl₃): δ = 0.9(3H), 1.25-1.4(5H), 1.45-1.65(5H), 1.75(2H), 2.25-2.8(13H), 3.5(3H), 3.75(2H), 4.75(1 H), 6.65(1 H), 6.85(1 H), 6.9(2H), 7.0(1 H), 7.05(1H), 7.25(1H), 7.55(1H), 7.6(1H), 7.65(1H), 8.1(1H) und 8.1(1H) ppm.

### Beispiel 183

### 4-(4-Propyl-piperazin-1-yl)-piperidin-1-karbonsäure-[5-cyano-1-(4-methoxy-benzolsulfonyl)-3-(2-isopropoxy-phenyl)-2-oxo-2,3-dihydro-1H-indol-3-yl]-amid

¹H-NMR (CDCl₃): δ = 0.9(3H), 1.3(3H), 1.35(2H), 1.45(3H), 1.5(2H), 1.75(2H), 2.25-2.8(13H), 3.7(2H), 3.85(3H), 4.7(1H), 6.4(1H), 6.8-7.0(4H), 7.1(1H), 7.25(1 H), 7.5(1 H), 7.6(1 H) und 8.0-8.1 (3H) ppm.

### Beispiel 184

### 4-(4-Propyl-piperazin-1-yl)-piperidin-1-karbonsäure-[5-cyano-1-(4-fluor-benzolsulfonyl)-3-(2-isopropoxy-phenyl)-2-oxo-2,3-dihydro-1H-indol-3-yl]-amid

¹H-NMR (CDCl₃): δ = 0.9(3H), 1.25-1.4(5H), 1.4-1.65(5H), 1.75(2H), 2.25-2.8(13H), 3.7(2H), 4.7(1H), 6.35(1H), 6.8(2H), 7.1-7.2(3H), 7.3(1H), 7.45(1H), 7.6(1H), 8.05(1 H) und 8.1(2H) ppm.

### Beispiel 185

### 4-(4-Isopropyl-piperazin-1-yl)-piperidin-1-karbonsäure-[5-cyano-1-(2,4-dimethoxy-benzolsulfonyl)-3-(2-ethoxy-phenyl)-2-oxo-2,3-dihydro-1H-indol-3-yl]-ester

¹H-NMR (CDCl₃): δ = 1.1(6H), 1.15-1.35(4H), 1.4-1.7(1H), 1.7-1.9(2H), 2.4(1H), 2.45-2.8(10H), 2.95(1H), 3.5(3H), 3.6(1H), 3.8(1H), 3.85(3H), 4.05(1H), 4.2(1 H), 6.4(1H), 6.55(1H), 6.75(1H), 7.0(1H), 7.2-7.35(2H), 7.6-7.7(2H), 8.05(1 H) und 8.1(1H) ppm.

### Beispiel 186

### 4-(4-Isopropyl-piperazin-1-yl)-pipeddin-1-karbonsäure-[5-cyano-1-(2,4-difluor-benzolsulfonyl)-3-(2-ethoxy-phenyl)-2-oxo-2,3-dihydro-1H-indol-3-yl]-ester

¹H-NMR (CDCl₃): δ = 1.1(6H), 1.25(3H), 1.4-1.95(4H), 2.35(1H), 2.4-2.9(10H), 2.95(1H), 3.5(1H), 3.8(1H), 4.0(1H), 4.2(1 H), 6.8(1H), 6.9(1H), 7.0(2H), 7.25(1 H), 7.3(1 H), 7.7(2H) und 8.15(2H) ppm.

### Beispiel 187

### 4-(4-isopropyl-piperazin-1-yl-piperidin-1-karbonsäure-[5-cyano-1-(2-fluor-benzolsulfonyl)-3-(2-ethoxy-phenyl)-2-oxo-2,3-dihydro-1H-indol-3-yl]-ester

¹H-NMR (CDCl₃): δ = 1.1(6H), 1.25(3H), 1.35-1.95(6H), 2.35-3.05(10H), 3.5(1 H), 3.8(1H), 4.0(1H), 4.2(1H), 6.8(1H), 7.0(1 H), 7.15(1H), 7.2-7.4(3H), 7.6(1 H), 7.65(2H) und 8.15(2H) ppm.

### Beispiel 188

### 4-(4-Isopropyl-piperazin-1-yl)-piperidin-1-karbonsäure-[5-cyano-1-(4-fluor-benzolsulfonyl)-3-(2-ethoxy-phenyl)-2-oxo-2,3-dihydro-1H-indol-3-yl]-ester

¹H-NMR (CDCl₃): δ = 1.1(6H), 1.25(3H), 1.35-1.95(4H), 2.35(1H), 2.4-2.8(10H), 2.95(1H), 3.5(1H), 3.8(1H), 4.0(1 H), 4.2(1 H), 6.8(1H), 7.05(1H), 7.15-7.4(4H), 7.6-7.75(2H) und 8.1-8.2(3H) ppm.

### Beispiel 189

### 4-(4-Propyl-piperazin-1-yl)-piperidin-1-karbonsäure-[5-cyano-1-(4-fluor-benzolsulfonyl)-3-(2-ethoxy-phenyl)-2-oxo-2,3-dihydro-1 H-indol-3-yl]-ester

¹H-NMR (CDCl₃): δ = 0.9(3H), 1.1-1.35(4H), 1.35-1.8(5H), 1.9(1H), 2.2-2.85(11 H), 2.95(1H), 3.5(1H), 3.8(1 H), 4.0(1H), 4.2(1H), 6.8(1 H), 7.05(1H), 7.15-7.3(3H), 7.35(1 H), 7.6-7.75(2H) und 8.0-8.15(3H) ppm.

### Beispiel 190

### 3-(2-Ethoxy-phenyl)-1-benzolsulfonyl-2-oxo-3-{2-oxo-2-[4-(4-propyl-piperazin-1-yl)-piperidin-1-yl]-ethyl}-2,3-dihydro-1H-indol-5-karbonsäurenitril

¹H-NMR (CDCl₃): δ = 0.9(3H), 1.0-1.2(1H), 1.2-1.35(3H), 1.35(1H), 1.45-1.8(4H), 1.85(1H), 2.2(1 H), 2.25-2.8(10H), 2.9(1H), 3.3(1 H), 3.65(1H), 3.75-4.1(4H), 6.8(1H), 6.95(1H), 7.25(2H), 7.35(1H), 7.45-7.7(4H), 8.05(1H) und 8.15(2H) ppm.

### Beispiel 191

### 3-(2-Ethoxy-phenyl)-1-(2-methoxy-benzolsulfonyl)-2-oxo-3-{2-oxo-2-[4-(4-propyl-piperazin-1-yl)-piperidin-1-yl]-ethyl}-2,3-dihydro-1H-indol-5-karbonsäurenitril

¹H-NMR (CDCl₃): δ = 0.9(3H), 1.2(1H), 1.4(4H), 1.5-1.9(5H), 2.2-2.75(11H), 2.9(1H), 3.25(1H), 3.6(3H), 3.75-3.85(2H), 3.9-4.05(2H), 4.15(1H), 6.75-6.95(3H), 7.05(1H), 7.15-7.3(3H), 7.5-7.65(2H). 8.1 (1 H) und 8.15(1H) ppm.

### Beispiel 192

### 3-(2-Ethoxy-phenyl)-1-(4-methoxy-benzolsulfonyl)-2-oxo-3-{2-oxo-2-[4-(4-propyl-piperazin-1-yl)-piperidin-1-yl]-ethyl}-2,3-dihydro-1H-indol-5-karbonsäurenitril

### a) 4-(4-Propyl-piperazin-1-yl)-piperidin-1-karbonsäure-tert-butylester

Zu 73 g (0,25 Mol) N-Propylpiperazindihydrobromid in 1L Methanol wurden bei 40°C 42 g (0,50 Mol) Natriumazetat portionsweise gegeben. Anschließend wurde auf 0°C abgekühlt und nacheinander 50 g (0,25 Mol) Boc-4-piperidon und portionsweise 16g (0.25Mol) Natriumcyanoborhydrid hinzugefügt. Man ließ alles für 16h bei Raumtemperatur rühren. Das Reaktionsgemisch wurde im Vakuum konzentriert und danach zwischen Essigester und 1 M NaOH verteilt. Die organische Phase wurde abgetrennt, mit 1 M NaOH , H₂O und conc. NaCl-Lösung gewaschen, getrocknet und im Vakuum eingeengt. Der Rückstand wurde chromatographisch über Kieselgel gereinigt (Fließmittel: MeOH/CH₂Cl₂= 1/15). Man erhielt 43,6g des Produktes.

### b) 1-Piperidin-4-yl-4-propyl-piperazinä-trihydrochlorid

43,5g (0,14 Mol) des Zwischenproduktes 192a wurden in 500 ml Methanol gelöst und anschließend bei 40°C langsam mit 100ml 5-6M isopropanolische HCl versetzt, wobei zeitweise eine kräftige Gasentwicklung einsetzte und das Produkt teilweise auskristallisierte. Nach 30 Minuten war die Gasentwicklung beendet. Danach gab man erneut 50ml 5-6M isopropanolische HCl hinzu und ließ alles für 1h bei 40°C rühren. Man ließ abkühlen und isolierte das ausgefallene Produkt. Man erhielt 34 g des Produktes.

### c) 3-(2-Ethoxy-phenyl)-3-hydroxy-5-iodo-1,3-dihydro-indol-2-on

8,0g (1,65 Mol) Magnesiumspäne wurden mit 40 ml Ether überschichtet, und nach Zugabe von wenig Jod wurde vorsichtig erwärmt bis die Reaktion ansprang. Zu der siedenden Lösung wurden 66,3 g (0,33 Mol) 2-Brom-1-ethoxybenzol, gelöst in 200 ml Ether, langsam so zugetropft, daß die Reaktion unter leichtem Sieden kontinuierlich ablief. Anschließend wurden unter leichter Kühlung auf 20°C 30 g (0,11 Mol) 5-Jodisatin in 800 ml wasserfreiem Tetrahydrofuran zugetropft. Danach wurde alles noch 30 Minuten bei Raumtemperatur gerührt. Die Reaktionslösung wurde unter Rühren in eine wäßrige NH₄Cl-Lösung gegossen. Diese wäßrige Phase wurde mehrmals mit Essigester extrahiert und die vereinigten wäßrigen Phasen viermal mit Wasser gewaschen, getrocknet und im Vakuum konzentriert, wonach langsam ein Festkörper ausfiel, der isoliert und getrocknet wurde. Man erhielt 33,6 g des Zwischenproduktes.

### d) 3-(2-Ethoxy-phenyl)-3-hydroxy-2-oxo-2,3-dihydro-1H-indol-5-karbonsäurenitril

37 g (94 mMol) des Zwischenproduktes 192c und 11 g (94 mMol) Zinkcyanid wurden in 300 ml DMF gegeben und alles zügig auf 90-95°C aufgeheizt. Danach wurden 1,6 g (1,4 mMol) Pd[Ph₃P]₄ in zwei Portionen innerhalb von 20 Minuten zugefügt. Nach weiteren 30 Minuten wurde das Reaktionsgemisch auf Eiswasser gegossen und mit Essigester extrahiert. Die organische Phase wurde isoliert mit Wasser und gesättigter NaCl-Lösung gewaschen, getrocknet und im Vakuum eingeengt. Der Rückstand wurde aus wenig Essigester kristallisiert und die Kristalle isoliert. Man erhielt 24 g des Produktes.

### e) 3-Chlor-3-(2-ethoxy-phenyl)-2-oxo-2,3-dihydro-1H-indol-5-karbonsäurenitril

10g (34 mMol) des Zwischenproduktes 192d und 5,6 ml (68 mMol) Pyridin wurden in 120 ml CH₂Cl₂ gelöst. Danach wurde alles auf 0°C abgekühlt und 3,7 ml (51 mMol) SOCl₂ zugetropft. Das Reaktionsgemisch wurde noch 1h gerührt. Anschließend wurde alles vorsichtig in Eiswasser gegeben, die organische Phase abgetrennt, mehrmals mit H₂O gewaschen, getrocknet und im Vakuum eingeengt. Der erhaltene Rückstand wurde mit n-Pentan behandelt und der entstandene Festkörper isoliert, wonach man 9,9 g des Produktes erhielt.

### f) 2-[5-Cyano-3-(2-ethoxy-phenyl)-2-oxo-2,3-dihydro-1H-indol-3-yl]-malonsäuredimethylester

3,8 g (96 mMol) NaH(60%) wurden vorsichtig zu 200ml wasserfreiem DMF gegeben. Bei 10°C wurden danach langsam 12 ml (105 mMol) Malonsäuredimethylester zugefügt. Alles wurde noch 30 Minuten bei Raumtemperatur gerührt. Anschließend wurden 10g (32 mMol) des Zwischenproduktes 192e portionsweise zugegeben und das Reaktionsgemisch noch 15 Minuten gerührt. Dieses Gemisch wurde vorsichtig in 1 M HCl eingerührt und anschließend alles gekühlt, wobei ein Niederschlag anfiel, der isoliert und aus CH₂Cl₂/Pentan umkristallisiert wurde. Man erhielt 10,7 g des Produktes.

### g) [5-Cyano-3-(2-ethoxy-phenyl)-2-oxo-2,3-dihydro-1H-indol-3-yl]-essigsäure-methylester

10,7 g (26 mMol) des Zwischenproduktes 192f wurden in 10 ml Ethanol gelöst. Man gab 100 ml 2M Natronlauge hinzu und rührte für 1h bei Raumtemperatur. Der Reaktionsansatz wurde in 1 M HCl eingerührt, wobei ein Niederschlag anfiel, der isoliert und getrocknet wurde. Dieser Festkörper wurde in ein 1 L Gefäß überführt und auf 150°C erwärmt, wobei dieser durch Gasentwicklung aufschäumt. Nach Beendigung der Reaktion läßt man abkühlen. Der Rückstand wurde mit Methanol behandelt und der erhaltene Niederschlag isoliert. Man emieit 6,4 g des Produktes.

### h) [5-Cyano-3-(2-ethoxy-phenyl)-2-oxo-2,3-dihydro-1H-indol-3-yl]-essigsäure

Zu 5,9 g (16,8 mMol) des Zwischenproduktes 192g in 25 ml Ethanol wurden 36 ml 2M NaOH gegeben und alles für 3h bei Raumtemperatur gerührt. Anschließend wurde das Reaktionsgemisch mit 6 ml Essigsäure angesäuert und mit Wasser verdünnt. Über Nacht fiel ein Festkörper aus, der isoliert und getrocknet würde. Man erhielt 5,2 g des Produktes.

### i) 3-(2-Ethoxy-phenyl-2-oxo-3-{2-oxo-2-[4-(4-propyl-piperazin-1-yl)-piperidin-1-yl]-ethyl}-2,3-dihydro-1H-indol-5-karbonsäurenitril

Zu 1,9 g (5,4 mMol) des Zwischenproduktes 192b in 25 ml wasserfreiem DMF wurden bei 0°C vorsichtig 1,8 g (16,3 mMol) t-BuOK gegeben. Anschließend gab man nacheinander 2 g (5,4 mMol) des Zwischenproduktes 192h, 0,8 g (5,4 mMol) HOBT, 2,9 ml (20,9 mMol) Et₃N und schließlich portionsweise 1,1 g (5,4 mMol) EDAC zu. Das Reaktionsgemisch wurde dann bei Raumtemperatur für 16h gerührt. Dieses Gemisch wurde nun in 5%ige K₂CO₃-Lösung eingerührt, wobei ein Niederschlag anfiel, der isoliert und getrocknet wurde. Man erhielt 2,6 g des Produktes.

### j) 3-(2-Ethoxy-phenyl)-1-(4-methoxy-benzenesulfonyl)-2-oxo-3-{2-oxo-2-[4-(4-propyl-piperazin-1-yl)-piperidin-1-yl]-ethyl}-2,3-dihydro-1H-indol-5-karbonsäurenitril

Zu 0,2 g (0,38 mmol) des Zwischenproduktes 192i in 4 ml DMF wurden bei 0°C 47 mg (0,42 mMol) t-BuOK gegeben. Alles wurde für 1 h bei 0°C gerührt. Anschließend wurden bei 0°C 86 mg (0,42 mMol) 4-Methoxybenzolsulfonylchlorid portionsweise hinzugefügt und alles für 16h gerührt. Der Reaktionsansatz wurde in 1 M NaOH eingerührt und der entstandene Niederschlag isoliert. Dieser wurde noch aus Methanol umkristallisiert, wobei 0,15 g des Produktes anfielen.
¹H-NMR (CDCl₃): δ = 0.9(3H), 1.0-1.2(1H), 1.2-1.3(3H), 1.35(1H), 1.45-1.75(4H), 1.85(1H), 2.25(1H), 2.3-2.8(10H), 2.9(1H), 3.3(1H), 3.65(1H), 3.75-3.95(5H), 4.0(1H), 6.8(1 H), 6.9-7.0(3H), 7.25(2H), 7.35(1H), 7.55(1H) und 8.0-8.15(3H) ppm.

### Beispiel 193

### 3-(2-Ethoxy-phenyl)-1-(2-fluor-benzenesulfonyl)-2-oxo-3-{2-oxo-2-[4-(4-propyl-piperazin-1-yl)-piperidin-1-yl]-ethyl}-2,3-dihydro-1H-indol-5-karbonsäurenitril

¹H-NMR (CDCl₃): δ = 0.9(3H), 1.15(1H), 1.25-1.4(4H), 1.4-1.75(4H), 1.8(1H), 2.1-2.8(11H), 2.9(1 H), 3.3(1 H), 3.65(1 H), 3.8(1 H), 3.85(1 H), 3.95(1 H), 4.0-4.15(2H), 6.8(1H), 6.95(1H), 7.15(1H), 7.2-7.4(3H), 7.6(2H), 8.15(1H) und 8.2(1 H) ppm.

### Beispiel 194

### 3-(2-Ethoxy-phenyl)-1-(2,4-dimethoxy-benzolsulfonyl)-2-oxo-3-{2-oxo-2-[4-(4-propyl-piperazin-1-yl)-piperidin-1-yl]-ethyl}-2,3-dihydro-1H-indol-5-karbonsäurenitril

¹H-NMR (CDCl₃): δ = 0.9(3H), 1.2(1H), 1.3-1.45(4H), 1.45-1.9(5H), 2.2-2.8(11H), 2.9(1H), 3.25(1H), 3.55(1H), 3.75-3.9(5H), 3.95(1H), 4.05(1H), 4.15(1H), 6.4(1H), 6.55(1H), 6.85(1H), 6.9(1H), 7.2(1H), 7.25(1H), 7.55(2H) und 8.1(2H) ppm.

### Beispiel 195

### 3-(2-Ethoxy-phenyl)-1-(2,4-difluor-benzolesulfonyl)-2-oxo-3-{2-oxo-2-[4-(4-propyl-piperazin-1-yl)-piperidin-1-yl]-ethyl}-2,3-dihydro-1H-indol-5-karbonsäurenitril

¹H-NMR (CDCl₃): δ = 0.9(3H), 1.15(1H), 1.2-1.4(4H), 1.4-1.75(3H), 1.85(1H), 2.15-2.7(11H), 2.9(1H), 3.3(1H), 3.65(1H), 3.75-4.0(2H), 4.1(1H), 6.8(1H), 6.85(1H), 6.9-7.0(2H), 7.3(3H), 7.6(1 H), 8.1(1H) und 8.2(1 H) ppm.

### Beispiel 196

### 3-(2-Ethoxy-phenyl)-1-(4-chlor-benzolsulfonyl)-2-oxo-3-{2-oxo-2-[4-(4-propyl-piperazin-1-yl)-piperidin-1-yl]-ethyl}-2,3-dihydro-1H-indol-5-karbonsäurenitril

¹H-NMR (CDCl₃): δ = 0.9(3H), 1.0(1H), 1.15(1H), 1.25-1.8(4H), 1.85(1H), 2.15-2.8(11H), 2.95(1H), 3.3(1H), 3.6(1H), 3.7-3.95(3H), 4.0-4.15(1H), 6.8(1 H), 6.95(1 H), 7.3(3H), 7.45(2H), 7.55(1 H) und 8.0-8.1(3H) ppm.

### Beispiel 197

### 4-(4-Isopropyl-piperazin-1-yl)-piperidin-1-karbonsäure-[5-cyano-1-benzolsulfonyl-3-(2-ethoxy-phenyl)-2-oxo-2,3-dihydro-1H-indol-3-yl]-amid

¹H-NMR (CDCl₃): δ = 1.05(6H), 1.3(2H), 1.5(3H), 1.75(2H), 2.3(1 H), 2.5-2.8(11H), 3.7(2H), 4.1(1H), 4.2(1 H), 6.45(1H), 6.9(2H), 7.05(1H), 7.3(1H), 7.5(3H), 7.55-7.65(2H), 8.05(1 H) und 8.15(1 H) ppm.

### Beispiel 198

### 4-(4-Isopropyl-piperazin-1-yl)-piperidin-1-karbonsäure-[5-cyano-1-(2-methoxy-benzolsulfonyl)-3-(2-ethoxy-phenyl)-2-oxo-2,3-dihydro-1H-indol-3-yl]-amid

¹H-NMR (CDCl₃): δ = 1.05(6H), 1.35(3H), 1.55(3H), 1.75(2H), 2.3(1 H), 2.45-2.8(11 H), 3.5(3H), 3.75(2H), 4.15(1 H), 4.25(1 H), 6.7(1H), 6.85(1 H), 6.9(2H), 6.95(1H), 7.05(1H), 7.25(1H), 7.55(1H), 7.6(2H), 8.1(1H) und 8.15(1 H) ppm.

### Beispiel 199

### 4-(4-Isopropyl-piperazin-1-yl)-piperidin-1-karbonsäure-[5-cyano-1-(4-methoxy-benzolsulfonyl)-3-(2-ethoxy-phenyl)-2-oxo-2,3-dihydro-1H-indol-3-yl]-amid

¹H-NMR (CDCl₃): δ = 1.05(6H), 1.35(3H), 1.5(3H), 1.75(2H), 2.3(1H), 2.5-2.8(11H), 3.7(2H), 3.85(3H), 4.0(1H), 4.2(1H), 6.5(1H), 6.85-7.0(4H), 7.05(1 H), 7.3(1H), 7.5(1 H), 7.55(1 H) und 7.95-8.1(3H) ppm.

### Beispiel 200

### 4-(4-Isopropyl-piperazin-1-yl)-piperidin-1-karbonsäure-[5-cyano-1-(2-fluor-benzolsulfonyl)-3-(2-ethoxy-phenyl)-2-oxo-2,3-dihydro-1H-indol-3-yl]-amid

¹H-NMR (CDCl₃): δ = 1.05(6H), 1.25(1H), 1.35(1H), 1.5(3H), 1.75(2H), 2.3(1H), 2.45-2.8(11H), 3.7(2H), 4.1(1H), 4.2(1H), 6.45(1H), 6.9(2H), 7.1(1H), 7.15(1H), 7.3(2H), 7.5(1 H), 7.6(2H), 8.1(1H) und 8.15(1 H) ppm.

### Beispiel 201

### 4-(4-Isopropyl-piperazin-1-yl)-piperidin-1-karbonsäure-[5-cyano-1-(2,4-difluor-benzolsulfonyl)-3-(2-ethoxy-phenyl)-2-oxo-2,3-dihydro-1H-indol-3-yl]-amid

¹H-NMR (D₆-DMSO): δ = 1.05(6H), 1.25(1H), 1.35(1 H), 1.45(3H), 1.75(2H), 2.3(1h), 2.5-2.8(11H), 3.7(2H), 4.1(1H), 4.2(1H), 6.4(1H), 6.8-7.05(4H), 7.15(1 H), 7.3(1 H), 7.5(1H), 7.6(1 H), 8.1(1H) und 8.2(1H) ppm.

### Beispiel 202

### 4-(4-Ethyl-piperazin-1-yl)-piperidin-1-karbonsäure-[5-cyano-1-(4-methoxy-benzolsulfonyl)-3-(2-ethoxy-phenyl)-2-oxo-2,3-dihydro-1H-indol-3-yl]-amid

¹H-NMR (D₆-DMSO): δ = 0.95(3H), 1.1(3H), 1.15(2H), 1.6(2H), 2.2-2.7(12H), 3.75(2H), 3.85(3H), 3.9(2H), 6.9-7.0(2H), 7.1(2H), 7.3(1H), 7.5(1 H), 7.55(1 H), 7.6(1 H), 7.8(1 H), 7.85(1 H) und 7.95(2H) ppm.

### Beispiel 203

### 4-(4-Ethyl-piperazin-1-yl)-piperidin-1-karbonsäure-[5-cyano-1-(2,4-difluor-benzolsulfonyl)-3-(2-ethoxy-phenyl)-2-oxo-2,3-dihydro-1H-indol-3-yl]-amid

¹H-NMR (CDCl₃): δ = 1.1(3H), 1.25(1H), 1.35(1H), 1.45(3H), 1.75(2H) 2.25-2.8(12H), 3.7(2H), 4.1(1H), 4.2(1H), 6.4(1H), 6.8-7.05(4H), 7.15(1H), 7.35(1 H), 7.5(1 H), 7.6(1 H), 8.1 (1 H) und 8.2(1 H) ppm.

### Beispiel 204

### 4-(4-Ethyl-piperazin-1-yl)-piperidin-1-karbonsäure-[5-cyano-1-(4-fluor-benzolsulfonyl)-3-(2-ethoxy-phenyl)-2-oxo-2,3-dihydro-1H-indol-3-yl]-amid

¹H-NMR (CDCl₃): δ = 1.1(3H), 1.3(2H), 1.5(3H), 1.8(2H), 2.2-2.8(13H), 3.7(2H), 4.1(1H), 4.2(1H), 6.4(1H), 6.9(2H), 7.05-7.25(3H), 7.3(1H), 7.45(1H), 7.6(1 H), 8.05(1 H) und 8.15(2H) ppm.

### Beispiel 205

### 4-(4-Propyl-piperazin-1-yl)-piperidin-1-karbonsäure-[5-cyano-1-(4-fluoro-benzolsulfonyl)-3-(2-ethoxy-phenyl)-2-oxo-2,3-dihydro-1H-indol-3-yl]-amid

¹H-NMR (CDCl₃): δ = 0.9(3H), 1.3(2H), 1.4-1.6(5H), 1.75(2H), 2.2-2.8(13H), 3.7(2H), 4.1(1H), 4.2(1 H), 6.4(1 H), 6.9(2H), 7.05-7.2(3H), 7.3(1 H), 7.45(1 H), 7.6(1 H), 8.0(1 H) und 8.15(2H) ppm.

### Beispiel 206

### 4-(1-Methyl-piperidin-4-yl)-piperazin-1-karbonsäure-1 (4-methoxy-1-benzolsulfonyl)-5-cyano-2-oxo-3-(2-ethoxyphenyl)-2,3-dihydro-1H-indol-3-yl]-ester

¹H-NMR (CDCl₃):δ = 1.25(3H), 1.6-1.9(4H), 2.1(2H), 2.25-2.45(4H), 2.5(1H), 2.6(1 H), 2.9-3.2(4H), 3.6(2H), 3.8(1 H), 3.85(3H), 4.0(1 H), 6.8(1H), 6.95(2H), 7.05(1 H), 7.25(1 H), 7.35(1 H), 7.65(1 H), 7.7(1 H) und 8.05(2H) ppm.

### Beispiel 207

### 4-(4-Isopropyl-piperazin-1-yl)-piperidin-1-karbonsäure-[5-cyano-1-(4-fluoro-benzolsulfonyl)-3-(2-ethoxy-phenyl)-2-oxo-2,3-dihydro-1H-indol-3-yl]-amid

¹H-NMR (D₆-DMSO): δ = 1.05(6H), 1.35(2H), 1.5(3H), 1.75(2H), 2.3(1H), 2.5-2.8(11H), 3.7(2H), 4.1(1H), 4.2(1H), 6.45(1H), 6.9(2H), 7.1-7.25(3H), 7.3(1 H), 7.45(1 H), 7.6(1 H), 8.05(1 H) und 8.15(2H) ppm.

### Beispiel 208

### 3-(2-Ethoxy-phenyl)-1-(4-fluoro-benzolsulfonyl)-2-oxo-3-2-oxo-{2-[4-(4-propyl-piperazin-1-yl)-piperidin-1-yl]-ethyl}-2,3-dihydro-1H-indol-5-karbonsäurenitril

¹H-NMR (CDCl₃): δ = 0.9(3H), 1.1(1H), 1.15(1H), 1.25-1.4(4H), 1.45-1.6(2H), 1.7(1H), 1.85(1H), 2.15-2.7(12H), 2.95(1H), 3.3(1H), 3.65(1H), 3.75-3.95(3H), 4.05(1H), 6.8(1H), 6.95(1H), 7.15(2H), 7.25-7.4(3H), 7.55(1H), 8.05(1 H) und 8.15(2H) ppm.

## Patentansprüche

1. Verbindung oder Verbindungen der allgemeinen Formel (I), 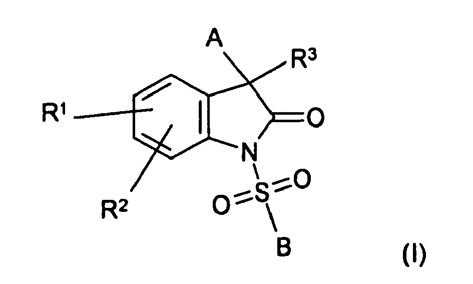 worin
A C₆₋₁₀-Aryl ist, das mit maximal vier Resten R⁴ substituiert sein kann, die unabhängig voneinander ausgewählt sind aus der Gruppe, bestehend aus Wasserstoff, Chlor, Brom, Jod, Fluor, (CH₂)₀₋₂-CN, CF₃, OCF₃, CONH₂, CONH(C₁-C₄-Alkyl), CON(C₁-C₄-Alkyl)(C₁-C₄-Alkyl), NHCHO, NHCONH₂, N(C₀-C₄-Alkylen)CONH₂, N(C₀-C₄-Alkylen)CONH(C₁-C₄-Alkyl), NHCOCH₃, NO₂, (CH₂)₀₋₂-OH, O-C₁₋C₆-Alkyl, (CH₂)₀₋₂-O-C₁-C₄-Alkyl, O-C₀-C₄-Alkylen-Phenyl, Phenyl, C₁-C₆-Alkyl, C₂-C₆-Alkenyl und C₂-C₆-Alkinyl,
B ein aromatischer oder teilaromatischer C₆₋₁₀-Mono- oder -Bicyclus ist, der mit den Resten R⁶, R⁷, R⁸ und/oder R⁹ substituiert sein kann, wobei R⁶, R⁷, R⁸ und R⁹ unabhängig voneinander ausgewählt sind aus der Gruppe, bestehend aus Wasserstoff, Chlor, Brom, Jod, Fluor, (CH₂)₀₋₂-CN, CF₃, OCF₃, CONH₂, CONH(C₁-C₄-Alkyl), CON(C₁-C₄-Alkyl)(C₁-C₄Alkyl), NHCHO, N(C₀₋₄-Alkylen)CONH(C₁-C₄-Alkyl), NHCOCH₃, NO₂, OH, O-C₁-C₄-Alkyl, (CH₂)₀₋₂-O-(CH₂)₀₋₃-CH₃, O-C₀-C₄-Alkylen-Phenyl, Phenyl, C₁-C₆-Alkyl, C₂-C₆-Alkenyl und C₂-C₆-Alkinyl,
R¹ Wasserstoff, C₁-C₆-Alkyl, OH, O-(C₁-C₄-Alkyl), N(C₁-C₄-Alkyl)(C₁-C₄-Alkyl), CN, CONH₂, OCF₃, CF₃, Br, F, Cl, J, NO₂, NHCHO, NHCO(C₁-C₄-Alkyl) oder NHCONH₂ ist,
R² Wasserstoff, C₁-C₄-Alkyl, O-(C₁-C₄-Alkyl), Cl oder F ist,
R³ ein Rest (W)-(X)-(Y)-Z ist, wobei
W C₁-C₄-Alkylen, (C₀-C₄-Alkylen)-O-(C₀-C₄-Alkylen) oder (C₀-C₄-Alkylen)-NR¹⁵-(C₀-C₄-Alkylen) ist, worin R¹⁵ Wasserstoff oder C₁-C₄-Alkyl ist,
X CO, SO₂, (C=NH) oder (C=N-CN) ist und
Y ein Rest ausgewählt aus der Gruppe, bestehend aus 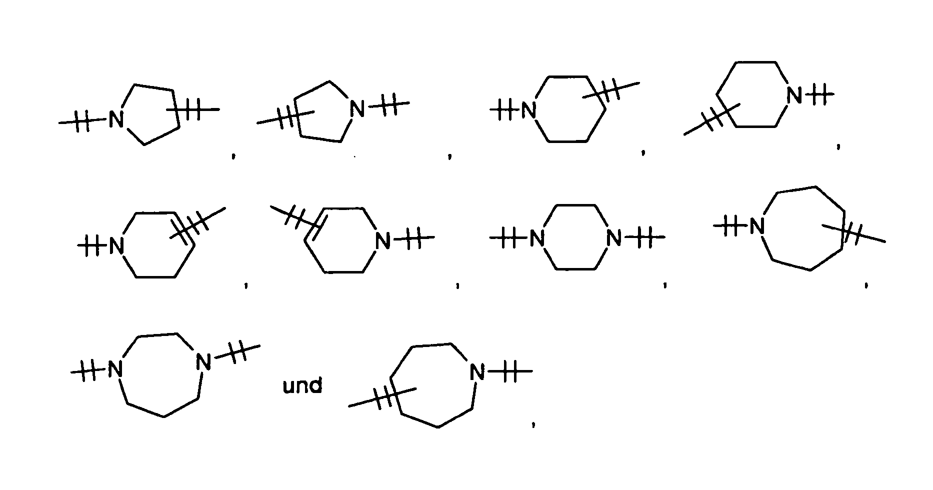 Ist, wobei Y zusätzlich durch R¹⁰ und/oder R¹¹ substituiert sein kann,und
R¹⁰ Wasserstoff, C₁-C₆-Alkyl, C₂-C₆-Alkenyl, C₂-C₆-Alkinyl, OH, O-C₁-C₄-Alkyl, O-C₀-C₄-Alkylen-Phenyl, NH₂, NH(C₁-C₄-Alkyl) oder N(C₁-C₄-Alkyl)(C₁-C₄-Alkyl) ist,
R¹¹ Wasserstoff, C₁-C₆-Alkyl, C₂-C₆-Alkenyl, C₂-C₆-Alkinyl, OH, O-C₁-C₄-Alkyl, O-C₀-C₄-Alkylen-Phenyl, NH₂, NH(C₁-C₄-Alkyl) oder N(C₁-C₄-Alkyl)(C₁-C₄-Alkyl) ist, und
Z ein Rest ausgewählt ist aus der Gruppe, bestehend aus 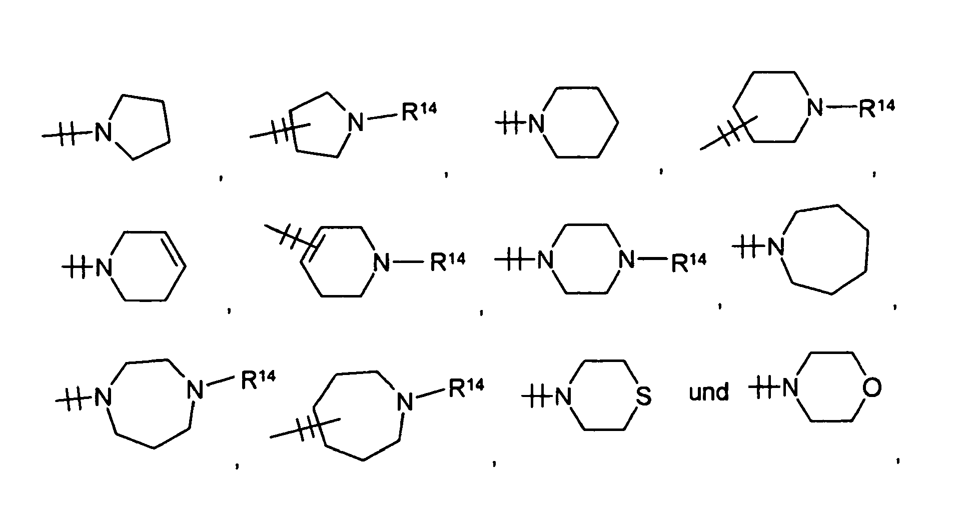 Ist, wobei Z zusätzlich durch R¹² und/oder R¹³ substituiert sein kann, wobei
R¹² Wasserstoff, C₁-C₆-Alkyl, C₂-C₆-Alkenyl, C₂-C₆-Alkinyl, OH, O-C₁-C₄-Alkyl, O-C₀-C₄-Alkylen-Phenyl, NH₂, NH(C₁-C₄-Alkyl) oder N(C₁-C₄-Alkyl)(C₁-C₄-Alkyl) ist,
R¹³ Wasserstoff, C₁-C₆-Alkyl, C₂-C₆-Alkenyl, C₂-C₆-Alkinyl, OH, O(C₁-C₄-Alkyl), O-C₀-C₄-Alkylen-Phenyl, NH₂, NH(C₁-C₄-Alkyl) oder N(C₁-C₄-Alkyl)(C₁-C₄-Alkyl) ist,
R¹⁴ Wasserstoff, C₁-C₆-Alkyl, C₂-C₆-Alkenyl, C₂-C₆-Alkinyl oder C₀-C₄-Alkylen-Phenyl ist, und
ihre tautomeren, enantiomeren und/oder diastereomeren Formen, sowie die physiologisch verträglichen Salze der vorstehend genannten Verbindung oder Verbindungen.

2. Verbindung oder Verbindungen nach Anspruch 1, worin A ein Phenyl-Ring ist, der mit maximal vier Resten R⁴ substituiert sein kann, und B ein Phenyl-Ring ist, der mit den Resten R⁶, R⁷, R⁸ und/oder R⁹ substituiert sein kann, wobei die Reste R⁶, R⁷, R⁸ und R⁹ die in Anspruch 1 genannten Bedeutungen haben sollen,
und ihre tautomeren, enantiomeren und/oder diastereomeren Formen, sowie die physiologisch verträglichen Salze der vorstehend genannten Verbindung oder Verbindungen.

3. Verbindung oder Verbindungen nach Anspruch 1, worin
A ein Phenyl-Ring ist, der mit maximal zwei Resten R⁴ substituiert sein kann, die unabhängig voneinander ausgewählt sind aus der Gruppe, bestehend aus Wasserstoff, Chlor, O-C₁-C₄-Alkyl, (CH₂)₀₋₂-O-(CH₂)₀₋₂-CH₃ und d-C₁-C₆Alkyl,
B ein Phenyl-Ring ist, der mit den Resten R⁶, R⁷, R⁸ und/oder R⁹ substituiert sein kann, wobei R⁶, R⁷, R⁸ und R⁹ unabhängig voneinander ausgewählt sind aus der Gruppe, bestehend aus Wasserstoff, Fluor, Chlor, O-C₁-C₄-Alkyl, (CH₂)₀₋₂-O-(CH₂)₀₋₂-CH₃ und C₁-C₆-Alkyl,
R¹ Wasserstoff, CN, F, Cl, C₁₋₄-Alkyl, OH oder O-(C₁₋₄-Alkyl) ist,
R² Wasserstoff ist,
R³ ein Rest (W)-(X)-(Y)-Z ist, wobei
W O, CH₂NH, NHCH₂, OCH₂, CH₂O oder NH ist,
X CO ist,
Y ein Rest ausgewählt ist aus der Gruppe, bestehend aus 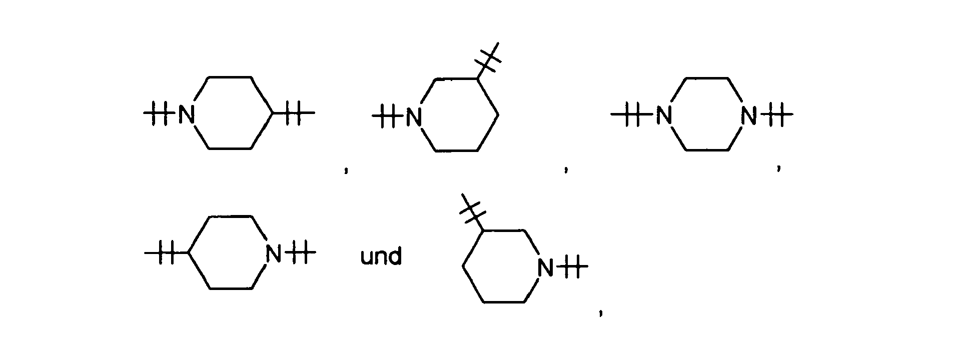
Z ein Rest ausgewählt ist aus der Gruppe, bestehend aus 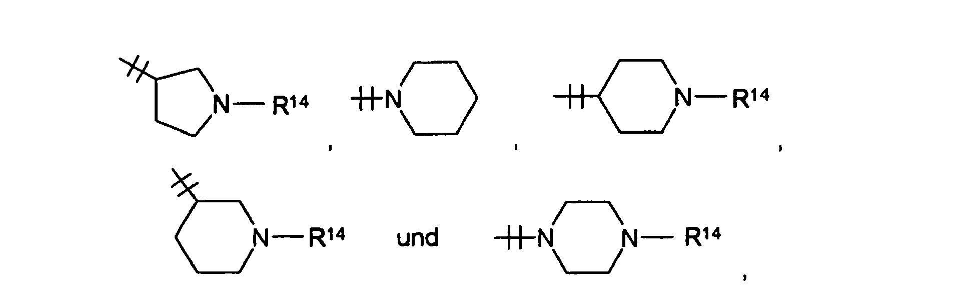 ist, wobei Z zusätzlich durch R¹² und/oder R¹³ substituiert sein kann, wobei
R¹² Wasserstoff oder C₁-C₄-Alkyl ist,
R¹³ Wasserstoff oder C₁-C₄-Alkyl ist und
R¹⁴ Wasserstoff, C₁-C₄-Alkyl, C₂-C₄-Alkenyl oder C₂-C₄-Alkinyl ist,
und ihre tautomeren, enantiomeren und/oder diastereomeren Formen, sowie die physiologisch verträglichen Salze der vorstehend genannten Verbindung oder Verbindungen.

4. Verbindung oder Verbindungen nach Anspruch 1, worin
A ein Phenyl-Ring ist, der mit maximal zwei Resten R⁴ substituiert sein kann, die unabhängig voneinander ausgewählt sind aus der Gruppe, bestehend aus Wasserstoff, Chlor, O-C₁-C₄-Alkyl, (CH₂)₀₋₂-O-(CH₂)₀₋₂-CH₃ und C₁-C₆-Alkyl,
B ein Phenyl-Ring ist, der mit den Resten R⁶ und/oder R⁷ substituiert sein kann, wobei R⁶ und R⁷ unabhängig voneinander ausgewählt sind aus der Gruppe, bestehend aus Wasserstoff, Fluor, Chlor, O-C₁-C₄-Alkyl und C₁-C₆-Alkyl,
R¹ Wasserstoff, F, Cl, CH₃, CN, CH₂CH₃, OCH₃ oder OCH₂CH₃ ist,
R² Wasserstoff ist,
R³ ein Rest (W)-(X)-(Y)-Z ist, wobei
W O, CH₂ oder NH ist,
X CO ist,
Y ein Rest ausgewählt aus der Gruppe 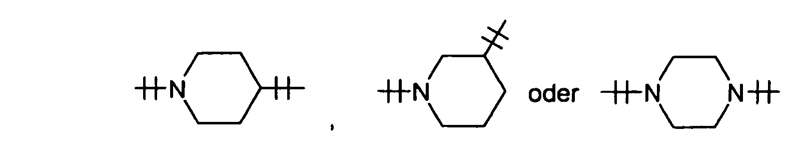 ist,
Z ein Rest ausgewählt aus der Gruppe 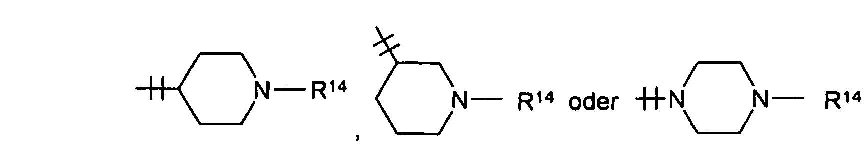 ist,
wobei Z durch R¹² und/oder R¹³ substituiert sein kann, wobei
R¹² Wasserstoff oder C₁-C₄-Alkyl ist,
R¹³ Wasserstoff oder C₁-C₄-Alkyl ist und
R¹⁴ Wasserstoff, C₁-C₄-Alkyl, C₂-C₄-Alkenyl oder C₂-C₄-Alkinyl ist,
und ihre tautomeren, enantiomeren und/oder diastereomeren Formen, sowie die physiologisch verträglichen Salze der vorstehend genannten Verbindung oder Verbindungen.

5. Verbindung oder Verbindungen nach Anspruch 1, worin
A ein Phenyl-Ring ist, der mit maximal zwei Resten R⁴ substituiert sein kann, die unabhängig voneinander ausgewählt sind aus der Gruppe, bestehend aus Wasserstoff, Chlor, O-C₁-C₄-Alkyl, (CH₂)₀₋₂-O-(CH₂)₀₋₂-CH₃, und C₁-C₆-Alkyl,
B ein Phenyl-Ring ist, der mit den Resten R⁶ und/oder R⁷ substituiert sein kann, wobei R⁶ und R⁷ unabhängig voneinander ausgewählt sind aus der Gruppe, bestehend aus Wasserstoff, Fluor, Chlor, O-C₁-C₄-Alkyl, und C₁-C₆-Alkyl,
R¹ Cl, CH₃, CN, CH₂CH₃ oder OCH₃ ist,
R² Wasserstoff ist,
R³ ein Rest (W)-(X)-(Y)-Z ist, wobei
W CH₂, O oder NH ist,
X CO ist,
Y ein Rest 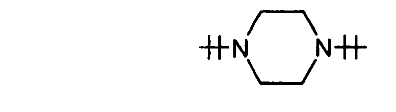 ist,
Z ein Rest 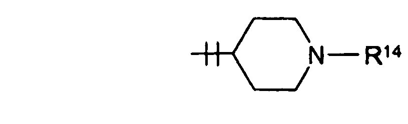 ist,
wobei
R¹⁴ Wasserstoff, C₁-C₄-Alkyl, C₂-C₄-Alkenyl oder C₂-C₄-Alkinyl ist,
und ihre tautomeren, enantiomeren und/oder diastereomeren Formen, sowie die physiologisch verträglichen Salze der vorstehend genannten Verbindung oder Verbindungen.

6. Verbindung oder Verbindungen nach Anspruch 1, worin
A ein Phenyl-Ring ist, der mit maximal zwei Resten R⁴ substituiert sein kann, die unabhängig voneinander ausgewählt sind aus der Gruppe, bestehend aus Wasserstoff, Chlor, O-C₁-C₄-Alkyl und C₁-C₄-Alkyl,
B ein Phenyl-Ring ist, der mit den Resten R⁶ und/oder R⁷ substituiert ist, wobei R⁶ und R⁷ unabhängig voneinander ausgewählt sind aus der Gruppe, bestehend aus Wasserstoff, Fluor, Chlor, O-C₁-C₄-Alkyl, und C₁-C₆-Alkyl,
R¹ Wasserstoff, Cl, CH₃, CN, CH₂CH₃, OCH₃ oder OCH₂CH₃ ist,
R² Wasserstoff ist,
R³ ein Rest (W)-(X)-(Y)-Z ist, wobei
W CH₂, O oder NH ist,
X CO ist,
Y ein Rest 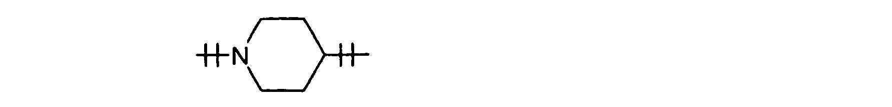 ist, und
Z 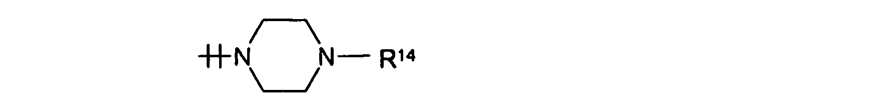 ist,
wobei
R¹⁴ Wasserstoff, C₁-C₄-Alkyl, C₂-C₄-Alkenyl oder C₂-C₄-Alkinyl ist,
und ihre tautomeren, enantiomeren und/oder diastereomeren Formen, sowie die physiologisch verträglichen Salze der vorstehend genannten Verbindung oder Verbindungen.

7. Arzneimittel, enthaltend mindestens eine Verbindung nach mindestens einem der Ansprüche 1 bis 6 sowie gegebenenfalls übliche pharmazeutische Hilfsstoffe.

8. Verwendung mindestens einer Verbindung nach einem der Ansprüche 1 bis 6 zur Herstellung eines Medikaments zur Behandlung und/oder Prophylaxe von Vasopressin-abhängigen oder Oxytocin-abhängigen Krankheiten.

9. Verwendung mindestens einer Verbindung nach einem der Ansprüche 1 bis 6 zur Herstellung eines Medikaments zur Behandlung und/oder Prophylaxe von Diabetes insipidus, Enuresis nocturna, Inkontinenz, Krankheiten, bei denen Blutgerinnungsstörungen auftreten und/oder zur Verzögerung der Miktion.

10. Verwendung mindestens einer Verbindung nach einem der Ansprüche 1 bis 6 zur Herstellung eines Medikaments zur Behandlung und/oder Prophylaxe von Hypertonie, pulmonale Hypertonie, Herzinsuffizienz, Myocardinfarkt, Koronarer Spasmus, instabile Angina, PTCA (percutaneous transluminal coronary angioplasie), Ischemien des Herzens, Störungen des renalen Systems, Ödeme, renalem Vasospasmus, Nekrose des renalen Cortex, Hyponaträmie, Hypokalämie, Schwartz-Bartter Syndrom, Störungen des Gastrointestinaltraktes, gastritischem Vasospasmus, Hepatozirrhose, Magen- und Darmulkus, Emesis, auftretender Emesis bei der Chemotherapie, und/oder Reisekrankheit.

11. Verwendung von mindestens einer Verbindung nach einem der Ansprüche 1 bis 6 zur Herstellung eines Medikaments zur Behandlung von Krankheiten.

12. Verwendung von mindestens einer Verbindung nach einem der Ansprüche 1 bis 6 zur Herstellung eines Medikaments zur Behandlung von affektiven Störungen.

13. Verwendung von mindestens einer Verbindung nach einem der Ansprüche 1 bis 6 zur Herstellung eines Medikaments zur Behandlung von Angststörungen und stressabhängigen Angststörungen.

14. Verwendung von mindestens einer Verbindung nach einem der Ansprüche 1 bis 6 zur Herstellung eines Medikaments zur Behandlung von Gedächnisleistungsstörungen und/oder Morbus Alzheimer.

15. Verwendung von mindestens einer Verbindung nach einem der Ansprüche 1 bis 6 zur Herstellung eines Medikaments zur Behandlung von Psychosen und/oder psychotischen Störungen.

16. Verwendung von mindestens einer Verbindung nach einem der Ansprüche 1 bis 6 zur Herstellung eines Medikaments zur Behandlung des Cushing-Syndroms.

17. Verwendung von mindestens einer Verbindung nach einem der Ansprüche 1 bis 6 zur Herstellung eines Medikaments zur Behandlung von Schlafstörungen.

18. Verbindung oder Verbindungen nach einem der Ansprüche 1 bis 6 zur Verwendung als Arzneimittel.

19. Verfahren zur Herstellung von einer Verbindung gemäß der allgemeinen Formel (I) 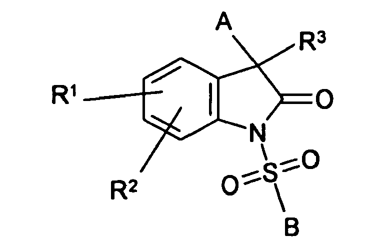 worin die Reste R¹, R², R³, A und B die in einem der Ansprüche 1 bis 6 genannten Bedeutungen besitzen, **dadurch gekennzeichnet, dass** gemäß Verfahrensvariante (A) das an sich bekannte Isatin oder Isatin-Derivat, welches mit den Resten R¹ und R² substituiert ist, nach an sich bekannten Verfahrensweisen durch Einführung des Restes A in 3-Position unter Erhalt der entsprechenden 3-Hydroxy-oxindol-Derivate, gefolgt von Einführung des B-SO₂-Restes am Ringstickstoff und Austausch des Restes R³ gegend die 3-Hydroxygruppe oder einer anderen geeigneten Abgangsgruppe umgesetzt wird
oder gemäß Verfahrensvariante (B) das an sich bekannte Isatin oder Isatin-Derivat, welches mit den Resten R¹ und R² substituiert ist, nach an sich bekannten Verfahrensweisen zunächst durch Einführung des Restes B-SO₂ am Ringstickstoff substituiert und anschließender Einführung des Restes A in 3-Position unter Erhalt der entsprechenden 3-Hydroxy-oxindol-Derivate, gefolgt vom Austausch des Restes R³ gegen die 3-Hydroxygruppe oder einer anderen geeigneten Abgangsgruppe umgesetzt wird.

## Claims

1. Compound or compounds of the general formula (I) 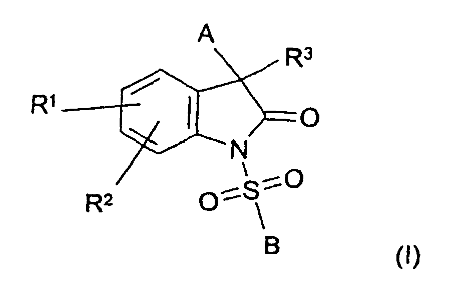 wherein
A is C₆₋₁₀-aryl which can be substituted by a maximum of four radicals R⁴ which are chosen independently of one another from the group consisting of hydrogen, chlorine, bromine, iodine, fluorine, (CH₂)₀₋₂-CN, CF₃, OCF₃, CONH₂, CONH(C₁-C₄-alkyl), CON(C₁-C₄-alkyl)(C₁-C₄-alkyl), NHCHO, NHCONH₂, N(C₀-C₄-alkylene)CONH₂, N(C₀-C₄-alkylene)CONH(C₁-C₄-alkyl), NHCOCH₃, NO₂, (CH₂)₀₋₂-OH, O-C₁-C₆-alkyl, (CH₂)₀₋₂-O-C₁-C₄-alkyl, O-C₀-C₄-alkylene-phenyl, phenyl, C₁-C₆-alkyl, C₂-C₆-alkenyl and C₂-C₆-alkynyl,
B is an aromatic or partly aromatic C₆₋₁₀-mono- or -bicyclic radical, which can be substituted by the radicals R⁶, R⁷, R⁸ and/or R⁹, wherein R⁶, R⁷, R⁸ and R⁹ are chosen independently of one another from the group consisting of hydrogen, chlorine, bromine, iodine, fluorine, (CH₂)₀₋₂-CN, CF₃, OCF₃, CONH₂, CONH (C₁-C₄-alkyl), CON (C₁-C₄alkyl) (C₁-C₄-alkyl), NHCHO, N (C₀-C₄-alkylene) CONH (C₁-C₄-alkyl), NHCOCH₃, NO₂, OH, O-C₁-C₄-alkyl, (CH₂)₀₋₂-O-(CH₂)₀₋₃-CH₃, O-C₀-C₄-alkylene-phenyl, phenyl, C₁-C₆-alkyl, C₂-C₆-alkenyl and C₂-C₆-alkynyl,
R¹ is hydrogen, C₁-C₆-alkyl, OH, O- (C₁-C₄-alkyl), N(C₁-C₄-alkyl)(C₁-C₄-alkyl), CN, CONH₂, OCF₃, CF₃, Br, F, Cl, I, NO₂, NHCHO, NHCO(C₁-C₄-alkyl) or NHCONH₂,
R² is hydrogen, C₁-C₄-alkyl, O-(C₁-C₄-alkyl), Cl or F,
R³ is a radical (W)-(X)-(Y)-Z, wherein
W is C₁-C₄-alkylene, (C₀-C₄-alkylene) -O- (C₀-C₄-alkylene) or (C₀-C₄-alkylene)-NR¹⁵-(C₀-C₄-alkylene), wherein R¹⁵ is hydrogen or C₁-C₄-alkyl,
X is CO, SO₂, (C=NH) or (C=N-CN) and
Y is a radical chosen from the group consisting of 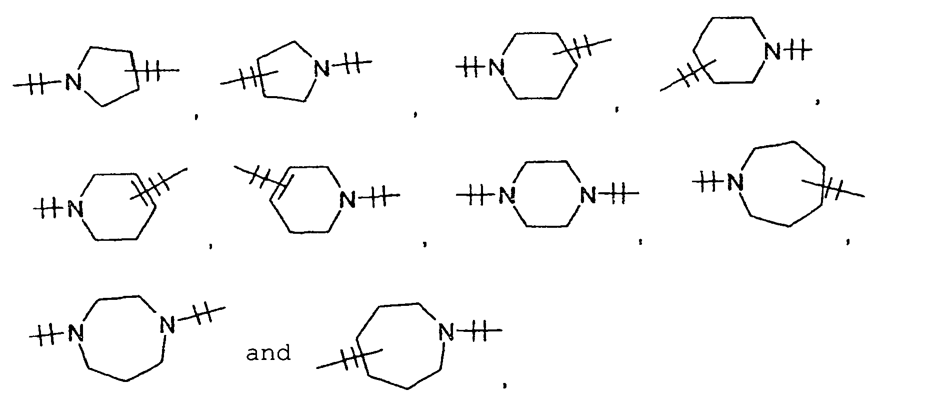
wherein Y can additionally be substituted by R¹⁰ and/or R¹¹, wherein
R¹⁰ is hydrogen, C₁-C₆-alkyl, C₂-C₆-alkenyl, C₂-C₆-alkynyl, OH, O-C₁-C₄-alkyl, O-C₀-C₄-alkylene-phenyl, NH₂, NH(C₁-C₄-alkyl) or N(C₁-C₄-alkyl) (C₁-C₄-alkyl),
R¹¹ is hydrogen, C₁-C₆-alkyl, C₂-C₆-alkenyl, C₂-C₆-alkynyl, OH, O-C₁-C₄-alkyl, O-C₀-C₄-alkylene-phenyl, NH₂, NH (C₁-C₄-alkyl) or N (C₁-C₄-alkyl) (C₁-C₄-alkyl), and
Z is a radical chosen from the group consisting of 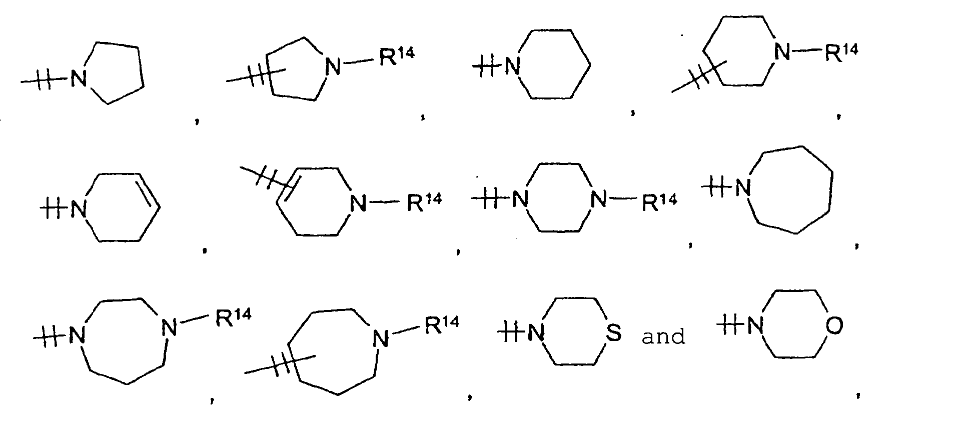 wherein Z can additionally be substituted by R¹² and/or R¹³, wherein
R¹² is hydrogen, C₁-C₆-alkyl, C₂-C₆-alkenyl, C₂-C₆-alkynyl, OH, O-C₁-C₄-alkyl, O-C₀-C₄-alkylene-phenyl, NH₂, NH (C₁-C₄-alkyl) or N(C₁-C₄-alkyl) (C₁-C₄-alkyl),
R¹³ is hydrogen, C₁-C₆-alkyl, C₂-C₆-alkenyl, C₂-C₆-alkynyl, OH, O (C₁-C₄-alkyl) , O-C₀-C₄-alkylene-phenyl, NH₂, NH(C₁-C₄-alkyl) or N(C₁-C₄-alkyl) (C₁-C₄-alkyl),
R¹⁴ is hydrogen, C₁-C₄-alkyl, C₂-C₆-alkenyl, C₂-C₆-alkynyl or C₀-C₄-alkylene-phenyl, and
their tautomeric, enantiomeric and/or diastereomeric forms, and the physiologically acceptable salts of the abovementioned compound or compounds.

2. Compound or compounds according to claim 1, wherein A is a phenyl ring which can be substituted by a maximum of four radicals R⁴, and B is a phenyl ring which can be substituted by the radicals R⁶, R⁷, R⁸ and/or R⁹, wherein the radicals R⁴, R⁶, R⁷, R⁸ and R⁹ are intended to have the meanings mentioned in claim 1,
and their tautomeric, enantiomeric and/or diastereomeric forms, and the physiologically acceptable salts of the abovementioned compound or compounds.

3. Compound or compounds according to claim 1, wherein
A is a phenyl ring which can be substituted by a maximum of two radicals R⁴ which are chosen independently of one another from the group consisting of hydrogen, chlorine, O-C₁-C₄-alkyl, (CH₂)₀₋₂-O-(CH₂)₀₋₂-CH₃ and C₁-C₆-alkyl,
B is a phenyl ring which can be substituted by the radicals R⁶, R⁷, R⁸ and/or R⁹, wherein R⁶, R⁷, R⁸ and R⁹ are chosen independently of one another from the group consisting of hydrogen, fluorine, chlorine, O-C₁-C₄-alkyl, (CH₂)₀₋₂-O-(CH₂)₀₋₂-CH₃ and C₁-C₆-alkyl,
R¹ is hydrogen, CN, F, Cl, C₁₋₄-alkyl, OH or O-(C₁₋₄-alkyl),
R² is hydrogen,
R³ is a radical (W)-(X)-(Y)-Z, wherein
W is 0, CH₂NH, NHCH₂, OCH₂, CH₂O or NH,
X is CO,
Y is a radical chosen from the group consisting of 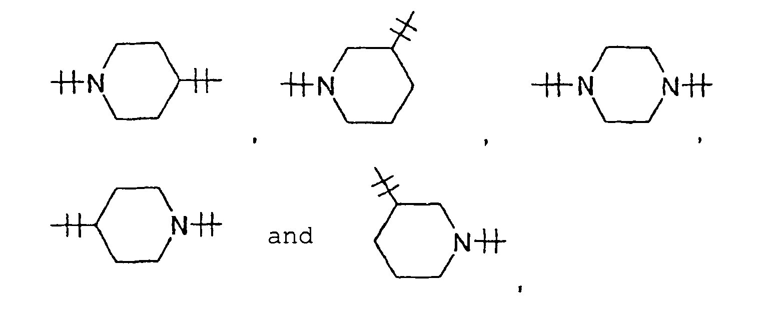
Z is a radical chosen from the group consisting of 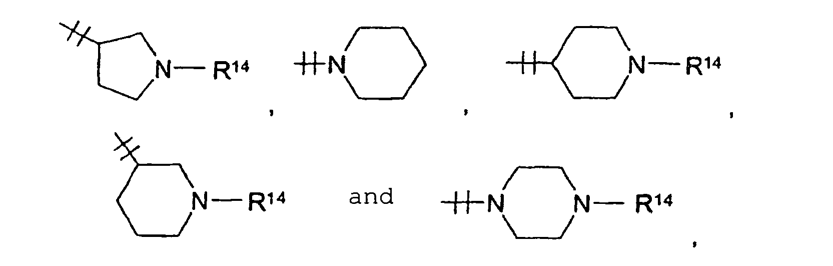 wherein Z can additionally be substituted by R¹² and/or R¹³, wherein
R¹² is hydrogen or C₁-C₄-alkyl,
R¹³ is hydrogen or C₁-C₄-alkyl and wherein
R¹⁴ is hydrogen, C₁-C₄-alkyl, C₂-C₄-alkenyl or C₂-C₄-alkynyl,
and their tautomeric, enantiomeric and/or diastereomeric forms, and the physiologically acceptable salts of the abovementioned compound or compounds.

4. Compound or compounds according to claim 1, wherein
A is a phenyl ring which can be substituted by a maximum of two radicals R⁴ which are chosen independently of one another from the group consisting of hydrogen, chlorine, O-C₁-C₄-alkyl, (CH₂)₀₋₂-O-(CH₂)₀₋₂-CH₃ and C₁-C₆-alkyl,
B is a phenyl ring which can be substituted by the radicals R⁶ and/or R⁷, wherein R⁶ and R⁷ are chosen independently of one another from the group consisting of hydrogen, fluorine, chlorine, O-C₁-C₄-alkyl and C₁-C₆-alkyl,
R¹ is hydrogen, F, Cl, CH₃, CN, CH₂CH₃, OCH₃ or OCH₂CH₃,
R² is hydrogen,
R³ is a radical (W)-(X)-(Y)-Z, wherein
W is O, CH₂ or NH,
X is CO,
Y is a radical chosen from the group consisting of 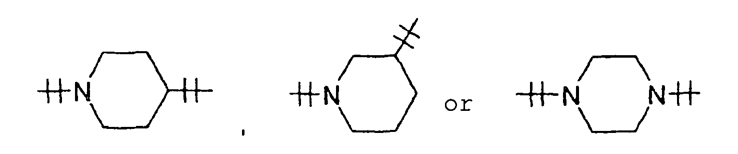
Z is a radical chosen from the group consisting of 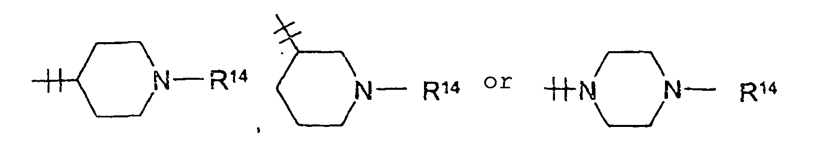 wherein Z can be substituted by R¹² and/or R¹³, wherein
R¹² is hydrogen or C₁-C₄-alkyl,
R¹³ is hydrogen or C₁-C₄-alkyl and
R¹⁴ is hydrogen, C₁-C₄-alkyl, C₂-C₄-alkenyl or C₂-C₄-alkynyl,
and their tautomeric, enantiomeric and/or diastereomeric forms, and the physiologically acceptable salts of the abovementioned compound or compounds.

5. Compound or compounds according to claim 1, wherein
A is a phenyl ring which can be substituted by a maximum of two radicals R⁴ which are chosen independently of one another from the group consisting of hydrogen, chlorine, O-C₁-C₄-alkyl, (CH₂)₀₋₂-O- (CH₂)₀₋₂-CH₃ and C₁-C₆-alkyl,
B is a phenyl ring which can be substituted by the radicals R⁶ and/or R⁷, wherein R⁶ and R⁷ are chosen independently of one another from the group consisting of hydrogen, fluorine, chlorine, O-C₁-C₄-alkyl, and C₁-C₆-alkyl,
R¹ is Cl, CH₃, CN, CH₂CH₃ or OCH₃,
R² is hydrogen,
R³ is a radical (W) - (X) - (Y) -Z, wherein
W is CH₂, O or NH,
X is CO,
Y is a radical 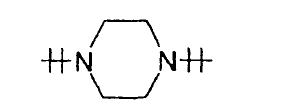
Z is a radical 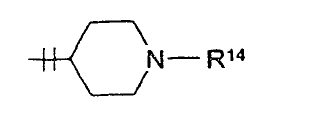 wherein
R¹⁴ is hydrogen, C₁-C₄-alkyl, C₂-C₄-alkenyl or C₂-C₄-alkynyl,
and their tautomeric, enantiomeric and/or diastereomeric forms, and the physiologically acceptable salts of the abovementioned compound or compounds.

6. Compound or compounds according to claim 1, wherein
A is a phenyl ring which can be substituted by a maximum of two radicals R⁴ which are chosen independently of one another from the group consisting of hydrogen, chlorine, O-C₁-C₄-alkyl and C₁-C₄-alkyl,
B is a phenyl ring which is substituted by the radicals R⁶ and/or R⁷, wherein R⁶ and R⁷ independently of one another are chosen from the group consisting of hydrogen, fluorine, chlorine, O-C₁-C₄-alkyl and C₁-C₆-alkyl,
R¹ is hydrogen, Cl, CH₃, CN, CH₂CH₃, OCH₃ or OCH₂CH₃,
R² is hydrogen,
R³ is a radical (W)-(X)-(Y)-Z, wherein
W is CH₂, 0 or NH,
X is CO,
Y is a radical 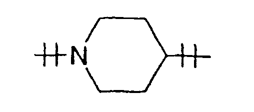 and
Z is a radical 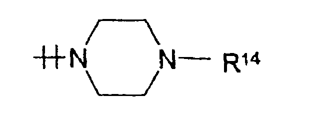
wherein
R¹⁴ is hydrogen, C₁-C₄-alkyl, C₂-C₄-alkenyl or C₂-C₄-alkynyl,
and their tautomeric, enantiomeric and/or diastereomeric forms, and the physiologically acceptable salts of the abovementioned compound or compounds.

7. Medicament comprising at least one compound according to one of claims 1 to 6 and optionally conventional pharmaceutical auxiliary substances.

8. Use of at least one compound according to one of claims 1 to 6 for the preparation of a medicament for treatment and/or prophylaxis of vasopressin-dependent or oxytocin-dependent diseases.

9. Use of at least one compound according to one of claims 1 to 6 for the preparation of a medicament for treatment and/or prophylaxis of at least one disease chosen from the group consisting of diabetes insipidus, enuresis nocturna, incontinence, diseases with which blood clotting disorders occur, and/or for delaying micturition.

10. Use of at least one compound according to one of claims 1 to 6 for the preparation of a medicament for treatment and/or prophylaxis of at least one disease chosen from the group consisting of hypertension, pulmonary hypertension, cardiac insufficiency, myocardial infarction, coronary spasm, unstable angina, PTCA (percutaneous transluminal coronary angioplasty), ischaemias of the heart, disorders of the renal system, oedemas, renal vasospasm, necrosis of the renal cortex, hyponatraemia, hypokalaemia, Schwartz-Bartter syndrome, disorders of the gastrointestinal tract, gastritic vasospasm, hepatocirrhosis, gastric and intestinal ulcer, emesis, emesis occurring with chemotherapy, and/or travel sickness.

11. Use of at least one compound according to one of claims 1 to 6 for the preparation of a medicament for treatment of diseases.

12. Use of at least one compound according to one of claims 1 to 6 for the preparation of a medicament for treatment of affective disorders.

13. Use of at least one compound according to one of claims 1 to 6 for the preparation of a medicament for treatment of anxiety disorders and/or stress-related anxiety disorders.

14. Use of at least one compound according to one of claims 1 to 6 for the preparation of a medicament for treatment of memory performance disorders and/or Alzheimer's disease.

15. Use of at least one compound according to one of claims 1 to 6 for the preparation of a medicament for treatment of psychoses and/or psychotic disorders.

16. Use of at least one compound according to one of claims 1 to 6 for the preparation of a medicament for treatment of Cushing's syndrome.

17. Use of at least one compound according to one of claims 1 to 6 for the preparation of a medicament for treatment of sleep disorders.

18. Compound or compounds according to one of claims 1 to 6 for use as medicaments.

19. Process for the preparation of a compound according to the general formula (I) 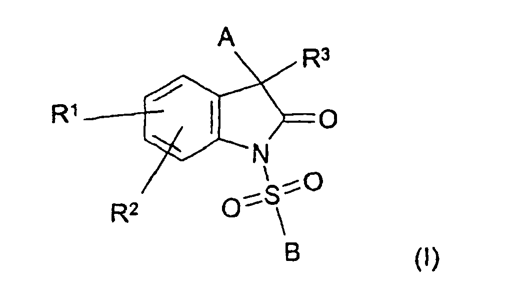 wherein the radicals R¹, R², R³, A and B have the meanings mentioned in one of claims 1 to 6, **characterized in that** according to process variant (A) the isatin or isatin derivative, which are known per se, substituted by the radicals R¹ and R² is reacted by procedures known per se by introduction of the radical A into the 3-position to give the corresponding 3-hydroxy-oxindole derivative, followed by introduction of the B-SO₂ radical on the ring nitrogen and exchange of the radical R³ for the 3-hydroxyl group or another suitable leaving group
or according to process variant (B) the isatin or isatin derivative, which are known per se, substituted by the radicals R¹ and R² is first substituted by procedures known per se by introduction of the radical B-SO₂ on the ring nitrogen and reacted by subsequent introduction of the radical A into the 3-position to give the corresponding 3-hydroxy-oxindole derivative, followed by exchange of the radical R³ for the 3-hydroxyl group or another suitable leaving group.

## Revendications

1. Composé ou composés de formule générale (I) 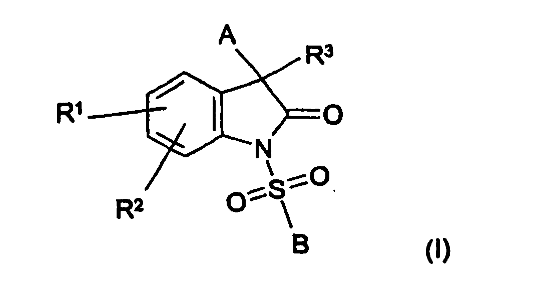 dans laquelle
A est un reste aryle en C₆-C₁₀, qui peut être substitué par au plus 4 restes R⁴ qui sont choisis indépendamment les uns des autres dans le groupe constitué par l'hydrogène, le chlore, le brome, l'iode, le fluor, (CH₂)₀₋₂-CN, CF₃, OCF₃, CONH₂, CONH(alkyle en C₁-C₄), CON(alkyle en C₁-C₄)(alkyle en C₁-C₄), NHCHO, NHCONH₂, N(alkylène en C₀-C₄)CONH₂, N(alkylène en C₀-C₄)CONH(alkyle en C₁-C₄), NHCOCH₃, NO₂, (CH₂)₀₋₂-OH, O-alkyle en C₁-C₄, (CH₂)₀₋₂-O-(alkyle en C₁-C₄), O-(alkylène en C₀-C₄)-phényle, phényle, alkyle en C₁-C₆, alcényle en C₂-C₆ et alcynyle en C₂-C₆,
B est un reste monocyclique ou bicyclique en C₆-C₁₀ aromatique ou partiellement aromatique, qui peut être substitué par les restes R⁶, R⁷, R⁸ et/ou R⁹, où R⁶, R⁷, R⁸ et R⁹ sont choisis indépendamment les uns des autres dans le groupe constitué par l'hydrogène, le chlore, le brome, l'iode, le fluor, (CH₂)₀₋₂-CN, CF₃, OCF₃, CONH₂, CONH(alkyle en C₁-C₄), CON(alkyle en C₁-C₄)(alkyle en C₁-C₄), NHCHO, N(alkylène en C₀-C₄)CONH(alkyle en C₁-C₄), NHCOCH₃, NO₂, OH, O-alkyle en C₁-C₄, (CH₂)₀₋₂-O-(CH₂)₀₋₃-CH₃, O-(alkylène en C₀-C₄)phényle, phényle, alkyle en C₁-C₆, alcényle en C₂-C₆ et alcynyle en C₂-C₆,
R¹ est l'hydrogène ou un reste alkyle en C₁-C₆, OH, O-(alkyle en C₁-C₄), N(alkyle en C₁-C₄)(alkyle en C₁-C₄), CN, CONH₂, OCF₃, CF₃, Br, F, CI, I, NO₂, NHCHO, NHCO(alkyle en C₁-C₄) ou NHCONH₂,
R² est l'hydrogène ou un reste alkyle en C₁-C₄, O-(alkyle en C₁-C₄), Cl ou F,
R³ est un reste (W)-(X)-(Y)-Z, dans lequel
W est un reste alkylène en C₁-C₄, (alkylène en C₀-C₄)-O-(alkylène en C₀-C₄) ou (alkylène en C₀-C₄)-NR¹⁵-(alkylène en C₀-C₄), où R¹⁵ est l'hydrogène ou un alkyle en C₁-C₄,
X est CO, SO₂, (C=NH) ou (C=N-CN) et
Y est un reste choisi dans le groupe constitué par 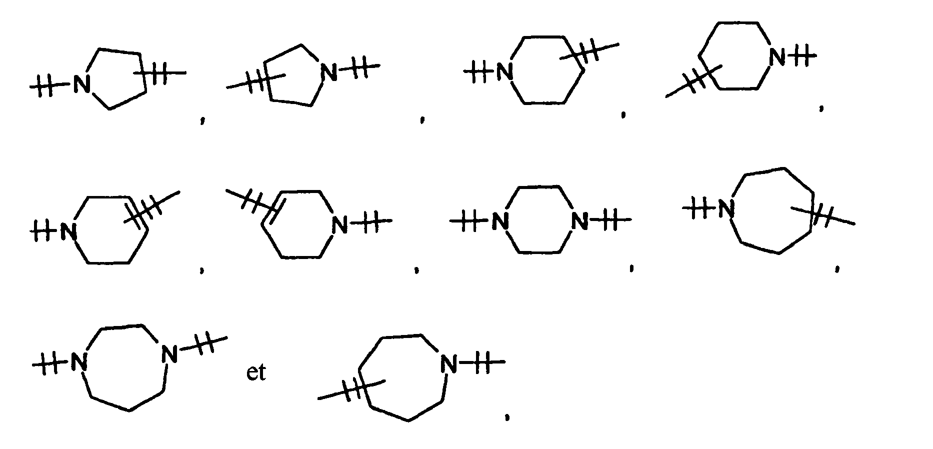
Y pouvant en outre être substitué par R¹⁰ et/ou R¹¹, où
R¹⁰ est l'hydrogène ou un reste alkyle en C₁-C₆, alcényle en C₂-C₆, alcynyle en C₂-C₆, OH, O-alkyle en C₁-C₄, O-(alkylène en C₀-C₄)-phényle, NH₂, NH(alkyle en C₁-C₄) ou N(alkyle en C₁-C₄)(alkyle en C₁-C₄),
R¹¹ est l'hydrogène ou un reste alkyle en C₁-C₆, alcényle en C₂-C₆, alcynyle en C₂-C₆, OH, O-alkyle en C₁-C₄, O-(alkylène en C₀-C₄)-phényle, NH₂, NH(alkyle en C₁-C₄) ou N(alkyle en C₁-C₄)(alkyle en C₁-C₄), et
Z est un reste choisi dans le groupe constitué par 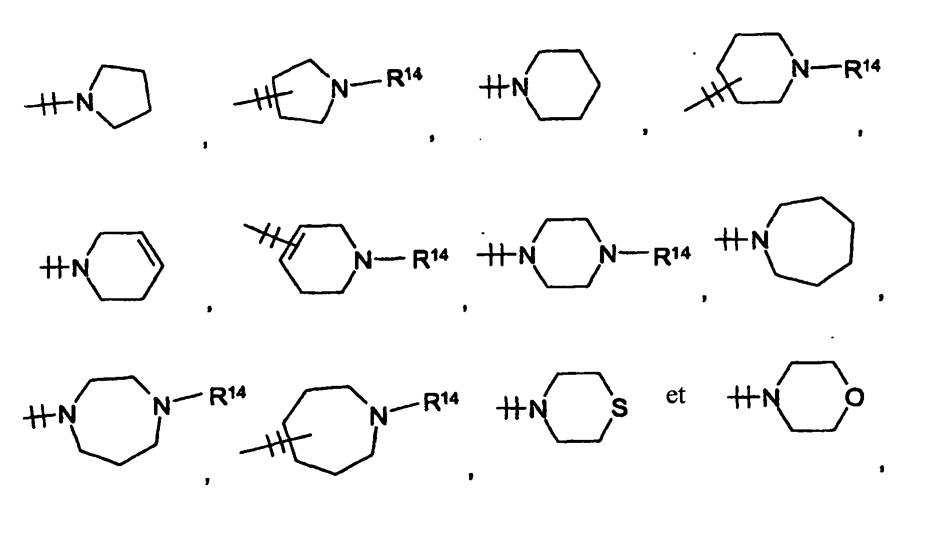 où Z peut être en outre substitué par R¹² et/ou R¹³, où
R¹² est l'hydrogène ou un reste alkyle en C₁-C₆, alcényle en C₂-C₆, alcynyle en C₂-C₆, OH, O-alkyle en C₁-C₄, O-(alkylène en C₀-C₄)-phényle, NH₂, NH(alkyle en C₁-C₄) ou N(alkyle en C₁-C₄)(alkyle en C₁-C₄),
R¹³ est l'hydrogène ou un reste alkyle en C₁-C₆, alcényle en C₂-C₆, alcynyle en C₂-C₆, OH, O-alkyle en C₁-C₄, O-(alkylène en C₀-C₄)-phényle, NH₂, NH(alkyle en C₁-C₄) ou N(alkyle en C₁-C₄)(alkyle en C₁-C₄),
R¹⁴ est l'hydrogène ou un reste alkyle en C₁-C₆, alcényle en C₂-C₆, alcynyle en C₂-C₆ ou (alkylène en C₀-C₄)-phényle, et
leurs formes tautomères, énantiomères et/ou diastéréoisomères, ainsi que les sels physiologiquement acceptables du composé ou des composés précités.

2. Composé ou composés selon la revendication 1, dans lesquels A est un cycle phényle qui peut être substitué par au plus 4 restes R⁴, et B est un cycle phényle qui peut être substitué par les restes R⁶, R⁷, R⁸ et/ou R⁹, les restes R⁴, R⁶, R⁷, R⁸ et R⁹ devant avoir les significations indiquées dans la revendication 1,
et leurs formes tautomères, énantiomères et/ou diastéréoisomères, ainsi que les sels physiologiquement acceptables du composé ou des composés précités.

3. Composé ou composés selon la revendication 1, dans lesquels
A est un cycle phényle qui peut être substitué par au plus deux restes R⁴ qui sont choisis indépendamment l'un de l'autre dans le groupe constitué par l'hydrogène, le chlore, O-alkyle en C₁-C₄, (CH₂)₀₋₂-O-(CH₂)₀₋₂-CH₃ et alkyle en C₁-C₆,
B est un cycle phényle qui peut être substitué par les restes R⁶, R⁷, R⁸ et/ou R⁹, où R⁶, R⁷, R⁸ et R⁹ sont choisis indépendamment les uns des autres dans le groupe constitué par l'hydrogène, le fluor, le chlore, O-alkyle en C₁-C₄, (CH₂)₀₋₂-O-(CH₂)₀₋₂-CH₃ et alkyle en C₁-C₆,
R¹ est l'hydrogène, CN, F, Cl, alkyle en C₁-C₄, OH ou O-(alkyle en C₁-C₄),
R² est l'hydrogène,
R³ est un reste (W)-(X)-(Y)-Z, dans lequel
W est O, CH₂NH, NHCH₂, OCH₂, CH₂O ou NH,
X est CO,
Y est un reste choisi dans le groupe constitué par 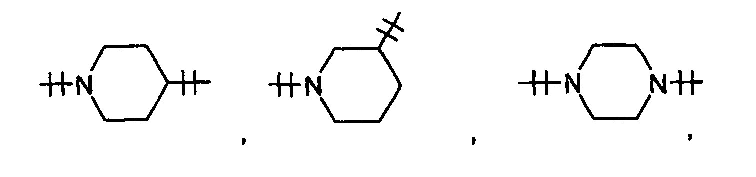 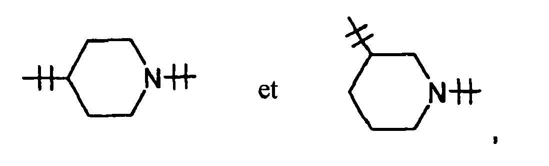
Z est un reste choisi dans le groupe constitué par 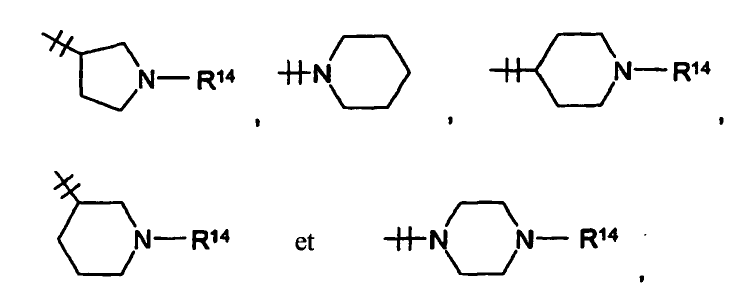
Z pouvant en outre être substitué par R¹² et/ou R¹³, où
R¹² est l'hydrogène ou un reste alkyle en C₁-C₄,
R¹³ est l'hydrogène ou un reste alkyle en C₁-C₄, et où
R¹⁴ est l'hydrogène ou un reste alkyle en C₁-C₄, alcényle en C₂-C₄ ou alcynyle en C₂-C₄,
et leurs formes tautomères, énantiomères et/ou diastéréoisomères, ainsi que les sels physiologiquement acceptables du composé ou des composés précités.

4. Composé ou composés selon la revendication 1, dans lesquels
A est un cycle phényle qui peut être substitué par au plus deux restes R⁴ qui sont choisis indépendamment l'un de l'autre dans le groupe constitué par l'hydrogène, le chlore, O-alkyle en C₁-C₄, (CH₂)₀₋₂-O-(CH₂)₀₋₂-CH₃ et alkyle en C₁-C₆,
B est un cycle phényle qui peut être substitué par les restes R⁶ et/ou R⁷, où R⁶ et R⁷ sont choisis indépendamment l'un de l'autre dans le groupe constitué par l'hydrogène, le fluor, le chlore, O-alkyle en C₁-C₄, et alkyle en C₁-C₆,
R¹ est l'hydrogène, F, Cl, CH₃, CN, CH₂CH₃, OCH₃ ou OCH₂CH₃,
R² est l'hydrogène,
R³ est un reste (W)-(X)-(Y)-Z, dans lequel
W est O, CH₂ ou NH,
X est CO,
Y est un reste choisi dans le groupe constitué par 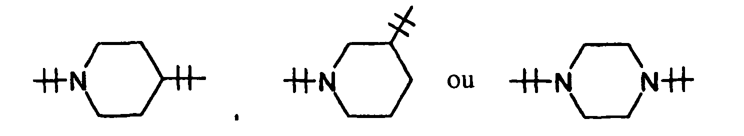
Z est un reste choisi dans le groupe constitué par 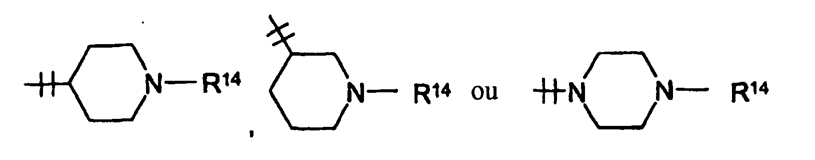
Z pouvant être substitué par R¹² et/ou R¹³, où
R¹² est l'hydrogène ou un reste alkyle en C₁-C₄,
R¹³ est l'hydrogène ou un reste alkyle en C₁-C₄, et où
R¹⁴ est l'hydrogène ou un reste alkyle en C₁-C₄, alcényle en C₂-C₄ ou alcynyle en C₂-C₄,
et leurs formes tautomères, énantiomères et/ou diastéréoisomères, ainsi que les sels physiologiquement acceptables du composé ou des composés précités.

5. Composé ou composés selon la revendication 1, dans lesquels
A est un cycle phényle qui peut être substitué par au plus deux restes R⁴ qui sont choisis indépendamment l'un de l'autre dans le groupe constitué par l'hydrogène, le chlore, O-alkyle en C₁-C₄, (CH₂)₀₋₂-O-(CH₂)₀₋₂-CH₃ et alkyle en C₁-C₆,
B est un cycle phényle qui peut être substitué par les restes R⁶ et/ou R⁷, où R⁶ et R⁷ sont choisis indépendamment l'un de l'autre dans le groupe constitué par l'hydrogène, le fluor, le chlore, O-alkyle en C₁-C₄, et alkyle en C₁-C₆,
R¹ est Cl, CH₃, CN, CH₂CH₃ ou OCH₃,
R² est l'hydrogène,
R³ est un reste (W)-(X)-(Y)-Z, dans lequel
W est CH₂, O ou NH,
X est CO,
Y est un reste 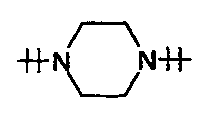
Z est un reste 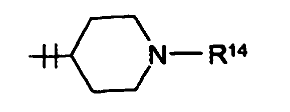 dans lequel
R¹⁴ est l'hydrogène ou un reste alkyle en C₁-C₄, alcényle en C₂-C₄ ou alcynyle en C₂-C₄,
et leurs formes tautomères, énantiomères et/ou diastéréoisomères, ainsi que les sels physiologiquement acceptables du composé ou des composés précités.

6. Composé ou composés selon la revendication 1, dans lesquels
A est un cycle phényle qui peut être substitué par au plus deux restes R⁴ qui sont choisis indépendamment l'un de l'autre dans le groupe constitué par l'hydrogène, le chlore, O-alkyle en C₁-C₄ et alkyle en C₁-C₄,
B est un cycle phényle qui peut être substitué par les restes R⁶ et/ou R⁷, où R⁶ et R⁷ sont choisis indépendamment l'un de l'autre dans le groupe constitué par l'hydrogène, le fluor, le chlore, O-alkyle en C₁-C₄, et alkyle en C₁-C₆,
R¹ est l'hydrogène, Cl, CH₃, CN, CH₂CH₃,OCH₃ ou OCH₂CH₃,
R² est l'hydrogène,
R³ est un reste (W)-(X)-(Y)-Z, dans lequel
W est CH₂, O ou NH,
X est CO,
Y est un reste 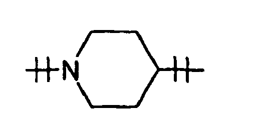 et
Z est un reste 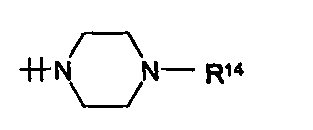 dans lequel
R¹⁴ est l'hydrogène ou un reste alkyle en C₁-C₄, alcényle en C₂-C₄ ou alcynyle en C₂-C₄,
et leurs formes tautomères, énantiomères et/ou diastéréoisomères, ainsi que les sels physiologiquement acceptables du composé ou des composés précités.

7. Médicaments contenant au moins un composé selon l'une des revendications 1 à 6 et éventuellement des agents auxiliaires pharmaceutiques classiques.

8. Utilisation d'au moins un composé selon l'une des revendications 1 à 6 pour la préparation d'un médicament destiné au traitement et/ou à la prophylaxie de maladies dépendant de la vasopressine ou dépendant de l'oxytocine.

9. Utilisation d'au moins un composé selon l'une des revendications 1 à 6 pour la préparation d'un médicament destiné au traitement et/ou à la prophylaxie d'au moins une maladie choisie dans le groupe constitué par le diabète insipide, l'énurésie nocturne, l'incontinence, des maladies dans lesquelles apparaissent des troubles de la coagulation sanguine et/ou pour retarder la miction.

10. Utilisation d'au moins un composé selon l'une des revendications 1 à 6 pour la préparation d'un médicament destiné au traitement et/ou à la prophylaxie d'au moins une maladie choisie dans le groupe constitué par l'hypertonie, l'hypertonie pulmonaire, l'insuffisance cardiaque, l'infarctus du myocarde, le spasme coronarien, l'angine instable, l'ACTP (angioplasie coronarienne transluminale percutanée), les ischémies cardiaques, les troubles du système rénal, les oedèmes, la vasospasme rénal, la nécrose du cortex rénal, l'hyponatrémie, l'hypokalémie, le syndrome de Schwartz-Bartter, les troubles des voies gastro-intestinales, le vasospasme gastritique, la cirrhose du foie, l'ulcère gastrique et intestinal, les vomissements, les vomissements apparaissant lors d'une chimiothérapie, et/ou le mal des transports.

11. Utilisation d'au moins un composé selon l'une des revendications 1 à 6 pour la préparation d'un médicament destiné au traitement de maladies.

12. Utilisation d'au moins un composé selon l'une des revendications 1 à 6 pour la préparation d'un médicament destiné au traitement de troubles affectifs.

13. Utilisation d'au moins un composé selon l'une des revendications 1 à 6 pour la préparation d'un médicament destiné au traitement des troubles d'angoisse et/ou des troubles d'angoisse dépendant du stress.

14. Utilisation d'au moins un composé selon l'une des revendications 1 à 6 pour la préparation d'un médicament destiné au traitement de troubles de la mémoire et/ou de la maladie d'Alzheimer.

15. Utilisation d'au moins un composé selon l'une des revendications 1 à 6 pour la préparation d'un médicament destiné au traitement de psychoses et/ou de troubles psychotiques.

16. Utilisation d'au moins un composé selon l'une des revendications 1 à 6 pour la préparation d'un médicament destiné au traitement du syndrome de Cushing.

17. Utilisation d'au moins un composé selon l'une des revendications 1 à 6 pour la préparation d'un médicament destiné au traitement de troubles du sommeil.

18. Composé ou composés selon l'une des revendications 1 à 6 à utiliser comme médicaments.

19. Procédé de préparation d'un composé selon la formule générale (I) 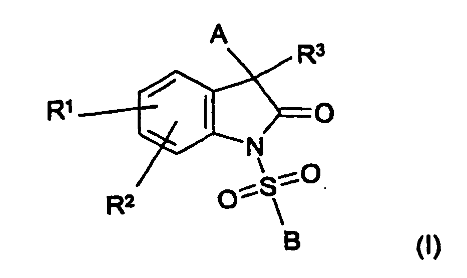 dans laquelle les restes R¹, R², R³, A et B ont les significations indiquées dans l'une des revendications 1 à 6, **caractérisé en ce que**
selon la variante de procédé (A), on transforme par des techniques connues en soi l'isatine connue en soi ou un dérivé d'isatine qui est substitué par les restes R¹ et R², en introduisant le reste A en position 3 pour obtenir les dérivés de 3-hydroxyoxindole correspondants, puis en introduisant le reste B-SO₂ sur l'azote cyclique et en échangeant le groupe 3-hydroxy ou un autre reste partant approprié contre le reste R³,
ou, selon la variante de procédé (B), par des techniques connues en soi, on transforme l'isatine connue en soi ou un dérivé d'isatine qui est substitué par les restes R¹ et R², en introduisant d'abord le reste B-SO₂ sur l'azote cyclique par substitution, et en introduisant ensuite le reste A en position 3 pour obtenir les dérivés de 3-hydroxyindole correspondants, puis on échange le groupe 3-hydroxy ou un autre reste partant approprié contre le reste R³.
